# EUROPEAN PATENT SPECIFICATION

(11) **EP 1 277 738 B1**
(45) Date of publication and mention of the grant of the patent: **30.03.2011**
(21) Application number: 01925981.1
(22) Date of filing: 26.04.2001
(51) Int. Cl.: C07D 215/42, C07D 239/94, C07D 471/04, C07D 495/14, C07D 413/04, C07D 401/04, C07D 401/12, C07D 409/04, C07D 409/12, C07D 405/04, C07D 405/12, C07D 403/04, C07D 417/12, C07D 487/04, C07D 495/04, C07D 491/048, A61K 31/5377, A61K 31/519, A61K 31/517, A61P 35/00

(54) **CONDENSED HETEROARYL DERIVATIVES**
KONDENSIERTE HETEROARYLDERIVATE
DERIVES D'HETEROARYLE CONDENSES

(30) Priority: 27.04.2000 JP 2000128472
(43) Date of publication of application: 22.01.2003
(73) Proprietor: Astellas Pharma Inc., Chuo-ku, Tokyo (JP); LUDWIG INSTITUTE FOR CANCER RESEARCH, New York, New York 10158 (US); IMPERIAL CANCER RESEARCH TECHNOLOGY LIMITED, London WC2A 3NL (GB)
(72) Inventor: HAYAKAWA, Masahiko, Tsukuba-shi, Ibaraki 305-8585 (JP); KAIZAWA, Hiroyuki, Tsukuba-shi, Ibaraki 305-8585 (JP); MORITOMO, Hiroyuki, Tsukuba-shi, Ibaraki 305-8585 (JP); KAWAGUCHI, Ken-ichi, Tsukuba-shi, Ibaraki 305-8585 (JP); KOIZUMI, Tomonobu, Tsukuba-shi, Ibaraki 305-8585 (JP); YAMANO, Mayumi, Tsukuba-shi, Ibaraki 305-8585 (JP); MATSUDA, Koyo, Tsukuba-shi, Ibaraki 305-8585 (JP); OKADA, Minoru, Tsukuba-shi, Ibaraki 305-8585 (JP); OHTA, Mitsuaki, Tsukuba-shi, Ibaraki 305-8585 (JP)
(74) Representative: Bates, Philip Ian
(86) International application number: PCT/JP2001/003650
(87) International publication number: WO 2001/083456

(56) References cited:
- EP-A1- 0 655 456
- EP-A1- 0 882 717
- WO-A1-99/51582
- GB-A- 2 295 387
- JP-A- 10 087 492
- JP-A- 10 175 977
- JP-A- 11 209 287
- JP-A- 58 172 379
- US-A- 3 808 211
- US-A- 5 990 117
- MERINO ISIDRO ET AL.: 'Synthesis and anti-HIV-1 activities of new pyrimido (5,4-b)indoles' FARMACO vol. 54, no. 4, 1999, pages 255 - 264, XP002944143
- VAIDYA V.P. ET AL.: 'Studies in benzofurans. Part XII. synthesis and reactions of 2-chloromethyl-3, 4-dihydro-4-oxobenzofuro(3, 2-d)pyrimidine' INDIAN J. CHEMICAL, SECT. B vol. 20B, no. 9, 1981, pages 780 - 783, XP002944144
- TSIZIN YU. S. ET AL.: 'Synthesis and antimicrobial action of N-substituted 2-phenyl-4-amino-6-hydroxyquinazolines' KHIM.-FARM. ZH. vol. 7, no. 7, 1973, pages 16 - 19, XP002944145
- DATABASE CAPLUS CHEMICAL ABSTRACTS SERVICE, COLUMBUS, OHIO, US; 1975, Bourguignon: "Synthesis of thieno..." Database accession no. 1975:514329

## Description

### FIELD OF THE INVENTION

The present invention relates to fused heteroaryl derivatives which are useful as medicaments, more particularly as phosphatidylinositol 3-kinase (PI3K) inhibitors and carcinostatic agents.

### BACKGROUND OF THE INVENTION

Phosphatidylinositol (hereinafter abbreviated as "PI") is one of phospholipids in cell membranes. In recent years it has become clear that PI plays an important role also in intracellular signal transduction. It is well recognized in the art that especially PI (4,5) bisphosphate (PI(4,5)P2) is degraded into diacylglycerol and inositol (1, 4, 5) triphosphate by phospholipase C to induce activation of protein kinase C and intracellular calcium mobilization, respectively [M. J. Berridge et al., Nature, 312, 315 (1984); Y Nishizuka, Science, 225, 1365 (1984)].

Turning back to the late 1980s, PI3K was found to be an enzyme to phosphorylate the 3-position of the inositol ring of phosphatidylinositol [D. Whitman et al., Nature, 332, 664 (1988)].

PI3K was originally considered to be a single enzyme at the time when PI3K was discovered. Recently it was clarified that a plurality of subtypes are present in the PI3K. Three major classes of PI3Ks have now been identified on the basis of their in vitro substrate specificity [B. Vanhaesebroeck, Trend in Biol. Sci., 22, 267(1997)].

Substrates for class I PI3Ks are PI, PI(4)P and PI(4,5)P2. In these substrates, PI(4,5)P2 is the most advantageous substrate in cells. Class I PI3Ks are further divided into two groups, class Ia and class Ib, in terms of their activation mechanism. Class Ia PI3Ks, which include PI3K p110α, p110β and p110δ subtypes, are activated in the tyrosine kinase system. Class Ib PI3K is a p110γ subtype activated by a G protein-coupled receptor.

PI and PI(4)P are known as substrates for class II PI3Ks but PI(4,5)P2 is not a substrate for the enzymes of this class. Class II PI3Ks include PI3K C2α, C2β and C2γ subtypes, which are characterized by containing C2 domains at the C terminus, implying that their activity will be regulated by calcium ions. The substrate for class III PI3Ks is PI only. A mechanism for activation of the class III PI3Ks is not clarified yet. Since each subtype has its own mechanism for the regulating activity, it is considered that the respective subtypes will be activated depending on their respective stimuli specific to each of them.

In the PI3K subtypes, the class Ia subtype has been most extensively investigated to date. The three subtypes of class Ia are hetero dimers of a catalytic 110 kDa subunit and regulatory subunits of 85 kDa and 55 kDa. The regulatory subunits contain SH2 domains and bind to tyrosine residues phosphorylated by growth factor receptors with a tyrosine kinase activity or oncogene products thereby inducing the PI3K activity of the p110 catalytic subunit. Thus, the class Ia subtypes are considered to be associated with cell proliferation and carcinogenesis. Furthermore, the class Ia PI3K subtypes bind to activated ras oncogene to express their enzyme activity. It has been confirmed that the activated ras oncogene is found to be present in many cancers, suggesting a role of class Ia PI3Ks in carcinogenesis.

As explained above, PI3K inhibitors are expected to be a novel type of medicaments useful against cell proliferation disorders, especially as carcinostatic agents. As for the PI3K inhibitor, wortmannin [H. Yano et al., J. Biol. Chem., 263, 16178 (1993)] and LY294002 [J. Vlahos et al., J. Biol. Chem., 269, 5241(1994)] which is represented by the formula below are known. However, development of PI3K inhibitors having a more potent cancer cell growth inhibiting activity is desired.

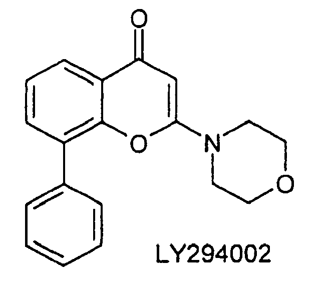

Japanese Patent KOKAI (Laid-Open) No. 6-220059 discloses fused heteroaryl derivatives shown by formula (a) below which possess an activity of reducing the blood glucose level. Furthermore, compounds shown by formula (b) and formula (c) below are described in Indian J. Chem., Sect. B (1993), 32B (9), 965-8 and J. Heterocycl. Chem. (1992), 29 (7), 1693-702, respectively. In addition, Al-AzharBull. Sci. (1992), 3(2), 767-75 discloses a compound shown by formula (d) below. However, none of these prior art publications disclose or suggest the PI3K inhibiting activity.

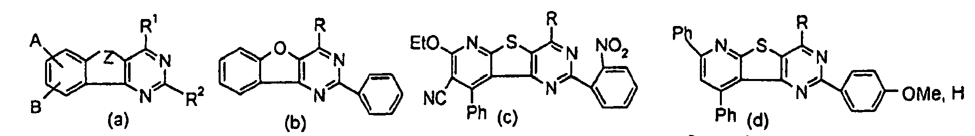

In formula (a) above, Z is **O,** S or =N-R⁰, R¹ is an amino which may be substituted, a heterocyclic group which may be substituted, etc.; R² is cyano, an amino which may be substituted, or a heterocyclic group which may be substituted; and with respect to the remaining substituents, see the specification of the patent. In formula (b) and (c) above, R is a (substituted) amino or a (substituted) nitrogen-containing saturated heterocyclic group.

WO98/23613 discloses fused pyrimidine derivatives, such as 7H-pyrrolo[2,3-d]pyrimidine derivatives, which having a tyrosine kinase receptor inhibiting activity and which are useful as carcinostatic agents, wherein the fused pyrimidine derivatives have at its fourth position a particular-heteroaryl-substituted amino, pheny-substituted amino, or indole-1-yl, and have no substituent at its second position.

Following compounds are known among the compounds shown by general formula (I), whereas "A" ring is a ring shown by (b) ;
(1) Ann. Pharm. Fr. (1974), 32(11), 575-9 discloses 4-(4-morpholinyl)-2-phenylpirido[2,3-d]pyrimidine as a compound having antiinflammatory and spasmolytic activities,
(2) Chem. Pharm. Bull. (1976), 24(9), 2057-77 discloses 4-(4-morpholinyl)-2-phenylpirido[2,3-d]pyrimidine-7(1H)-one as a compound having a diuretic activity,
(3) Khim.-Farm. Zh. (1993), 7(7), 16-19 and Khim. Geterotsiki. Soedin. (1971), 7(3), 418-20 disclose 4-(4-morpholinyl)-2-phenyl-6-quinazolinol and 6-methoxy-4-(4-morpholinyl)-2-phenylquinazoline as compounds having an antibiotic activity,
(4) W02000/41697 discloses 2,4-diamino-6-phenyl-8-piperidinopyrimido[5,4-d]pyrimidine as a compound having celebral ischemia prevention and treatment effects,
(5) WO99/32460 discloses, as cardiovascular drugs, compounds of general formula (Ib) described hereinafter wherein B is a benzene ring, W is N, n is 2 or 3, existing R^{1,}s are all - OMe, and R^{4b} is an unsubstituted phenyl or a phenyl substituted by 1 to 3 substituents which are selected from -a halogen, NO₂, -a lower alkyl, -O-a lower alkyl, -a halogenated lower alkyl and -CONRaRc,
(6) BE841669 discloses, as antiparasitics, compounds of general formula (Ib) described hereinafter wherein B is a benzene ring, W is N, n is 1, R¹ is -a halogen or -a lower alkyl, and R^{4b} is -(an imidazolyl which may have one or more substituents),
(7) WO99/43682 discloses, as antianxiety agents, compounds of general formula (Ib) described hereinafter wherein B is a thiophene ring, and W is CH,
(8) Japanese Patents KOKAI (Laid-Open) Nos. 62-10085 and 61-158983 disclose compounds of general formula (Ib) described hereinafter wherein B is an imidazole ring, and W is N, whereas the compounds have an antiinflammatory activity, a platelet aggregation inhibiting activity, etc.,
(9) US3,873,545 and Act Pol. Pharm. (1994), 51(4-5), 359-63 disclose compounds of general formula (Ib) described hereinafter wherein B is a pyridine ring, and R^{4b} is an unsubstituted phenyl, an unsubstituted pyridyl, or -a lower alkylene-(a nitrogen-containing saturated heterocyclic group which may have one or more substituents), whereas the compounds have a spasmolytic, diuretic or hypotensive activity,
(10) US2,940,972 discloses compounds of general formula (Ib) described hereinafter wherein B is a pyrazine ring, and R^{4b} is an unsubstituted phenyl, or a benzyl, whereas the compounds have a coronary dilating or sedative activity,
(11) US3,753,981 and German Patent Publication No. 2,140,280 disclose compounds of general formula (Ib) described hereinafter wherein B is a benzene ring, and R^{4b} is a styryl or 2-(5-nitro-2-furyl)vinyl, whereas the compounds have an antiinflammatory or antibiotic activity, and
(12) Eur. J. Med. Chem. (1996), 31(5), 417-425, discloses compounds of general formula (Ib) described hereinafter wherein B is a benzene ring, W is CH, and R² and R³ are bonded together with an adjacent N atom to form -(piperidinyl which may have one or more substituents) or -(piperazinyl which may have one or more substituents), as compounds working as a benzodiazepine receptor ligand, US4,560,692 discloses them as those having a spasmolytic and ataractic activity, and Japanese Patents KOKAI (Laid-Open) No. 2-129169 discloses them as those having a lipoperoxidation inhibiting activity.

Furthermore, compounds of general formula (Ib) described hereinafter wherein B is a pyridine ring and n is 0 are disclosed in Japanese Patent KOKAI (Laid-Open) No. 51-138689 (antiparasitics), Japanese Patent KOKAI (Laid-Open) No. 56-120768 (a dye component for thermosensitive recording materials), Antimicrob. Agents Chemother., (1975), 8 (2), 216-19 (an antibacterial activity), Cancer Res. (1975), 35 (12), 3611-17 (a mutagenic activity), CA 64: 19608c, Collect. Czech. Chem. Commun., (1994), 59 (6), 1463-6, US5,304,554 (an anti-HIV activity), Chem. Pharm. Bull., (1982), 30(6), 1974-9, and J. Heterocycl. Chem. (1980), 17(5), 1029-34. However, none of the prior publications teach or even suggest the PI3K inhibiting activity and carcinostatic activity.

### SUMMARY OF THE INVENTION

The present inventors have performed extensive investigations on compounds with a PI3K inhibiting activity. As a result, it has been found that novel fused heteroaryl derivatives have an excellent PI3K inhibiting activity as well as a cancer cell growth inhibiting activity. Based on the finding, it has been discovered that the fused heteroaryl derivatives could be excellent PI3K inhibitors and antitumor agents. The present invention has thus been achieved. Therefore, the present invention provides a pharmaceutical compositions for use as PI3K inhibitors or antitumor agents, comprising a fused heteroaryl derivative represented by general formula (I) below or a salt thereof and a pharmaceutically acceptable carrier: 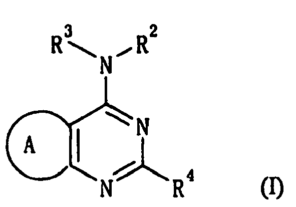 wherein: 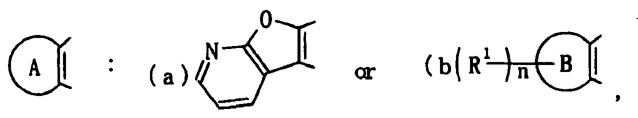 B represents a benzene ring, or pyridine, pyrazine or thiophene rings;
R¹ represents -C₁₋₆ alkyl, -alkenyl of up to 6 carbon atoms, -alkynyl of up to 6 carbon atoms, -C₃₋₈ cycloalkyl, -C₆₋₁₄ aryl which may have 1 to 5 substituents selected from Group A, -a heteroaryl which is a 5- or 6-membered monocyclic heteroaryl containing 1 to 4 hetero atoms selected from N, S and O or a bicyclic heteroaryl made of a monocyclic heteroaryl fused to a benzene ring, which may be partially saturated, and which may have 1 to 5 substituents selected from Group A, -a halogen, - NO₂, -CN, -a halogenated C₁₋₆ alkyl, -ORb, -SRb, -SO₂-Rb, -SO-Rb, -COORb, -CO-Rb, -CONRaRb, -SO₂NRaRb, -NRaRb, -NRa-CORb, - NRa-SO₂Rb, -O-CO-NRaRb or -NRaCO-COORb, -CO-a nitrogen-containing saturated heterocyclic group which is a 5- to 7-membered heterocyclic group containing one or two nitrogen atoms on the ring, and which may further contain one O or S atom and may form a bridge structure or may be fused with one benzene ring, -CONRa-C₁₋₆ alkylene-ORb, -CONRa-C₁₋₆ alkylene-NRbRc, -O-C₁₋₆ alkylene-ORb, -O-C₁₋₆ alkylene-O-C₁₋₆ alkylene-ORb, -O-C₁₋₆ alkylene-NRaRb, -O-C₁₋₆ alkylene-O-C₁₋₆ alkylene-NRaRb, - O-C₁₋₆ alkylene-NRc-C₁₋₆ alkylene-NRaRb, -NRc-C₁₋₆ alkylene-NRaRb, -N(C₁₋₆ alkylene-NRaRb)₂, -CONRa-ORb, -NRa-CO-NRbRc, or - OCORb;
Group A comprises of -C₁₋₆ alkyl, -alkenyl of up to 6 carbon atoms, -alkynyl of up to 6 carbon atoms, -a halogen, -a halogenated C₁₋₇ alkyl, -C₁₋₆ alkylene-OR, -NO₂, -CN, =O, -OR, - O-a halogenated C₁₋₆ alkyl, -O-C₁₋₆ alkylene-NRR' , -O-C₁₋₆ alkylene-OR, -O-C₁₋₆ alkylene- C₆₋₁₄ aryl, -SR, -SO₂-C₁₋₆ alkyl, - SO-C₁₋₆ alkyl, -COOR, -COO-C₁₋₆ alkylene- C₆₋₁₄ aryl, -COR, -CO- C₆-₁₄ aryl, - C₆₋₁₄ aryl, -CONRR' , -SO₂NRR' , -NRR' , -NR" -C₁₋₆ alkylene-NRR' , -NR' -C₁₋₆ alkylene-OR, -NR-C₁₋₆ alkylene- C₆₋₁₄ aryl, -NRCO-C₁₋₆ alkyl, -NRSO₂-C₁₋₆ alkyl, -C₃₋₈ cycloalkyl and - C₃₋₈ cycloalkenyl;
R, R' and R", which may be the same or different, represent H or C₁₋₆ alkyl;
R² and R³ are combined together with the N atom adjacent thereto to form a nitrogen-containing saturated heterocyclic group as -NR²R³ selected from a group consisting of 1-pyrrolidinyl, 1-piperazinyl, piperidino and morpholino, and which may have 1 to 2 substituents selected from the group comprising of -OH, =O and -C₁₋₆ alkyl;
each of Ra and Rc, which may be the same or different, represents -H or -C₁₋₆ alkyl;
Rb represents -H, -C₁₋₆ alkyl, -C₃₋₈ cycloalkyl, -C₆₋₁₄ aryl which may have 1 to 5 substituents selected from Group A or -a heteroaryl which is a 5- or 6-membered monocyclic heteroaryl containing 1 to 4 hetero atoms selected from N, S and **O** or a bicyclic heteroaryl made of a monocyclic heteroaryl fused to a benzene ring, which may be partially saturated, and which may have 1 to 5 substituents selected from Group A;
n represents 0, 1, 2 or 3, whereas n represents 1, 2 or 3 when B represents a benzene ring;
R⁴ represents -C₆₋₁₄ aryl which may have 1 to 5 substituents selected from Group A⁴, -C₁₋₆ alkylene- C₆₋₁₄ aryl which may have 1 to 5 substituents selected from Group A⁴, - alkenylene of up to 6 carbon atoms -C₆₋₁₄ aryl which may have 1 to 5 substituents selected from Group A⁴, -alkynylene of up to 6 carbon atoms -C₆₋₁₄ aryl which may have 1 to 5 substituents selected from Group A⁴, -C₃₋₈ cycloalkyl which may have 1 to 5 substituents selected from Group A, -C₃₋₈ cycloalkenyl which may have 1 to 5 substituents selected from Group A, -C₁₋₆ alkylene-C₃₋₈ cycloalkyl which may have 1 to 5 substituents selected from Group A, -alkenylene of up to 6 carbon atoms -C₃₋₈ cycloalkyl which may have 1 to 5 substituents selected from Group A, -C₁₋₆ alkylene-a nitrogen-containing saturated heterocyclic group which is a 5- to 7-membered heterocyclic group containing one or two nitrogen atoms on the ring, and which may further contain one O or S atom and may form a bridge structure or may be fused with one benzene ring, and which may have 1 to 5 substituents selected from Group A, -alkenylene of up to 6 carbon atoms -a nitrogen-containing saturated heterocyclic group which is a 5- to 7-membered heterocyclic group containing one or two nitrogen atoms on the ring, and which may further contain one O or S atom and may form a bridge structure or may be fused with one benzene ring, and which may have 1 to 5 substituents selected from Group A, -a heteroaryl which is a 5-or 6-membered monocyclic heteroaryl containing 1 to 4 hetero atoms selected from N, S and O or a bicyclic heteroaryl fused said monocyclic heteroaryl to a benzene ring, which may be partially saturated, and which may have 1 to 5 substituents selected from Group A⁴, -C₁₋₆ alkylene-a heteroaryl which is a 5- or 6-membered monocyclic heteroaryl containing 1 to 4 hetero atoms selected from N, S and O or a bicyclic heteroaryl made of a monocyclic heteroaryl fused to a benzene ring, which may be partially saturated, and which may have 1 to 5 substituents selected from Group A⁴, or -alkenylene of up to 6 carbon atoms -a heteroaryl which is a 5- or 6-membered monocyclic heteroaryl containing 1 to 4 hetero atoms selected from N, S and O or a bicyclic heteroaryl fused said monocyclic heteroaryl to a benzene ring, which may be partially saturated, and which may have 1 to 5 substituents selected from Group A⁴ ;
Group A⁴ comprises of a): -C₁₋₆ alkyl, -alkenyl of up to 6 carbon atoms, -alkynyl of up to 6 carbon atoms, -a halogen, -a halogenated C₁₋₆ alkyl, -C₁₋₆ alkylene-OR, -NO₂, -CN, =O, -O-halogenated C₁₋₆ alkyl, -SO₂-C₁₋₆ alkyl, -SO-C₁₋₆ alkyl, - COOR, -COO-C₁₋₆ alkylene-C₆₋₁₄ aryl, -COR, -CO- C₆₋₁₄ aryl, - CONRR', -SOµ₂NRR', -Cyc or -Alp-Cyc, wherein Alp represents C₁₋₆ alkylene, alkenylene of up to 6 carbon atoms or alkynylene of up to 6 carbon atoms, and Cyc represents C₆₋₁₄ aryl which may have 1 to 5 substituents selected from Group A; a heteroaryl which is a 5- or 6-membered monocyclic heteroaryl containing 1 to 4 hetero atoms selected from N, S and O or a bicyclic heteroaryl made of a monocyclic heteroaryl fused to a benzene ring, which may be partially saturated, and which may have 1 to 5 substituents selected from Group A; a nitrogen-containing saturated heterocyclic group which is a 5- to 7-membered heterocyclic group containing one or two nitrogen atoms on the ring, and which may further contain one O or S atom and may form a bridge structure or may be fused with one benzene ring, and which may have 1 to 5 substituents selected from Group A; C₃₋₈ cycloalkyl which may have 1 to 5 substituents selected from Group A; or C₃₋₈ cycloalkenyl which may have 1 to 5 substituents selected from Group A,
b): -NR-E-F, wherein E represents -CO-, -COO-, -CONR'-, - SO₂NR' - or -SO₂-; F represents -Cyc or -C₁₋₆ alkyl, alkenyl of up to 6 carbon atoms or alkynyl of up to 6 carbon atoms, which may be substituted by one or more substituents selected from the group comprising of -a halogen, -NO₂, -CN, -OR, -O-C₁₋₆ alkylene-NRR' , -O-C₁₋₆ alkylene-OR, -SR, -SO₂-C₁₋₆ alkyl, -SO-C₁₋₆ alkyl, -COOR, -COR, -CO- C₆₋₁₄ aryl, -CONRR' , -SO₂NRR', -NRCO-C₁₋₆ alkyl, -NRR', -N'R' -C₁₋₆ alkylene-OR, -NR"-C₁₋₆ alkylene-NRR' and -Cyc and
c) : -Z-R' , -Z-Cyc, -Z-Alp-Cyc, -Z-Alp-Z'-R' or -Z-Alp-Z'-Cyc, wherein each of Z and Z', which may be the same or different, independently represents O, S or NR;
   with the proviso that when 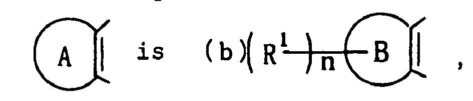 R² and R³ forms -NR²R³ which is -morpholino, and R⁴ represents -C₆₋₁₄ aryl which may have 1 to 5 substituents selected from Group A⁴ or -a heteroaryl which is a 5- or 6-membered monocyclic heteroaryl containing 1 to 4 hetero atoms selected from N, S and O or a bicyclic heteroaryl fused said monocyclic heteroaryl to a benzene ring, which may be partially saturated, and which may have 1 to 5 substituents selected from Group A⁴, and that the following compounds are excluded:
   (1) 4-(4-morpholinyl)-2-phenylpyrido[2,3-d]pyrimidine,
   (2) 4-(4-morpholinyl)-2-phenylpyrido[2,3-d]pyrimidin-7(1H)-one,
   (3) 4-(4-morpholinyl)-2-pheny-6-quinazolinol and 6-methoxy-4-(4-morpholinyl)-2-phenyquinazoline,
   (4) compounds in which B represents a benzene ring, n is 2 or 3, existing R¹'s all represent -OMe, and R⁴ is an unsubstituted phenyl or a phenyl which is substituted by 1 to 3 substituents selected from -halogen, -NO₂, -C₁₋₆ alkyl, -O-C₁₋₆ alkyl, -a hanogenated C₁₋₆ alkyl and -CONRaRc,
   (5) compounds in which B represents a benzene ring, n is 1, R¹ represents -halogen or -a lower alkyl, and R⁴ represents -(imidazolyl which may have one or more substituents),
   (6) compounds in which B represents a pyridine ring, and R⁴ represents an unsubstituted phenyl or an unsubstituted pyridyl,
   (7) compounds in which B represents a pyrazine ring, and R⁴ represents an unsubstituted phenyl, and
   (8) 4-(4-morpholinyl)-2-phenylthieno[2,3-d]pyrimidine. The same applies hereinbelow.

The present invention further relates to a novel fused heteroaryl derivative represented by general formula (Ia) or salts thereof, as well as a novel pharmaceutical composition comprising the same and a pharmaceutically acceptable carrier: 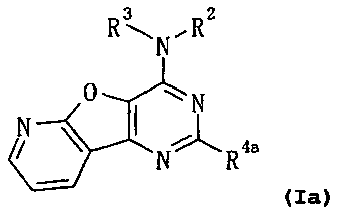 wherein:
; R² and R³ are combined together with the N atom adjacent thereto to form a nitrogen-containing saturated heterocyclic group as -NR²R³ selected from a group consisting of 1-pyrrolidinyl, 1-piperazinyl, piperidino and morpholino, and which may have 1 to 2 substituents selected from the group comprising of -OH, =O and -C₁₋₆ alkyl;
R^{4a} represents - C₆₋₁₄ aryl which may have 1 to 5 substituents selected from Group A⁴, -C₁₋₆ alkylene-C₆₋₁₄ aryl which may have 1 to 5 substituents selected from Group A⁴, alkenylene of up to 6 carbon atoms -C₆₋₁₄ aryl which may have 1 to 5 substituents selected from Group A⁴, -alkynylene of up to 6 carbon atoms - C₆₋₁₄ aryl which may have 1 to 5 substituents selected from Group A⁴, -C₃₋₈ cycloalkyl which may have 1 to 5 substituents selected from Group A, -C₃₋₈ cycloalkenyl which may have 1 to 5 substituents selected from Group A, -C₁₋₆ alkylene-C₃₋₈ cycloalkyl which may have 1 to 5 substituents selected from Group A, -alkenylene of up to 6 carbon atoms -C₃₋₈ cycloalkyl which may have 1 to 5 substituents selected from Group A, -C₁₋₆ alkylene-a nitrogen-containing saturated heterocyclic group which is a 5- to 7-membered heterocyclic group containing one or two nitrogen atoms on the ring, and which may further contain one O or S atom and may form a bridge structure or may be fused with one benzene ring, and which may have 1 to 5 substituents selected from Group A, -alkenylene of up to 6 carbon atoms -a nitrogen-containing saturated heterocyclic group which is a 5- to 7-membered heterocyclic group containing one or two nitrogen atoms on the ring, and which may further contain one O or S atom and may form a bridge structure or may be fused with one benzene ring, and which may have 1 to 5 substituents selected from Group A, -a heteroaryl which is a 5-or 6-membered monocyclic heteroaryl containing 1 to 4 hetero atoms selected from N, S and O or a bicyclic heteroaryl fused said monocyclic heteroaryl to a benzene ring, which may be partially saturated, and which may have 1 to 5 substituents selected from Group A⁴, -C₁₋₆ alkylene-a heteroaryl which is a 5- or 6-membered monocyclic heteroaryl containing 1 to 4 hetero atoms selected from N, S and O or a bicyclic heteroaryl fused said monocyclic heteroaryl to a benzene ring, which may be partially saturated, and which may have 1 to 5 substituents selected from Group A⁴, or -alkenylene of up to 6 carbon atoms -a heteroaryl which is a 5- or 6-membered monocyclic heteroaryl containing 1 to 4 hetero atoms selected from N, S and O or a bicyclic heteroaryl made of a monocyclic heteroaryl fused to a benzene ring, which may be partially saturated, and which may have 1 to 5 substituents selected from Group A⁴;
Group A comprises of -C₁₋₆ alkyl, -alkenyl of up to 6 carbon atoms, -alkynyl of up to 6 carbon atoms, -a halogen, -a halogenated C₁₋₆ alkyl, -C₁₋₆ alkylene-OR, -NO₂, -CN, =O, -OR, - O-a halogenated C₁₋₆ alkyl, -O-C₁₋₆ alkylene-NRR' , -O-C₁₋₆ alkylene-OR, -O-C₁₋₆ alkylene-C₆₋₁₄ aryl, -SR, -SO₂-C₁₋₆ alkyl, - SO-C₁₋₆ alkyl, -COOR, -COO-C₁₋₆ alkylene- C₆₋₁₄ aryl, -COR, -CO- C₆₋₁₄ aryl, -C₆₋₁₄ aryl, -CONRR' , -SO₂NRR' , -NRR' , -NR"-C₁₋₆ alkylene-NRR', -NR'-C₁₋₆ alkylene-OR, -NR-C₁₋₆ alkylene-C₁₋₆ aryl, -NRCO-C₁₋₆ alkyl, -NRSO₂-C₁₋₆ alkyl, -C₃₋₈ cycloalkyl and - C₃₋₈ cycloalkenyl;
R, R' and R" , which may be the same or different, represent H or C₁₋₆ alkyl ; and,
Group A⁴ comprises of a) : -C₁₋₆ alkyl, -alkenyl of up to 6 carbon atoms, -alkynyl of up to 6 carbon atoms, -a halogen, -a halogenated C₁₋₆ alkyl, -C₁₋₆ alkylene-OR, -NO₂, -CN, =O, -O-halogenated C₁₋₆ alkyl, -SO₂-C₁₋₆ alkyl, -SO-C₁₋₆ alkyl, - COOR, -COO-C₁₋₆ alkylene-C₆₋₁₄ aryl, -COR, -CO-C₆₋₁₄ aryl, - CONRR', -SO₂NRR', -Cyc or -Alp-Cyc, wherein Alp represents C₁₋₆ alkylene, alkenylene of up to 6 carbon atoms or alkynylene of up to 6 carbon atoms, and Cyc represents C₆₋₁₄ aryl which may have 1 to 5 substituents selected from Group A, a heteroaryl which is a 5- or 6-membered monocyclic heteroaryl containing 1 to 4 hetero atoms selected from N, S and O or a bicyclic heteroaryl fused said monocyclic heteroaryl to a benzene ring, which may be partially saturated, and which may have 1 to 5 substituents selected from Group A, a nitrogen-containing saturated heterocyclic group which is a 5- to 7-membered heterocyclic group containing one or two nitrogen atoms on the ring, and which may further contain one O or S atom and may form a bridge structure or may be fused with one benzene ring, and which may have 1 to 5 substituents selected from Group A, C₃₋₈ cycloalkyl which may have 1 to 5 substituents selected from Group A, or C₃₋₈ cycloalkenyl which may have 1 to 5 substituents selected from Group A,
   b): -NR-E-F, wherein E represents -CO-, -COO-, -CONR'-, - SO₂NR' - or -SO₂-; F represents -Cyc or -C₁₋₆ alkyl, alkenyl of up to 6 carbon atoms or alkynyl of up to 6 carbon atoms, which may be substituted by one or more substituents selected from the group comprising of -a halogen, -NO₂, -CN, -OR, -O-C₁₋₆ alkylene-NRR' , -O-C₁₋₆ alkylene-OR, -SR, -SO₂-C₁₋₆ alkyl, -SO-C₁₋₆ alkyl, -COOR, -COR, -CO- C₆₋₁₄ aryl, -CONRR' , -SO₂NRR' , -NRCO-C₁₋₆ alkyl, -NRR', -NR'-C₁₋₆ alkylene-OR, -NR"-C₁₋₆ alkylene-NRR' and -Cyc; and
   c): -Z-R', -Z-Cyc, -Z-Alp-Cyc, -Z-Alp-Z'-R' or -Z-Alp-Z'-Cyc, wherein each of Z and Z', which may be the same or different, independently represents O, S or NR. The same applies hereinbelow.

The present invention also provides a fused heteroaryl derivative represented by general formula (Ib) or salts thereof, as well as a novel pharmaceutical composition comprising the same and a pharmaceutically acceptable carrier:or a salt thereof: 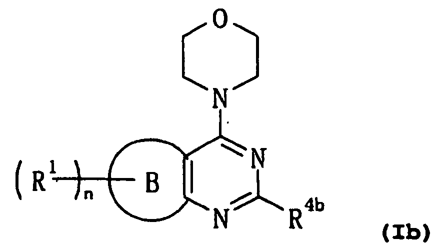 wherein:
B represents a benzene ring, or pyridine, pyrazine or thiophene rings;
R¹ represents -C₁₋₆ alkyl, -alkenyl of up to 6 carbon atoms, -alkynyl of up to 6 carbon atoms, -C₃₋₈ cycloalkyl, -C₆₋₁₄ aryl which may have 1 to 5 substituents selected from Group A, -a heteroaryl which is a 5- or 6-membered monocyclic heteroaryl containing 1 to 4 hetero atoms selected from N, S and **O** or a bicyclic heteroaryl fused said monocyclic heteroaryl to a benzene ring, which may be partially saturated, and which may have 1 to 5 substituents selected from Group A, -a halogen, - NO₂, -CN, -a halogenated C₁₋₆ alkyl, -ORb, -SRb, -SO₂-Rb, -SO-Rb, -COORb, -CO-Rb, -CONRaRb, -SO₂NRaRb, -NRaRb, -NRa-CORb, - NRa-SO₂Rb, -O-CO-NRaRb, -NRaCO-COORb, -NRaCOORb, -NRaCO-C₁₋₆ alkylene- C₆₋₁₄ aryl, -NRa-SO₂-C₁₋₆ alkylene- C₆₋₁₄ aryl, -NRa-C₁₋₆ alkylene- C₆₋₁₄ aryl, -C₁₋₆ alkylene-ORb, -C₁₋₆ alkylene-NRaRb, - CO-a nitrogen-containing saturated heterocyclic group which is a 5- to 7-membered heterocyclic group containing one or two nitrogen atoms on the ring, and which may further contain one O or S atom and may form a bridge structure or may be fused with one benzene ring, -CONRa-C₁₋₆ alkylene-ORb, -CONRa-C₁₋₆ alkylene-NRcRb, -CONRa-C₁₋₆ alkylene-a nitrogen-containing saturated heterocyclic group which is a 5- to 7-membered heterocyclic group containing one or two nitrogen atoms on the ring, and which may further contain one **O** or S atom and may form a bridge structure or may be fused with one benzene ring, -O-C₁₋₆ alkylene-ORb, -O-C₁₋₆ alkylene-NRaRb, -O-C₁₋₆ alkylene-a nitrogen-containing saturated heterocyclic group which is a 5-to 7-membered heterocyclic group containing one or two nitrogen atoms on the ring, and which may further contain one O or S atom and may form a bridge structure or may be fused with one benzene ring, -O-C₁₋₆ alkylene-O-C₁₋₆ alkylene-ORb, -O-C₁₋₆ alkylene-O-C₁₋₆ alkylene-NRaRb, -O-C₁₋₆ alkylene-NRc-C₁₋₆ alkylene-NRaRb, -NRc-C₁₋₆ alkylene-NRaRb, -N(C₁₋₆ alkylene-NRaRb)₂, -CONRa-ORb, -NRa-CO-NRbRc, or -OCORb;
Group A comprises of -C₁₋₆ alkyl, -alkenyl of up to 6 carbon atoms, -alkynyl of up to 6 carbon atoms, -a halogen, -a halogenated C₁₋₆ alkyl, -C₁₋₆ alkylene-OR, -NO₂, -CN, =O, -OR, - O-a halogenated C₁₋₆ alkyl, -O-C₁₋₆ alkylene-NRR' , -O-C₁₋₆ alkylene-OR, -O-C₁₋₆ alkylene-C₆₋₁₄ aryl, -SR, -SO₂-C₁₋₆ alkyl, - SO-C₁₋₆ alkyl, -COOR, -COO-C₁₋₆ alkylene- C₆₋₁₄ aryl, -COR, -CO- C₆₋₁₄ aryl, - C₆₋₁₄ n aryl, -CONRR' , -SO₂NRR' , -NRR' , -NR"-C₁₋₆ alkylene-NRR' , -NR'-C₁₋₆ alkylene-OR, -NR-C₁₋₆ alkylene-C₆₋₁₄ aryl, -NRCO-C₁₋₆ alkyl, -NRSO₂-C₁₋₆ alkyl, -C₃₋₈ cycloalkyl and - C₃₋₈ cycloalkenyl;
R, R' and R" , which may be the same or different, represent H or C₁₋₆ alkyl ; ;
Ra and Rc, which may be the same or different, represent -H or -C₁₋₆ alkyl;
Rb represents -H, -C₁₋₆ alkyl, -C₃₋₈ cycloalkyl, - C₆₋₁₄ aryl which may have 1 to 5 substituents selected from Group A or -a heteroaryl which is a 5- or 6-membered monocyclic heteroaryl containing 1 to 4 hetero atoms selected from N, S and O or a bicyclic heteroaryl made of a monocyclic heteroaryl fused to a benzene ring, which may be partially saturated, and which may have 1 to 5 substituents selected from Group A;
n represents 0,1, 2 or 3, whereas n represents 1, 2 or 3 when B represents a benzene ring
R^{4b} represents -C₆₋₁₄ aryl which may have 1 to 5 substituents selected from Group A⁴ or -a heteroaryl which is a 5- or 6-membered monocyclic heteroaryl containing 1 to 4 hetero atoms selected from N, S and O or a bicyclic heteroaryl made of a monocyclic heteroaryl fused to a benzene ring, which may be partially saturated, and which may have 1 to 5 substituents selected from Group A⁴ and,
Group A⁴ comprises of a) : -C₁₋₆ alkyl , -alkenyl of up to 6 carbon atoms, -alkynyl of up to 6 carbon atoms, -a halogen, -a halogenated C₁₋₆ alkyl, -C₁₋₆ alkylene-OR, -NO₂, -CN, =O, -O-halogenated C₁₋₆ alkyl, -SO₂-C₁₋₆ alkyl, -SO-C₁₋₆ alkyl, - COOR, -COO-C₁₋₆ alkylene-C₆₋₁₄ aryl, -COR, -CO-C₆₋₁₄ aryl , - CONRR', -SO₂NRR', -Cyc or -Alp-Cyc, wherein Alp represents C₁₋₆ alkylene, alkenylene of up to 6 carbon atoms or alkynylene of up to 6 carbon atoms, and Cyc represents C₆₋₁₄ aryl which may have 1 to 5 substituents selected from Group A; a heteroaryl which is a 5- or 6-membered monocyclic heteroaryl containing 1 to 4 hetero atoms selected from N, S and O or a bicyclic heteroaryl made of a monocyclic heteroaryl fused to a benzene ring, which may be partially saturated, and which may have 1 to 5 substituents selected from Group A; a nitrogen-containing saturated heterocyclic group which is a 5- to 7-membered heterocyclic group containing one or two nitrogen atoms on the ring, and which may further contain one O or S atom and may form a bridge structure or may be fused with one benzene ring, and which may have 1 to 5 substituents selected from Group A; C₃₋₈ cycloalkyl which may have 1 to 5 substituents selected from Group A; or C₃₋₈ cycloalkenyl which may have 1 to 5 substituents selected from Group A,
   b): -NR-E-F, wherein E represents -CO-, -COO-, -CONR'-, - SO₂NR' - or -SO₂-; F represents -Cyc or -C₁₋₆ alkyl, alkenyl of up to 6 carbon atoms or alkynyl of up to 6 carbon atoms, which may be substituted by one or more substituents selected from the group comprising of -a halogen, -NO₂, -CN, -OR, -O-C₁₋₆ alkylene-NRR', -O-C₁₋₆ alkylene-OR, -SR, -SO₂-C₁₋₆ alkyl, -SO-C₁₋₆ alkyl, -COOR, -COR, -CO-C₆₋₁₄ aryl, -CONRR', -SO₂NRR', -NRCO-C₁₋₆ alkyl, -NRR' , -NR'-C₁₋₆ alkylene-OR, -NR"-C₁₋₆ alkylene-NRR' and -Cyc, and
   c): -Z-R', -Z-Cyc, -Z-Alp-Cyc, -Z-Alp-Z' -R' or -Z-Alp-Z'-Cyc, wherein each of Z and Z', which may be the same or different, independently represents O, S or NR;
with the proviso that the following compounds are excluded:
(1) 4-(4-morpholinyl)-2-phenylpyrido[2,3-d]pyrimidine,
(2) 4-(4-morpholinyl)-2-phenylpyrido[2,3-d]pyrimidin-7(1H)-one,
(3) 4-(4-morpholinyl)-2-pheny-6-quinazolinol and 6-methoxy-4-(4-morpholinyl)-2-phenyquinazoline,
(4) compounds in which B represents a benzene ring, n is 2 or 3, existing R¹'s all represent -OMe, and R^{4b} is an unsubstituted phenyl or a phenyl which is substituted by 1 to 3 substituents selected from -halogen, -NO₂, -C₁₋₆ alkyl, -O-C₁₋₆ alkyl, -a hanogenated C₁₋₆ alkyl and -CONRaRc,
(5) compounds in which B represents a benzene ring, n is 1, R¹ represents -halogen or -a lower alkyl, and R^{4b} represents -(imidazolyl which may have one or more substituents),
(6) compounds in which B represents a pyridine ring, and R^{4b} represents an unsubstituted phenyl or an unsubstituted pyridyl,
(7) compounds in which B represents a pyrazine ring, and R^{4b} represents an unsubstituted phenyl, and
(8) 4-(4-morpholinyl)-2-phenylthieno[2,3-d]pyrimidine. The same applies hereinbelow.

Further teaching of the present invention provides a use of said fused heteroaryl derivative or a salt thereof for producing a medicament (especially a carcinostatic agent) which inhibit PI3K.

The compounds of general formula (I), (Ia) or (Ib) are described below in more detail.

The term "C₁₋₆" throughout the specification is used to mean a straight or branched hydrocarbon chain having 1 to 6, and preferably 1 to 3 carbon atoms.

Preferred examples of the "C₁₋₆ alkyl" are an alkyl having 1 to 3 carbon atoms, more preferably methyl and ethyl. Preferred examples of the "alkenyl up to 6 carbon atoms" include vinyl, allyl, 1-propenyl, isopropenyl, 1-butanyl, 2-butenyl and 3-butenyl. Preferred examples of the "alkynyl up to 6 carbon atoms" include ethynyl, 1-propynyl, 2-propynyl, 1-butynyl, 2-butynyl, 3-butynyl and 1-methyl-2-propynyl. The terms "C₁₋₆ alkylene", "alkenylene up to 6 carbon atoms" and "alkynylene up to 6 carbon atoms" are used to mean bivalent groups of the C₁₋₆ alkyl, alkenyl up to 6 carbon atoms and alkynyl up to 6 carbon atoms described above. Preferred examples of these groups are methylene, ethylene, vinylene, propenylene, ethynylene and propynylene. The terms "C₃₋₈ cycloalkyl" and "C₃₋₈ cycloalkenyl" refer to cycloalkyl and cycloalkenyl groups having 3 to 8 carbon atoms. Preferred examples of these groups include cyclopropyl, cyclopentyl, cyclohexyl and cyclopentenyl.

Examples of the "halogen" are F, Cl, Br and I. Examples of the "halogenated C₁₋₆ alkyl" are the aforementioned C₁₋₆ alkyl groups which are further substituted with one or more halogen atoms described above, preferably CF₃ .

Examples of "a nitrogen-containing saturated heterocyclic group which is a 5- to 7-membered.heterocyclic group containing one or two nitrogen atoms on the ring, and which may further contain one O or S atom and may form a bridge structure or may be fused with one benzene ring" are pyrrolidinyl, piperazinyl, piperidyl and morpholinyl. Preferred examples of the nitrogen-containing saturated heterocyclic group as -NR²R³ are 1-pyrrolidinyl, 1-piperazinyl, piperidino and morpholino, with particular preference to morpholino.

The term "C₆₋₁₄ aryl" is used throughout the specification to mean an aromatic cyclic hydrocarbon group having 6 to 14 carbon atoms. Preferred examples of such aryl are phenyl and naphthyl.

The term "heteroaryl which is a 5- or 6-membered monocyclic heteroaryl containing 1 to 4 hetero atoms selected from N, S and O or a bicyclic heteroaryl made of a monocyclic heteroaryl fused to a benzene ring, which may be partially saturated", preferred examples of the monocyclic heteroaryl are furyl, thienyl, pyrrolyl, imidazolyl, pyrazolyl, thiazolyl, isothiazolyl, oxazolyl, triazolyl, tetrazolyl, pyridyl, pyrimidinyl, pyridazinyl and pyrazinyl. Examples of the bicyclic heteroaryl are preferably benzofuranyl, benzothienyl, benzothiadiazolyl, benzothiazolyl, benzimidazolyl, indolyl, isoindolyl, indazolyl, quinolyl, isoquinolyl, cinnolinyl, quinazolinyl, quinoxalinyl and benzodioxolyl. Specific examples of the partially saturated heteroaryl are 1,2,3,4-tetrahydroquinolyl, etc. Particularly preferred are 5- to 6-membered monocyclic groups, more preferably imidazolyl, thiazolyl, triazolyl, pyridyl and pyrazinyl.

Examples of a "5- or 6-membered monocyclic heteroaryl containing 1 or 2 hetero atoms selected from O, S and N" in B include a furan, thiophene, pyrole, imidazole, pyrazole, thiazole, isothiazole, oxazole, pyridine, pyrimidine, pyridazine and pyrazine ring. Preferably, it is a pyridine, pyrazine or thiophene ring. More preferable, it is a pyridine ring.

The substituent for the "which may have 1 to 5 substituents selected from Group A" are 1 ∼ 5 substituents, which may be the same or different, and, these substituents are selected from Group A.

The particularly preferred ones of Group A⁴ are -C₁₋₆ alkylene-OR, -CONRR', -NR-CO-Cyc¹, -NR-SO₂-Cyc¹, -OR, -NRR', -0-C₁₋₆ alkylene-NRR' and -O-C₁₋₆ alkylene-a nitrogen-containing saturated heterocyclic group which is a 5- to 7-membered heterocyclic group containing one or two nitrogen atoms on the ring, and which may further contain one O or S atom and may form a bridge structure or may be fused with one benzene ring, and which may have 1 to 5 substituents selected from Group A; wherein Cyc¹ is - C₆₋₁₄ aryl which may have 1 to 5 substituents selected from Group A; -a heteroaryl which is a 5- or 6-membered monocyclic heteroaryl containing 1 to 4 hetero atoms, selected from N, S and O or a bicyclic heteroaryl made of a monocyclic heteroaryl fused to a benzene ring, which may be partially saturated, and which may have 1 to 5 substituents selected from Group A; or -a nitrogen-containing saturated heterocyclic group which is a 5- to 7-membered heterocyclic group containing one or two nitrogen atoms on the ring, and which may further contain one O or S atom and may form a bridge structure or may be fused with one benzene ring, and which may have 1 to 5 substituents selected from Group A.

When n is 2 to 4, each R¹ group may be the same or different, independently.

In the compounds which are shown by formulas (I), (Ia) and (Ib) of the present invention, the following compounds are preferred:
(1) Compounds in which R² and R³ forms -NR²R³ which is - morpholino;
(2) Compounds in which R^{4a} represents -C₆₋₁₄ aryl which may have 1 to 5 substituents selected from Group A⁴ or -a heteroaryl which is a 5- or 6-membered monocyclic heteroaryl containing 1 to 4 hetero atoms selected from N, S and O or a bicyclic heteroaryl fused said monocyclic heteroaryl to a benzene ring, which may be partially saturated, and which may have 1 to 5 substituents selected from Group A⁴;
(3) Compounds in which B is a pyridine, pyrazine or thiophene ring and n is 0;
and(4) Compounds in which R⁴, R^{4a} or R^{4b} represents C₆₋₁₄ aryl which has one or more substituents selected from the group comprising of -C₁₋₆ alkylene-OR, -CONRR', -NR-CO-Cyc¹, -NR-SO₂-Cyc¹, -OR, -NRR' , -O-C₁₋₆ alkylene-NRR' and -O-C₁₋₆ alkylene-a nitrogen-containing saturated heterocyclic group which is a 5-to 7-membered heterocyclic group containing one or two nitrogen atoms on the ring, and which may further contain one **O** or S atom and may form a bridge structure or may be fused with one benzene ring, and which may have 1 to 5 substituents selected from Group A.

The particularly preferred compounds shown by general formula (Ia) are those having R^{4a} which is C₆₋₁₄ phenyl having at least one substituent which is selected from of the group comprising of -OH, -NH₂, -NH-C₁₋₆ alkyl, -N(C₁₋₆alkyl)₂, -O-C₁₋₆ alkylene-NH₂ and -O-C₁₋₆ alkylene-a nitrogen-containing saturated heterocyclic group which is a 5- to 7-membered heterocyclic group containing one or two nitrogen atoms on the ring, and which may further contain one O or S atom and may form a bridge structure or may be fused with one benzene ring, and which may have 1 to 5 substituents selected from Group A.

Moreover, the following compounds shown by general formula (Ib) are particularly preferred:
(1) Compounds in which B represents a benzene ring, n is 1 or 2, and R¹ represents -a halogen, -NO₂, -CN, -a halogenated C₁₋₆ alkyl, -ORb, -SRb, -NRaRb, -NRa-CORb or -NRa-SO₂Rb; and
(2) Compounds in which B represents a pyridine, pyrazine or thiophene ring, n is 0, and R^{4b} represents a phenyl which has at least one substituent which is selected from -OH, -CH₂OH and -CONCH₂.

Among the compounds of the present invention, the preferred ones which are shown by general formula (Ia) are (Co 17) 6-amino-3'-.(4-morpholinopyrido[3',2':4,5]furo[3,2-d]pyrimidin-2-yl)nicotinanilide, (Co 33) 4-(4-morpholinopyrido[3',2':4,5]furo[3,2-d]pyrimidin-2-yl)aniline, (Co 50) 3-(4-morpholinopyrido[3',2':4,5]furo[3,2-d]pyrimidin-2-yl)phenol, (Co 69) 4-morpholino-2-[3-(2-piperazin-1-ylethoxy)phenyl]pyrido[3',2':4,5]furo[3,2-d]pyrimidine, (Co 73) 3'-(4-morpholinopyrido[3';2':4,5]furo[3,2-d]pyrimidin-2-yl)acrylanilide, and salts thereof. The preferred ones which are shown by general formula (Ib) are (Co 144) N-[2-(3-benzenesulfonylaminophenyl) -4-morphoniloquinazolin-6-yl]acetamide, (Co 164) 3-(4-morpholinopyrido[4,3-d]pyrimidin-2-yl)phenol, (Co 172) 3-(4-morpholinopyrido[3,2-d]pyrimidin-2-yl)phenol, (Co 174) 3-(4-morpholinopyrido[3,4-d]pyrimidin-2-yl)phenol, (Co 186) 3-(6-methoxy-4-morpholinoquinazolin-2-yl)phenol, (Co 190) 3-(4-morpholinothieno[3,2-d]pyrimidin-2-yl)phenol, (Co 191) 3-(4-morpholinopteridin-2-yl)phenol, and salts thereof.

The compound of this invention may exist in the form of geometrical isomers or tautomers depending on the kinds of substituent groups, and these isomers in separated forms or mixtures thereof are included in the present invention. Also, the compound of the present invention may have asymmetric carbon atoms, so that optical isomer forms may exist based on such carbon atoms. All of the mixtures and the isolated forms of these optical isomers are included in the present invention.

Some of the compounds of the invention may form salts. There is no particular limitation so long as the formed salts are pharmacologically acceptable. Specific examples of acid salts are salts with inorganic acids such as hydrochloric acid, hydrobromic acid, hydroiodic acid, sulfuric acid, nitric acid, phosphoric acid, organic acids such as formic acid, acetic acid, propionic acid, oxalic acid, malonic acid, succinic acid, fumaric acid, maleic acid, lactic acid, malic acid, tartaric acid, citric acid, methanesulfonic acid, ethanesulfonic acid, aspartic acid, glutamic acid, etc. Specific examples of basic salts include salts with inorganic bases containing metals such as sodium, potassium, magnesium, calcium, aluminum, or salts with organic bases such as methylamine, ethylamine, ethanolamine, lysine, ornithine. In addition, various hydrates and solvates and polymorphism of the compound (I), (Ia) or (Ib) and salts thereof are also included in this invention.

### (Methods for synthesizing compounds)

The following describes typical methods for synthesizing the compounds of the present invention. In this connection, depending on the kind of functional group, it may sometimes be effective from the viewpoint of synthesis techniques to replace the functional group with an appropriate protecting group, namely a group which can be easily converted into the functional group, at the stage of starting materials or synthetic intermediates. Thereafter, a desired compound can be obtained by removing the protecting group as occasion demands. Examples of such functional groups include a amino group, a hydroxyl group, a carboxyl group and examples of their protecting groups include those which are described in "Protective Groups in Organic Synthesis", 2nd edition, edited by Greene and Wuts, which may be optionally used depending on the reaction conditions.

### Synthesis Method 1

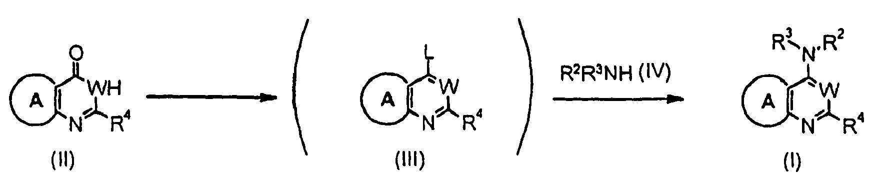

(Here and hereinafter, L represents a leaving group.)

This method for synthesizing the compound (I) of the present invention comprises converting the compound shown by general formula (II) to reactive derivative thereof (III) in a conventional manner and then reacting an amine (IV) with the reactive derivative. When another reactive site containing the leaving group L also exists on the ring A or the substituent R⁴ in the reactive derivative (III), the same or different amine (IV) may be reacted again, if necessary. In a similar manner, when the A ring or R⁴ of the compound of the present invention has a leaving group L such as a chloro or fluoro, transformations of functional groups may be conducted such as a hydrolysis reaction according to a method described in J. Am. Chem. Soc., 68, 1288 (1946) or an ipso-substitution reaction using alkoxide as a reacting agent according to a method described in Tetrahedron Lett., 40, 675 (1999).

The leaving group shown by L is preferably a halogen, or an organic sulfonyloxy group, e.g., methanesulfonyloxy, p-toluenesulfonyloxy.

The reaction for preparing the reactive derivative (III) can be carried out by the usual procedures. Where the leaving group is a chloro, the compound (II) can be reacted with phosphorus oxychloride, oxalyl chloride or thionyl chloride under cooling or heating or at room temperature in an inert organic solvent or without solvents. As such an inert organic solvent, there is an aromatic hydrocarbon solvent such as benzene or toluene; an ethereal solvent such as tetrahydrofuran (THF) or 1,4-dioxane; a halogenated hydrocarbon solvent such as dichloromethane or chloroform; and a basic solvent such as pyridine or collidine. These solvents may be used alone or as a mixture of two or more. The solvent is optionally selected depending on the kinds of starting compounds. The addition of a base (preferably a dialkylaniline, triethylamine, ammonia, lutidine, collidine ), phosphorus chloride (e.g., phosphorus pentachloride), a quaternary ammonium salt (e.g., tetraethylammonium chloride), or an N,N-dialkylamide compound (e.g., dimethylformamide (DMF)) may be advantageous in some cases from the viewpoint of accelerating the reaction. Where the leaving group is sulfonyloxy, the active intermediate (III) can be synthesized from the corresponding sulfonyl chloride by the usual procedures, e.g., using a method described in Tetrahedron Lett. 23 (22), 2253 (1982) or Tetrahedron Lett. 27 (34), 4047 (1986).

The reaction for synthesizing the compound (I) from the reactive derivative (III) and the amine (IV) can be carried out by reacting the amine (IV) in an inert organic solvent or in the absence of any solvents under cooling or heating or at room temperature. The solvent described above is available and it may be used alone or as a mixture of two or more. The addition of an inorganic base such as sodium hydride, or an organic base such as triethylamine (TEA), pyridine or 2,6-lutidine, may be advantageous in some cases from the viewpoint of accelerating the reaction

### Synthesis Method 2

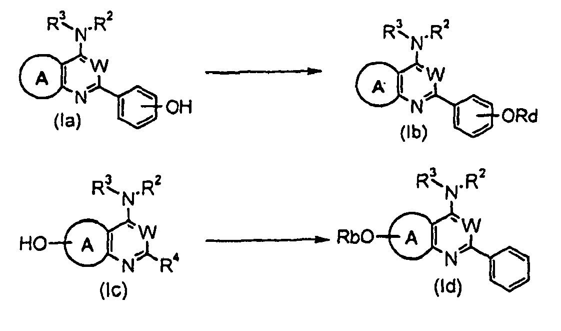

(Wherein Rd is a C₁₋₆ alkyl which may have one or more substituents and Rb has the same definition as defined above; and the same shall apply hereinafter.)

This method comprises O-alkylation of the hydroxy-substituted compound shown by general formula (Ia) or (Ic) in a conventional manner to obtain the compound (Ib) or (Id). The reaction may be carried out, e.g., by reacting the compound (Ia) or (Ic) with an alkylating agent such as an alkyl halide or a sulfonic acid ester in the presence of a base such as triethylamine, potassium carbonate, sodium carbonate, cesium carbonate, sodium hydroxide, sodium hydride or potassium t-butoxide. The reaction temperature can be under cooling or heating or at room temperature, and can be appropriately chosen depending on the kinds of starting compounds. When water is used or contained as a solvent in an O-alkylation reaction, the reaction may be accelerated by the addition of a phase transfer catalyst such as tetra n-butylammonium hydrogensulfate.

Another method for the O-alkylation reaction is Mitsunobu reaction. For example, methods described in Synthesis, 1 (1981) or modified methods may be used. For the hydroxyethylation of a hydroxyl group, methods using carbonate ester such as [1,3]dioxolane-2-one are also effective. As an example, methods described in J. Am. Chem. Soc., 68, 781 (1946) can be used.

Moreover, when functional groups exist on R^{b} and R^{d} of the compounds (Ib) and (Id) of the present invention, known reactions may be employed to convert the functional group. For example, when a hydroxyl group is present on R^{b} and R^{d}, the aforementioned O-alkylation reaction can be conducted, and when a leaving group is present such as a halogen, an appropriate alcohol or amine can be reacted with utilizing the conditions of said O-alkylation or N-alkylation described hereinafter in Synthesis Method 4. When an ester group is present, the functional group can be converted to a carboxylic acid, hydroxymethyl group, and amido, using a method described hereinafter in Synthesis Method 3.

The starting compounds (Ia) and (Ic) used in this method can be prepared by the method described for Synthesis Method 1, using starting compounds whose OH group has been protected by an acyl type protective group (e.g., acetyl or tosyl). Further, when phosphorus oxychloride is used as a reacting agent for synthesizing reactive derivative (III) and then a desired amino is reacted to synthesize the compound (I), protective group for OH group may be removed and O-phosphoramide may be produced, depending on the kind of starting compounds, a protective group, reaction conditions and conditions of after treatment. In that case, for example, using a method described in Chem. Pharm. Bull.,37, 2564 (1989), the phosphoramide group can be removed. Other general protective groups can be introduced and removed by the methods described in "Protective Groups in Organic Synthesis" supra.

### Synthesis Method 3

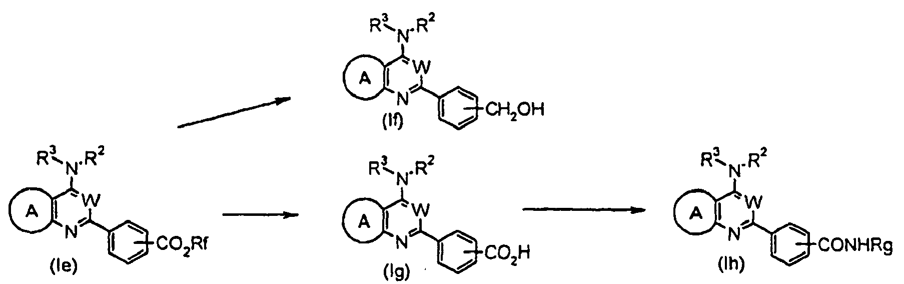

(Wherein Rf is a lower alkyl according to the claims and Rg is a lower alkyl which may have one or more substituents according to the claims, and the same shall apply hereinafter.)

Synthesis Method 3 comprises modifications of the functional group of the ester compound of the present invention shown by general formula (Ie) to produce the hydroxymethyl compound (If), carboxylic acid derivative (Ig) and amide derivative (Ih) of the present invention, respectively. Each of the reactions can be carried out in a conventional manner, e.g., as described in Jikken Kagaku Kouza (Encyclopedia for Experimental Chemistry) edited by Nihon Kagaku Kai (Japanese Association of Chemistry) and published by Maruzen Co., Ltd., and "Protective Groups in Organic Synthesis" supra.

Preferably, the reduction of the compound (Ie) to give the hydroxymethyl compound (If) can be conducted in an inert organic solvent, e.g., an ethereal solvent or an aromatic hydrocarbon solvent, using a reducing agent such as lithium aluminum hydride, lithium borohydride, zinc borohydride, boran, Vitride. The hydrolysis to give the carboxylic acid derivative (Ik) can be conducted by reacting the compound (Ie) with lithium hydroxide, sodium hydroxide or potassium hydroxide in a solvent selected from methanol, ethanol, THF and water, or a mixture of two or more. The amidation to give the amide compound (Ih) may be performed by converting carboxylic acid to reactive derivative such as acyl halide (acyl chloride) or acid anhydride, and then reacting the reactive derivative with amine. In the reaction with amine, it is preferred to conduct the reaction in an inert organic solvent in the presence of a base (an inorganic base such as sodium hydroxide, or an organic base such as TEA, diisopropylethylamine or pyridine). Furthermore, the amidation using the carboxylic acid as a starting compound can also be carried out in an inert organic solvent in the presence of a condensation agent such as (1-ethyl-3-(3-dimethylaminopropyl) carbodiimide (EDCI), 1,1'-carbonylbis-1H-imidazole (CDI), etc.). In this case, an additive such as 1-hydroxybenzotriazol (HOBt) may also be added to the reaction. The reaction temperature and solvent can be appropriately chosen depending on the kinds of starting compounds.

### Synthesis Method 4

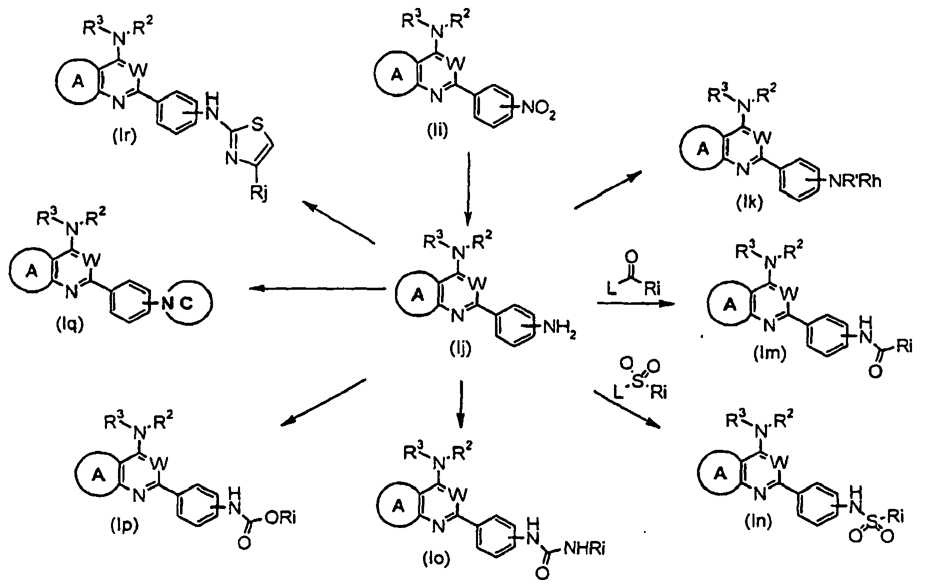

(Wherein R' has the same definition as defined above, Rh is a lower alkyl according to the claims which may have one or more substituents according to the claims Ri is -Cyc or -Alp which may have one or more substituents according to the claims, a C ring is a nitrogen-containing saturated heterocyclic group which may have one or more substituent according to the claims, and Rj is -H, -a lower alkyl according to the claims, -an aryl; and the same shall apply hereinafter.)

Synthesis Method 4 comprises the reduction of the nitro compound shown by general formula (Ii) to the corresponding amino compound (Ij) and then subjecting the amino compound (Ij) to various modification reactions including N-alkylation, amidation, sulfonamidation, conversion to the corresponding urea, conversion to the corresponding carbamic acid, imidation or conversion to the corresponding thiazole, to give the compounds (Ik), (Im), (In), (Io), (Ip), (Iq) and (Ir), respectively. These products can be appropriately subjected to further known modification reactions such as N-alkylation, if necessary.

These reactions can all be carried out in a conventional manner, e.g., using the methods described in "Jikken Kagaku Kouza" supra, or "Protective Groups in Organic Synthesis" supra. Preferred procedures in these methods are described below.

The reduction of the nitro compound can be carried out in an alcoholic solvent such as methanol in a gaseous hydrogen atmosphere using palladium on carbon (Pd-C).

When various aldehydes are employed as the starting compounds, the N-alkylation can be conducted by reductive amination using aldehydes and reducing agents such as sodium borohydride, sodium triacetoxyborohydride or sodium cyanoborohydride. Reducting amination using Dean-Stark apparatus could be useful, too. When an alkyl halide such as methyl iodide or benzyl bromide, or dimethyl sulfate is employed as an alkylating agent, the reaction can be carried out in an inert organic solvent, e.g., DMF, acetonitrile or toluene, in the presence of base such as potassium carbonate, sodium hydroxide or sodium hydride, under cooling or heating or at room temperature. Monoalkylation can be carryed out advantageously by protection of amino group by acyl group such as trifluoroacetyl, alkylation of acylamide by conventional methods using halogenated alkyl, and removal of protection. The dialkylation can be conducted by using two or more reaction equivalent amounts of halogenated alkyl. For dimethylation, the reaction with formalin in formic acid at room temperature or under heating is also useful.

The amidation reaction may be performed in a similar manner to that described above for Synthesis Method 3. The sulfonamidation can be carried out in an inert organic solvent using a reactive derivative such as an acid halide (acid chloride) or an acid anhydride. The conversion to the corresponding urea can be conducted by reacting with isocyanate in an inert organic solvent, e.g., an aromatic hydrocarbon solvent, under cooling or heating or at room temperature. The conversion to the corresponding carbamic acid can be conducted by reacting chloroformate derivative in an inert organic solvent under cooling or heating or at room temperature. The imidation can be carried out using agents such as succinic anhydride or maleic anhydride.

The conversion to the corresponding aminothiazole compound can be conducted by converting the amino compound to the corresponding thiourea derivative and then reacting the derivative with an α-halogenated ketone. Compound (Ij) can be converted into the thiourea derivative by methods described in, e.g., Synth. Commun. 1998, 28 (8), 1451; J. Org. Chem., 1984, 49 (6), 997, Org. Synth., 1963, IV, 180; J. Am. Chem. Soc., 1934, 56, 1408, etc. The conversion of the thiourea derivative into the thiazole derivative can be conducted by reacting the thiourea derivative with the α-halogenated ketone in an alcoholic solvent such as ethanol or a carbonyl solvent such as methyl ethyl ketone, under cooling or heating or at room temperature. The addition of a base (potassium carbonate, sodium carbonate) may be effective in some cases from the viewpoint of accelerating the reaction.

### Synthesis Method 5

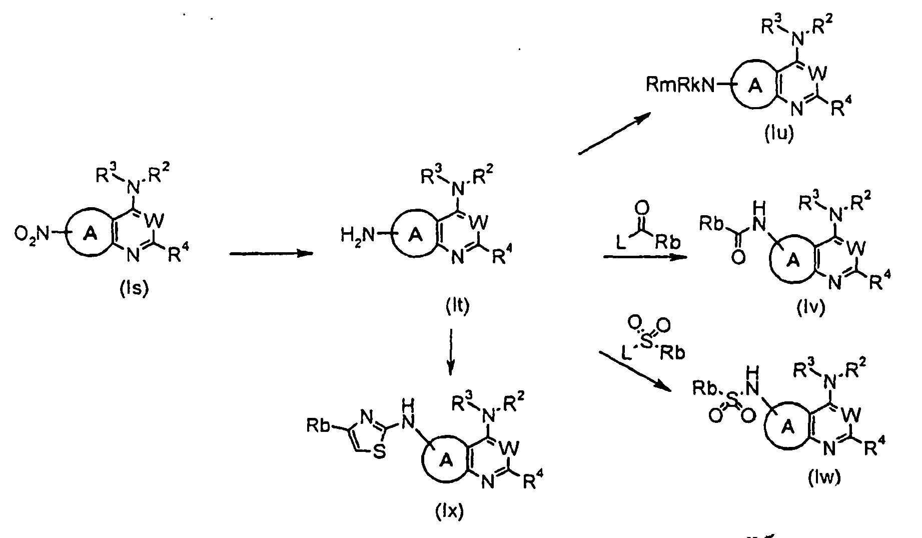

(Wherein Rk and Rm each represents -a lower alkyl according to the claims which may have one or more substituents.)

Synthesis Method 5 comprises converting the nitro compound of the invention shown by general formula (Is) to the corresponding amino compound (It) and then subjecting it to various modification reactions to obtain the other compounds of the present invention. Each reaction can be carried out as described for Synthesis Method 4.

### Other Synthesis Method

Other compounds included in the present invention can be obtained in the same manner as described above or by using methods well known to those skilled in the art. For instance, the reactions are carried out appropriately using methods described in "Jikken Kagaku Kouza" supra, or "Protective Groups in Organic Synthesis" supra.

For example, the demethylation reaction of methoxy substituted pheny derivative into the corresponding phenol derivative can be carried out by the methods described in "Protective Groups in Organic Synthesis" supra, i.e., the method of reacting with a demethylating agent such as sodium cyanide or potassium cyanide in a solvent such as dimethylsulfoxide (DMSO) at room temperature or under heating.

### Methods for synthesizing starting compounds

The starting compounds (II) for the synthesis of the present invention can be obtained in conventional manners, e.g., by the reactions shown in the following synthetic schemes.

### Synthesis Scheme 1

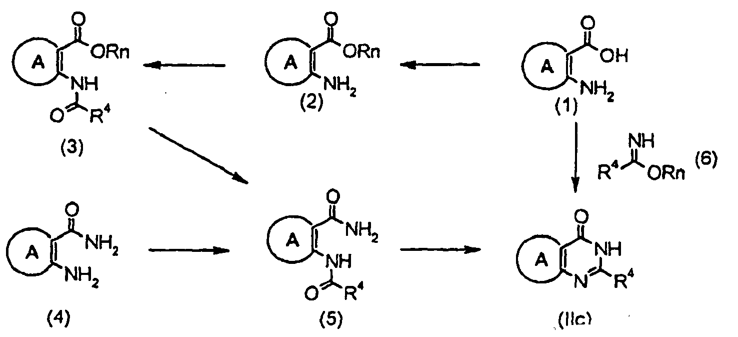

(Wherein Rn is a lower alkyl according to the claims; and the same shall apply hereinafter.)

The starting compound (IIc) can be synthesized by a cyclization reaction of amide intermediate (5) or cyclization conducted by reacting anthranylic acid derivative (1) as the starting compounds with imidate (6). Conventional cyclization reactions for preparing pyrimidine ring are available for the cyclization reaction for this purpose. For instance, the method described in Chem. Pharm. Bull., 39 (2), 352 (1991) can be used for the cyclization of the intermediate (5) and the intermediates (1) and (6) as the starting materials can be cyclized by the method described in J. Med. Chem., 9, 408 (1966). The amide intermediate (5) can be synthesized by amidation of the aniline derivative (4) in a conventional manner, or by sequential conversions of esterification of a carboxylic acid in intermediate(1), acylation of an amino, and amidation of the ester group according to conventional methods. For example, the amide intermediate (5) can be obtained in accordance with the methods described in J. Med. Chem., 33, 1722 (1990), Eur. J. Med. Chem.-Chim. Ther., 9(3), 305 (1974). When compound (3) is obtained by acylation using compound (2) as the starting materials, diacylation may take place depending on the starting compounds and reaction conditions. In such a case, treatment with basic conditions will give desired monoacyl compound (3).

### Synthesis Scheme 2

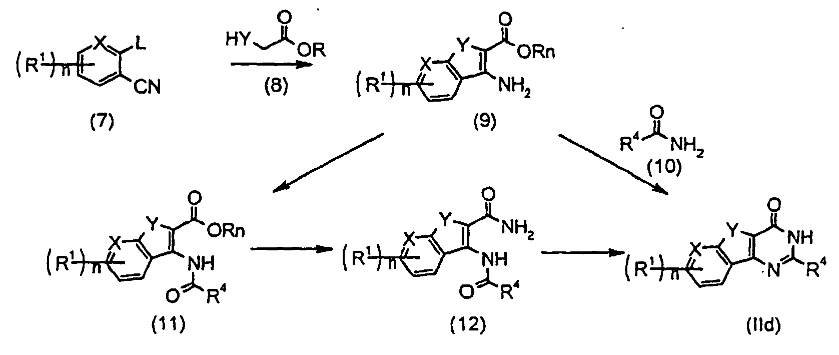

The starting compound (IId) can be synthesized by cyclization of the amide intermediate (12) or by cyclization of the ester intermediate (7) and the amide compound (10). The intermediate (12) can be cyclized in the same manner as described above; where the intermediates (7) and (10) are used as the starting compounds, the cyclization can be carried out by the method described in, e.g., J. Med. Chem., 37, 2106 (1994). The amide intermediate (12) can be prepared by conversion of the functional group in ester compound (7) in a conventional manner. The bicyclic ester intermediate (9) can be synthesized by formation of a 5-membered ring by reacting nitrile compound (7) with ester compound (8) in the presence of a base, for example, in accordance with the method described in J. Org. Chem., 37, 3224 (1972) or J. Heterocycl. Chem., 11 (6), 975 (1974), etc.

### Synthesis Scheme 3

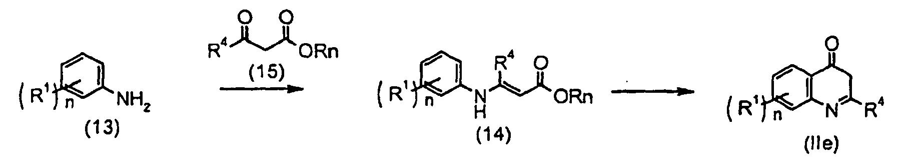

The starting compound (IIe) can be synthesized, e.g., by cyclization of the starting compound (14). Preferably, the compound (14) is heated in a solvent with a high boiling point such as diphenyl ether or in the absence of any solvents. The starting compound (14) can be synthesized in a conventional manner, e.g., by condensation of the corresponding aniline (13) with the compound (15).

Each of the reaction products obtained by the aforementioned synthesis methods is isolated and purified as a free base or a salt thereof. The salt can be produced by a usual salt forming method. The isolation and purification are carried out by applying general chemical techniques such as extraction, concentration, evaporation, crystallization, filtration, recrystallization, various types of chromatography.

Each form of isomers can be isolated by the usual procedures making use of physicochemical differences among isomers. For instance, racemic compounds can be separated by means of a conventional optical resolution method (e.g., by forming diastereomer salts with a conventional optically active acid such as tartaric acid and then optically resolving the salts) to give optically pure isomers. A mixture of diastereomers can be separated by conventional means, e.g., fractional crystallization or chromatography. In addition, an optical isomer can also be synthesized from an appropriate optically active starting compound.

### INDUSTRIAL APPLICABILITY

The compounds of the present invention exhibit a PI3K inhibitory activity and therefore, can be utilized in order to inhibit abnormal cell growths in which PI3K plays a role. Thus, the compounds are effective in the treatment of disorders with which abnormal cell growth actions of PI3K are associated, such as restenosis, atherosclerosis, bone disorders, arthritis, diabetic retinopathy, psoriasis, benign prostatic hypertrophy, atherosclerosis, inflamation, angiogenesis, immunological disorders, pancreatitis, kidney disease, cancer. In particular, the compounds of the present invention possess excellent cancer cell growth inhibiting effects and are effective in treating cancers, preferably all types of solid cancers and malignant lymphomas, and especially, leukemia, skin cancer, bladder cancer, breast cancer, uterus cancer, ovary cancer, prostate cancer, lung cancer, colon cancer, pancreas cancer, renal cancer, gastric cancer, brain tumor.

The pharmacological effect of the compounds according to the invention have been verified by the following pharmacological tests.

### Test Example 1 Inhibition of PI3K (p110α subtype)

Inhibition was determined using enzyme (bovine p110α) prepared in the baculovirus expression system. Bovine p110 was prepared according to a modification from the method by I. Hiles et al., Cell, 70, 419 (1992). Each compound to be assayed was dissolved in DMSO and the obtained 10 mM DMSO solution was serially diluted with DMSO.

The compound (0.5µl) to be assayed and enzyme were mixed in 25µl of buffer solution (40 mM Tris-HCl (pH 7.4), 200 mM NaCl, 2 mM dithiothreitol, 5 mM MgCl₂). Then, 25µl of 5 mM Tris-HCl (pH 7.4) buffered solution supplemented with 10µg PI (Sigma), 2µ Ci [γ-³²P] ATP (Amersham Pharmacia) and 80µM nonradiolabeled ATP (Sigma) was added to the mixture to initiate the reaction. After reacting at 37°C for 15 minutes, 200µl of 1M HCl and 400µl of CHCl₃ / MeOH (1 : 1) were added to the reaction mixture. The resulting mixture was stirred and then centrifuged. After the organic layer was again extracted twice with 150µl of MeOH / 1M HCl (1 : 1). The radioactivity was measured using Cerenkov light.

The IC₅₀ inhibition activity was defined by a 50% inhibition concentration of each compound assayed, which was converted from the radioactivity determined as 100% when DMSO alone was added and as 0% when no enzyme was added.

The compounds of the prevent invention exhibited an excellent p110α subtype inhibition activity. For example, IC₅₀ of Compound (hereinafter, abbreviated as Co) 10, Co 17, and Co 24 were less than 1µM.

Moreover, compounds of the prevent invention were confirmed to have inhibiting activities against other subtypes (such as C2β subtype).

### Test Example 2 Colon cancer cell growth inhibition

HCT116 cells from a colon cancer cell line were cultured in McCoy's 5A medium (GIBCO) supplemented with 10% fetal bovine serum. HCT116 cells were inoculated on a 96 well plate (5000 cells/well) followed by overnight incubation. The test compound diluted with the medium was added to the medium in a final concentration of 0.1 to 30M (final DMSO concentration, 1%). After incubation over 72 hours, Alamar Blue reagent was added to the medium. Two hours after the addition, a ratio of fluorescent intensity at an excitation wavelength of 530 nm to that at an emission wavelength of 590 nm was measured to determine the IC₅₀. Co 14, Co 24, Co 25, Co 31, Co 46 and Co 47 of the present invention exerted an excellent cancer cell growth inhibition activity.

### Test Example 3 Melanoma cell growth inhibition

A375 cells from a melanoma cell line were cultured in DMEM medium (GIBCO) supplemented with 10% fetal bovine serum. A375 cells at 10,000 cells/100µl were added to a 96 well plate which contained 1µl/well of the test compounds (final concentration of 0.001 - 30µM). After incubation for over 46 hours, Alamar Blue reagent was added to the medium (10µl/well). Two hours after the addition, a ratio of fluorescent intensity at an excitation wavelength of 530 nm to that at an emission wavelength of 590 nm was measured to determine the IC₅₀ of the test compounds in the same manner as in the above examples.

The compounds of the prevent invention exhibited an excellent cancer cell growth inhibition activity. For example, Co17, Co 33, Co 50, Co 69, Co 164, Co 172, Co 174, Co 186, Co 190 and Co 191 exerted a good melanoma cell growth inhibition activity. Their IC₅₀ values were 0.33 - 4.26 µM. Contrarily, the known PI3K inhibitor LY294002 showed a value of 8.39µM.

In addition to the above cancer cell lines, the compounds of the present invention exhibited excellent cancer cell growth inhibiting activities against Hela cells from a cervix cancer cell line, A549, H460 cells from a lung cancer cell line, COL0205,
WiDr, Lovo cells from a colon cancer cell line, PC3, LNCap cells from a prostate cancer cell line, SKOV-3, OVCAR-3, CH1 cells from an ovary cancer cell line, U87 MG cells from a glioma cell line and BxPC-3 cells from a pancreas cancer cell line.

### Test Example 4 In vivo cancer cell growth inhibition

A single-cell suspension of HelaS3 (5 x 10⁶ cells), a human cervix cancer cell line, was inoculated into the flank of female Balb/c nude mice by subcutaneously injection. When the tumor reached 100 ∼ 200 mm³ in volume, test compounds were intraperitoneally administered once a day for two weeks. 20% Hydroxypropyl-β-cyclodextrin/saline was intraperitoneally administered with the same schedule as a control group. The diameter of the tumors was measured with a vernier caliper at certain time intervals until one day after the final doze administration. The tumor volume was calculated by the following formula: 1/2 x (a shorter diameter)² x (a longer diameter).

In the present test, test compounds exhibited superior anti-tumor activities as compared with the control group.

The pharmaceutical composition of the present invention can be prepared in a conventional manner by mixing one or more compounds of the invention shown by general formula (I), (Ia) or (Ib) with a carrier for medical use, a filler and other additives usually used in pharmaceutical preparation. The pharmaceutical composition of the invention may be administered either orally in the form of tablets, pills, capsules, granules, powders, liquid, or parenterally such as by intravenous or intramuscular injection, in the form of suppositories, or through pernasal, permucosal or subcutaneous route.

For oral administration of the composition in the present invention, a solid composition in the form of, e.g., tablets, powders or granules is available. In such a solid composition, one or more active or effective ingredients are blended with at least one inert diluent such as lactose, mannitol, glucose, hydroxypropyl cellulose, microcrystalline cellulose, starch, polyvinyl pyrrolidone or magnesium aluminate metasilicate. The composition may further contain additives other than the inert diluent by the usual procedures. Examples of such additives include a lubricant such as magnesium stearate, a disintegrating agent such as calcium cellulose glycolate, a solubilization assisting agent such as glutamic acid or aspartic acid. Tablets or pills may be coated, if necessary, with films of sugar or a gastric or enteric substance such as sucrose, gelatin, hydroxypropyl cellulose, hydroxypropylmethyl cellulose phthalate.

A liquid composition for oral administration includes pharmaceutically acceptable emulsions, solutions, suspensions, syrups, elixirs, etc. and contains an inert diluent conventionally employed, e.g., purified water or ethanol. In addition to the inert diluent above, the liquid composition may further contain an auxiliary agent such as a moistening agent or a suspending agent, a sweetener, a flavor, an aromatics and/or a preservative.

A injections for parenteral administration contains a sterile aqueous or non-aqueous solution, a suspension or an emulsion. Examples of the aqueous solution and suspension include distilled water for injection use and physiological saline. Typical examples of the non-aqueous solution and suspension are propylene glycol, polyethylene glycol, vegetable oil such as olive oil, an alcohol such as ethanol, polysorbate 80, and the like. These compositions may further contain a preservative, a moistening agent, an emulsifier, a dispersing agent, a stabilizer and a solubilization assisting agent. These compositions are sterilized, e.g., by filtering them through a bacteria retention filter, incorporating a bactericide or through irradiation. Alternatively, they may be prepared into a sterile solid composition, which is dissolved in sterile water or a sterile solvent for injection prior to use.

In the case of oral administration, suitable daily does is usually about 0.0001 to 50 mg/kg body weight, preferably about 0.001 to 10 mg/kg, more preferably about 0.01 to 1 mg/kg, and the daily does is administered once a day or divided into 2 to 4 doses per day. In the case of intravenous injection, suitable daily dose is usually about 0.0001 to 1 mg/kg body weight, preferably about 0.0001 to 0.1 mg/kg. And the daily does is administered once a day or divided into a plurality of doses per day. The dose may be appropriately determined for each case, depending on conditions, age, sex.

The compounds of the present invention can be utilized alone, or in conjunction with other treatments (e.g., radiotherapy and surgery). Moreover, they can be utilized in conjunction with other antitumor agents, such as alkylation agents (cisplatin, carboplatin), antimetabolites (methotrexate, 5-FU), antitumor antibiotics (adriamymycin, bleomycin), antitumor vegetable alkaloids (taxol, etoposide), antitumor hormones (dexamethasone, tamoxifen), antitumor immunological agents (interferon α, β, γ).

### EXAMPLES

The present invention will be described in more detail by referring to the following EXAMPLES.

The following Tables 1 ∼ 3 and 13 ∼ 16 show starting compounds which were used in EXAMPLES, and Tables 4 ∼ 11 and 17 ∼ 24 show structural formulas as well as physicochemical properties of the compounds of the present invention. Only the compounds falling under the claims are part of the present invention: Compounds 36, 37, 39, A₁, A₃, A₅ A₇, A₁₁, B₁₆, B₁₉, B₂₂, B₂₅, B₂₈, B₃₁, for example, are not. Moreover, the compounds of the present invention with structural formulas shown in Tables 12 and 25 ∼ 26 can be easily produced in the same manner as in the EXAMPLES mentioned hereinafter or in accordance with the Synthesis Methods mentioned hereinabove, or by applying thereto some modifications which are obvious to those skilled in the art.

In the tables, abbreviations are used to mean the following.
Rco: starting compounds number
Rex: Synthesis method of starting compounds (a following number represents a Reference Example number described hereinafter, indicating that the compound was prepared using the method described in the Reference Example or the one similar thereto.)
Co: compounds number of the present invention
Str: structural formula
Sal: salt
Syn: synthesis method (a following number represents a number of an EXAMPLE described hereinbelow, indicating that the compound is produced using the method described in the EXAMPLE or a similar method.)
Dat: physicochemical properties wherein:
- F:: FAB-MS (M+H)⁺
- FN:: FAB-MS (M-H)⁻
- E:: EI-MS
- M:: melting point [°C]
- (dec.):: Decomposition
- d6, TMS N1:: characteristic peaks δ ppm of NMR (DMSO- internal standard)
- Ac :: acetyl
- Bn:: benzyl
- Ph:: phenyl
- Ts:: 4-toluenesulfonyl
- Ms:: methanesulfonyl
- Me:: methyl
- Et:: ethyl

Where two or more positions to permit substitution are present, the position substituted is indicated as a prefix (e.g.,6-MeO-7-HO represents 6-methoxy-7-hydroxy).

**Table 1**

| **Rco** | **Rex** | **Str** | **DAT** | **Rco** | **Rex** | **Str** | **DAT** |
|---|---|---|---|---|---|---|---|
| **1** | 1 | 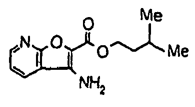 | F: 249 | **2** | 1 | 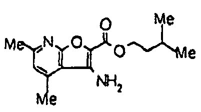 | F: 277 |
| **3** | 2 | 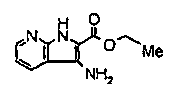 | F: 206 | **4** | 3 | 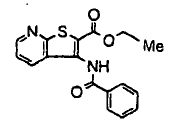 | F: 327 |
| **5** | 3 | 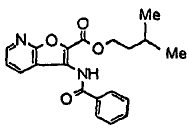 | F: 353 | **6** | 3 | 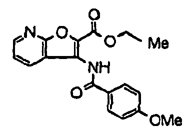 | F: 341 |
| **7** | 3 | 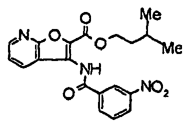 | F: 398 | **8** | 3 | 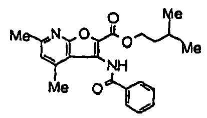 | F: 381 |
| **9** | 3 | 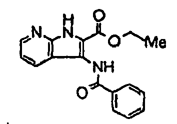 | F: 310 | **10** | 3 | 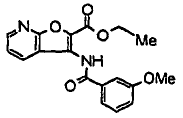 | F: 341 |
| **11** | 4 | 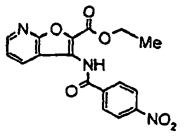 | F: 356 | **12** | 4 | 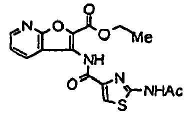 | F: 375 |
| **13** | 5 | 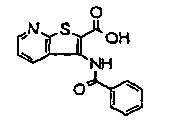 | F: 299 | **14** | 5 | 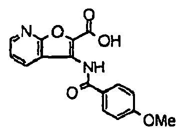 | FN: 311 |
| **15** | 5 | 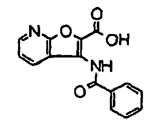 | FN: 281 | **16** | 5 | 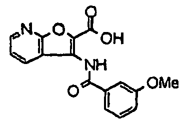 | FN: 311 |

**Table 2**

| **Rco** | **Rex** | **Str** | **DAT** | **Rco** | **Rex** | **Rtr** | **DAT** |
|---|---|---|---|---|---|---|---|
| **17** | 5 | 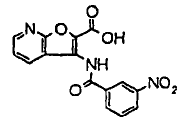 | FN: 326 | **18** | 5 | 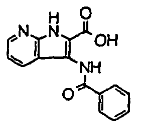 | F: 282 |
| **19** | 5 | 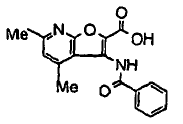 | F: 311 | **20** | 5 | 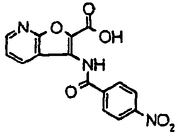 | FN: 326 |
| **21** | 6 | 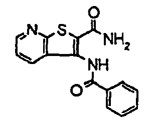 | F: 298 | **22** | 6 | 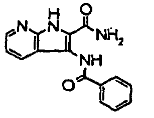 | FN: 279 |
| **23** | 6 | 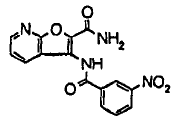 | FN: 325 | **24** | 6 | 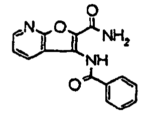 | F: 282 |
| **25** | 6 | 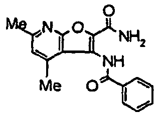 | F: 310 | **26** | 6 | 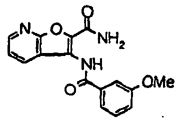 | F: 312 |
| **27** | 6 | 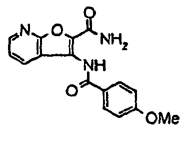 | F: 312 | **28** | 6 | 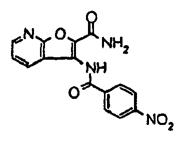 | FN: 325 |
| **29** | 7 | 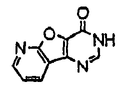 | F: 188 | **30** | 8 | 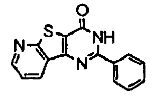 | E: 279 |
| **31** | 8 | 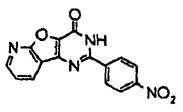 | E: 308 | **32** | 8 | 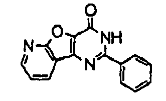 | F: 264 |

**Table 3**

| **Rco** | **Rex** | **Str** | **DAT** | **Rco** | **Rex** | **Str** | **DAT** |
|---|---|---|---|---|---|---|---|
| **33** | 8 | 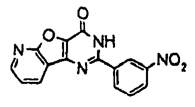 | F: 309 | **34** | 8 | 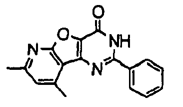 | F: 292 |
| **35** | 8 | 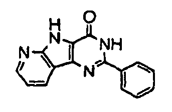 | F: 263 | **36** | 8 | 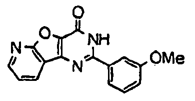 | F: 294 |
| **37** | 8 | 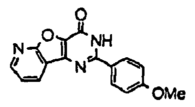 | E: 293 | **38** | 9 | 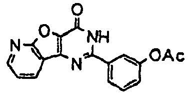 | F: 322 |
| **39** | 9 | 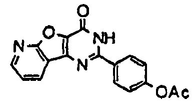 | E: 321 | **40** | 3 | 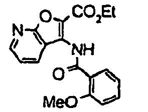 | F: 341 |
| **41** | 4 | 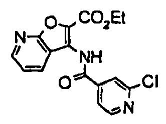 | FN: 344 | **42** | 5 | 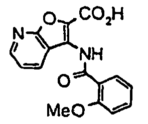 | FN: 311 |
| **43** | 5 | 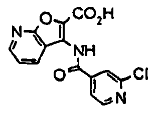 | FN: 316 | **44** | 6 | 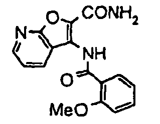 | F: 312 |
| **45** | 6 | 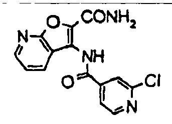 | F: 317 | **46** | 8 | 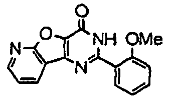 | F: 293 |
| **47** | 8 | 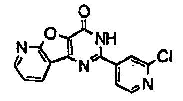 | FN: 297 | **48** | 9 | 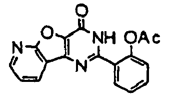 | F: 322 |
| **49** | 19 | 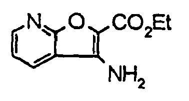 | F: 207 | | | | |

**Table 4**

| **Co** | Syn | Str | DAT | **Co** | Syn | Str | DAT |
|---|---|---|---|---|---|---|---|
| **1** | 1 | 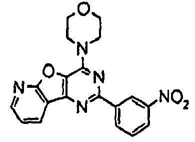 | F: 378 | **2** | 1 | 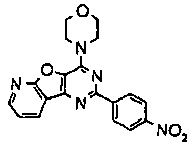 | E: 377 |
| **3** | 2 | 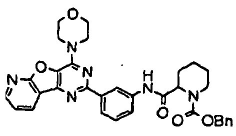 | F: 593 | **4** | 2 | 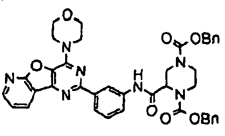 | N1: 4.15 (4H, t, *J*=4.4 Hz ), 8.52 (1H, s), 10.41 (1 H, s). |
| **5** | 2 | 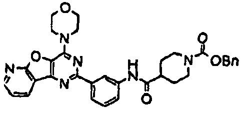 | F: 593 | **6** | 2 | 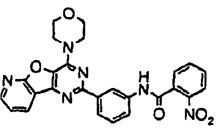 | F: 497 |
| **7** | 12 | 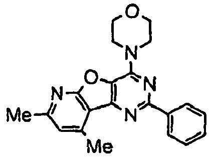 | M: 206-2 08 | **Z** | - | 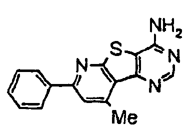 | mfd. by SPEC S and BioSPE CS B.V. (Catal og No.: AE-84 8/3855062) |

**Table 5**

| | | | | | | | |
|---|---|---|---|---|---|---|---|
| 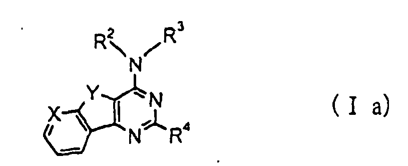 | | | | | | | |

| **Co** | Syn | X | Y | NR²R³ | R⁴ | Sal | DAT |
|---|---|---|---|---|---|---|---|
| **8** | 3 | N | O | 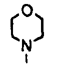 | 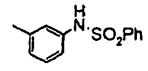 | - | M: 229-230 ; N1: 3.85 (4H, t, *J*=4.8 Hz), 7.22-7.25 (1H, m), 10.47 (1H, s) |
| **9** | 3 | N | O | 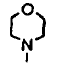 | 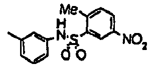 | - | M: 291-293 ; N1: 2.74 (3H, s), 7.39 (1H, t, *J*=7.8 Hz), 8.23 (1H, s) |
| **10** | 4 | N | O | 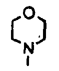 | 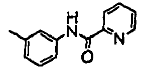 | - | M: 266-268 ; N1: 3.87 (4H, t, *J*=4.8 Hz), 7.51 (1H, t, *J*=7.8 Hz), 10.78 (1H,s) |
| **11** | 4 | N | O | 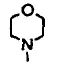 | 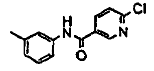 | - | F: 487 |
| **12** | 5 | N | O | 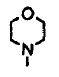 | 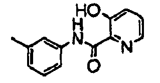 | HCl | N1: 3.87 (4H, t, *J*=4.8 Hz), 8.87 (1H, s), 11.06 (1H, s) |
| **13** | 5 | N | O | 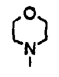 | 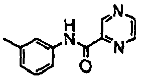 | HCl | N1: 3.88 (4H, t, *J*=4.8 Hz), 9.34 (1H, s), 10.88 (1H, s) |
| **14** | 5 | N | O | 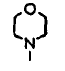 | 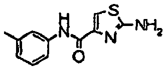 | 2HCl | N1: 3.86 (4H, t, *J*=4.8 Hz), 8.14 (1H, s), 10.85 (1H, s) |
| **15** | 5 | N | O | 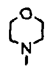 | 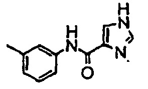 | 2HCl | N1: 3.87 (4H, t, *J*=4.4 Hz), 7.56 (1H, t, *J*=7.8 Hz), 11.26 (1H, s) |
| **16** | 5 | N | O | 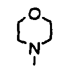 | 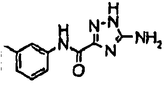 | HCl | N1: 3.87 (4H, t, *J*=4.4 Hz), 8.84 (1H, s), 10.72 (1H, s) |
| **17** | 5 | N | O | 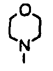 | 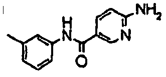 | 2HCl | M: 203-207 ; N1: 3.86 (4H, t, *J*=4.9 Hz), 7.52 (1H, t, *J*=7.8 Hz), 10.71 (1H,s) |
| **18** | 5 | N | O | 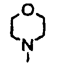 | 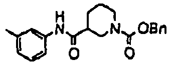 | | N1: 1.35-1.48 (1H, m), 3.85 (4H, t, *J*=4.4 Hz), 10.19 (1H, s) |

**Table 6**

| **Co** | Syn | X | Y | NR²R³ | R⁴ | Sal | DAT |
|---|---|---|---|---|---|---|---|
| **19** | 5 | N | O | 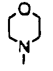 | 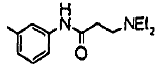 | 2HCl | M: 203-206 |
| **20** | 5 | N | O | 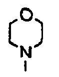 | 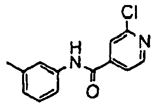 | - | F:487 |
| **21** | 5 | N | O | 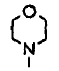 | 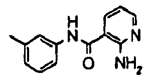 | 2HCl | M: 173-175 ; N1: 7.53 (1H, t, *J*=7.8 Hz), 8.24-8.29 (2H, m), 11.01 (1 H, s) |
| **22** | 5 | N | O | 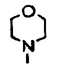 | 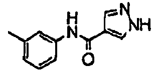 | HCl | N1: 3.87 (4H, m), 8.30 (2H, s), 10.06 (1H,s) |
| **23** | 5 | N | O | 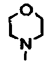 | 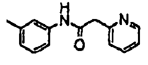 | 2HCl | N1: 4.39 (2H, s), 7.47 (1H, t, J = 7.7 Hz), 10.87 (1H, s) |
| **24** | 6 | N | O | 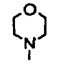 | 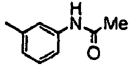 | - | N1: 2.09 (3H, s), 3.87 (4H, t, *J*=4.9 Hz), 10.11 (1H, s) |
| **25** | 7 | N | O | 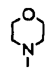 | 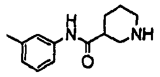 | 2HCl | M: 213-217 |
| **26** | 7 | N | O | 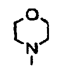 | 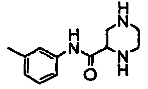 | 3HCl | M: 203-245 |
| **27** | 7 | N | O | 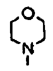 | 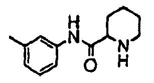 | 2HCl | N1: 1.50-1.90 (5H, m), 3.86 (4H, t, *J*=4.9 Hz), 11.00 (1H, s) |
| **28** | 7 | N | O | 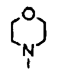 | 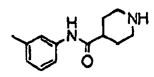 | 2HCl | N1: 1.83-2.06 (4H, m), 3.86 (4H, t, *J*=4.4 Hz), 10.37(1H, s) |
| **29** | 8 | N | O | 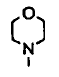 | 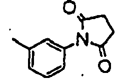 | - | N1: 2.84 (4H, s), 3.85 (4H, t, *J*=4.9 Hz), 7.3 8-7.40 (1H, m) |
| **30** | 9 | N | O | 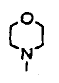 | 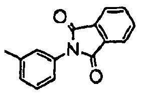 | HCl | M: 293-295 |
| **31** | 10 | N | O | 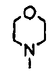 | 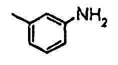 | 2HCl | M: 237-240 |

**Table 7**

| **Co** | Syn | X | Y | NR²R³ | R⁴ | Sal | DAT |
|---|---|---|---|---|---|---|---|
| **32** | 10 | N | O | 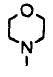 | 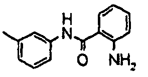 | 2HCl | N1: 3.87 (4H, t, *J*=4.4 Hz), 7.51-7.55 (2H, m), 10.68 (1H, s) |
| **33** | 10 | N | O | 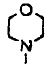 | 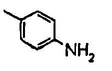 | HCl | M: 262-266 ; N1: 3.86 (4H, t, *J*=4.4 Hz), 8.37 (2H, d, *J*=8.8 Hz), 8.70 (1H, dd, *J*=1.5, 4.9 Hz) |
| **34** | 11 | N | O | NH₂ | H | - | N1: 7.59 (1H, dd, *J*=4.8, 7.8 Hz), 7.72 (2H, br s), 8.45 (1H, s) |
| **35** | 12 | N | O | 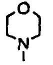 | 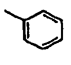 | - | M: 237-239 |
| **36** | 12 | N | NH | 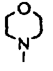 | 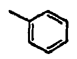 | - | M: 248-250 |
| **37** | 12 | N | S | 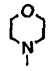 | 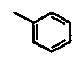 | - | M: 201-202 |
| **38** | 12 | N | O | 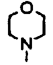 | H | - | M: 182-183 |
| **39** | 12 | CH | S | 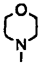 | H | HCl | M: 202-205 |
| **40** | 13 | N | O | 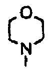 | 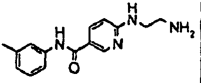 | 2HCl | M: 237-240 ; N1: 3.09-3.14 (2H, m), 7.50 (1H, t, *J*=7.8 Hz), 10.59 (1H, s) |
| **41** | 13 | N | O | 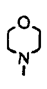 | 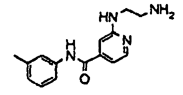 | 3HCl | M: 178(dec.) ; N1: 3.13-3.16 (2H, m), 7.54 (1H, t, *J*=7.8 Hz), 11.04 (1H, s) |
| **42** | 13 | N | O | 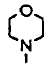 | 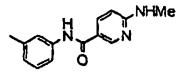 | 2HCl | M: 282-285 ; N1: 3.09 (3H, s), 7.51 (1H, t, *J*=7.8 Hz), 10.79 (1H, s) |
| **43** | 13 | N | O | 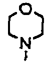 | 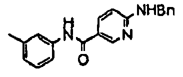 | 2HCl | M: 257-261 ; N1: 4.81 (2H, s), 7.33-7.53 (6H, m), 10.76 (1H, s) |
| **44** | 14 | N | O | 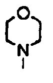 | 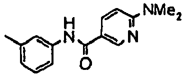 | 2HCl | M: 234-237 ; N1: 3.32 (6H, s), 7.53 (1H, t, *J*=7.8 Hz), 10.75 (1H, s) |
| **45** | 15 | N | O | 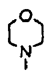 | 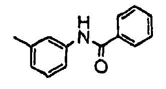 | HCl | M: 244-245 ; N1: 3.87 (4H, t, *J*=4.9 Hz), 7.49-7.67 (5H, m), 10.47 (1H, s) |

**Table 8**

| **Co** | Syn | X | Y | NR²R³ | R⁴ | Sal | DAT |
|---|---|---|---|---|---|---|---|
| **46** | 15 | N | O | 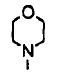 | 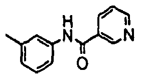 | 2HCl | N1: 3.87 (4H, t, *J*=4.9 Hz), 7.53 (1H, t, *J*=7.8 Hz), 10.73 (1H, s) |
| **47** | 15 | N | O | 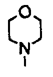 | 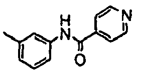 | 2HCl | N1: 3.87 (4H, t, *J*=4.9 Hz), 7.55 (1H, t, *J*=7.8 Hz), 10.86 (1H, s) |
| **48** | 15 | N | O | 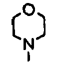 | 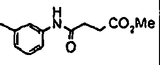 | HCl | M: 195-197 ; N1: 3.62 (3H, s), 7.44 (1H, t, *J*=7.8 Hz), 10.25 (1H, s) |
| **49** | 16 | N | O | 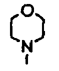 | 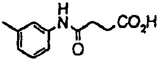 | HCl | M: 164-167 ; N1: 2.55-2.64 (4H, m), 7.43 (1H, t, *J*=7.8 Hz), 10.17 (1H, s) |
| **50** | 17 | N | O | 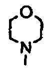 | 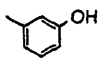 | HCl | M: 270-272 ; N1: 4.15 (4H, t, *J*=4.8 Hz), 7.32 (1H, t, *J*=7.8 Hz), 8.70 (1H, dd, 7=1.9, 4.9 Hz) |
| **51** | 17 | N | O | 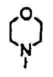 | 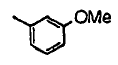 | HCl | M: 182-184 ; N1: 3.87 (3H, s), 7.45 (1H, t, *J*=7.8 Hz), 8.69 (1H, dd, *J*=1.5, 4.9 Hz) |
| **52** | 17 | N | O | 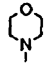 | 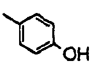 | HCl | M: 306 (dec.) ; N1: 3. 85 (4H, t, *J*=4.9 Hz), 6.91 (2H, d, *J*=8.8 Hz), 8.32 (2H, d, *J*=8.8 Hz) |
| **53** | 17 | N | O | 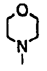 | 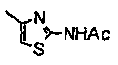 | HCl | N1: 2.18 (3H, s), 8.11 (1H, s), 12.50 (1H, s) |
| **54** | 18 | N | O | 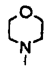 | 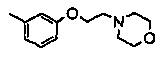 | 2HCl | M: 186-190 ; N1: 4.15 (4H, t, *J*=4.4 Hz), 4.59 (2H, t, *J*=4.8 Hz), 7.49 (1H, t, *J*=7.8 Hz) |
| **55** | 18 | N | O | 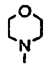 | 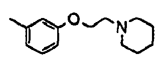 | 2HCl | M: 283-286 ; N1: 1.35-1.47 (1H, m), 4.56 (2H, t, *J*=4.9 Hz), 7.49 (1H, t, *J*=7.8 Hz) |
| **56** | 18 | N | O | 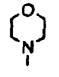 | 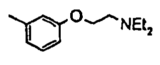 | 2HCl | M: 233-235 ; N1: 1.30 (6H, t, *J*=7.3 Hz), 4.53 (2H, t, *J*=4.9 Hz), 7.49 (1H, t, *J*=7.8 Hz) |
| **57** | 18 | N | O | 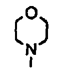 | 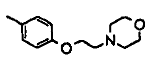 | 2HCl | M: 275-277 ; N1: 3.19-3.28 (2H, m), 7.15 (2H, d, *J*=8.8 Hz), 8.46 (2H, d, *J*=8.8 Hz) |

**Table 9**

| | | | | |
|---|---|---|---|---|
| 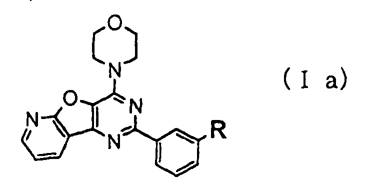 | | | | |

| **Co** | Syn | R | Sal | DAT |
|---|---|---|---|---|
| **58** | 47 | 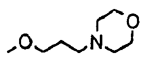 | HCl | N1: 3.29-3.34 (2H, m), 7.46 (1H, t,) *J*=7.8 Hz), 8.68-8.71 (2H, m) |
| **59** | 48 | 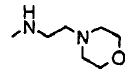 | 2HCl | M: 206-210 ; N1: 4.17 (4H, t, *J*=4.9 Hz), 7.73 (1H, d, *J*=7.8 Hz), 7.81 (1H, s) |
| **60** | 49 | -NHCOCF₃ | - | N1: 3.86 (4H, t, *J*=4.9 Hz), 7.64 (1H, dd, *J*=5.0, 7.7 Hz), 11.43 (1H, s) |
| **61** | 18 | 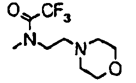 | - | N1: 7.56-7.67 (3H, m), 8.48-8.53 (2H, m), 8.62-8.65 (1H, m) |
| **62** | 50 | 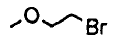 | - | N1: 3.83-3.88 (6H, m), 7.45 (1H, t, *J*=7.9 Hz), 8.67-8.72 (2H, m) |
| **63** | 50 | 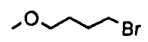 | - | N1: 1.85-2.07 (4H, m), 4.10-4.14 (6H, m), 8.65-8.70 (2H, m) |
| **64** | 51 | 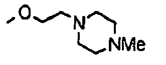 | 3HCl | N1: 2.85 (3H, br s), 4.16 (4H, t, *J*=4.4 Hz), 7.66 (1H, dd, *J*=4.9, 7.8 Hz) |
| **65** | 18 | 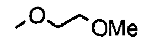 | - | N1: 4.19 (2H, t, *J*=4.9 Hz), 7.62 (1H, t, *J*=7.8 Hz), 7.96 (1H, s) |
| **66** | 51 | 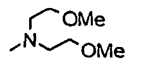 | 2HCl | M: 196-198; N1: 3.33 (6H, s), 7.50 (1H, t, *J*=7.8 Hz), 8.07-8.11 (2H, m) |
| **67** | 51 | 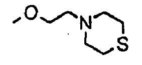 | HCl | N1: 4.12 (4H, t, *J*=4.3 Hz), 8.09 (1H, d, *J*=8.0 Hz), 8.65-8.69 (2H, m) |
| **68** | 51 | 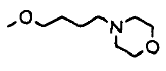 | 2HCl | M: 243-248 ; N1: 3.01-3.10 (2H, m), 3.15-3.20 (2H, m), 7.65 (1H, dd, *J*=4.9, 7.8 Hz) |
| **69** | 52 | 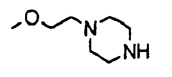 | 3HCl | M: 250-253 ; N1: 4.56 (2H, t, *J*=4.9 Hz), 7.49 (1H, t, *J*=7.8 Hz), 8.10 (1H, d, *J*=7.8 Hz) |
| **70** | 53 | 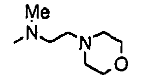 | 2HCl | N1: 3.05 (3H, s), 4.19 (4H, t, *J*=4.4 Hz), 7.91 (1H, br s) |

**Table 10**

| **Co** | Syn | R | Sal | DAT |
|---|---|---|---|---|
| **71** | 15 | 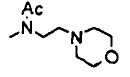 | 2HCl | M: 244-245 ; N 1: 1.84 (3H, s), 4.11-4.18 (6H, m), 7.64-7.72 (3H, m) |
| **72** | 51 | 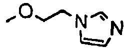 | 2HCl | M: 196-201 ; N1: 4.15 (4H, t, *J*=4.4 Hz), 7.74 (1H, s), 9.34 (1H, s) |
| **73** | 15 | 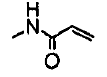 | HCl | N1: 5.79 (1H, d, *J*=10.8 Hz), 6.30 (1H, d, *J*=17.1 Hz), 10.40 (1H, s) |
| **74** | 51 | 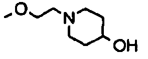 | 2HCl | N1: 4.15 (4H, t, *J*=4.9 Hz), 4.53-4.56 (2H, m), 8.05 (1H, s) |
| **75** | 18 | 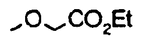 | HCl | N1: 1.24 (3H, t, *J*=6.8 Hz), 4.22 (2H, q, *J*=6.8 Hz), 4.89 (2H, s) |
| **76** | 16 | 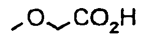 | HCl | M: 264-267 ; N1: 4.79 (2H, s), 7.45 (1H, t, *J*=7.8 Hz), 7.64 (1H, dd, *J*=4.9, 7.8 Hz) |
| **77** | 54 | 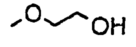 | HCl | M: 243-244 ; N1: 3.78 (2H, t, *J*=4.9 Hz), 4.14 (4H, t, *J*=4.9 Hz), 7.98-7.99 (1H, m) |

**Table 11**

| | | | | |
|---|---|---|---|---|
| 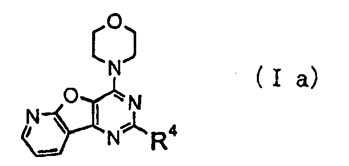 | | | | |

| **Co** | Syn | R⁴ | Sal | DAT |
|---|---|---|---|---|
| **78** | 47 | 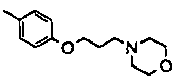 | 2HCl | N1: 3.05-3.14 (2H, m), 3.26-3.31 (2H, m), 7.10 (2H, d, *J*=8.8 Hz) |
| **79** | 55 | 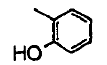 | - | N1: 4.15 (4H, t, *J*=4.9 Hz), 7.66 (1H, dd, *J*=4.9, 7.8 Hz), 8.15 (1H, dd, *J*=1.5, 7.8 Hz) |
| **80** | 12 | 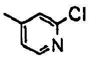 | - | N1: 4.16 (4H, t, *J*=4.9 Hz), 7.67 (1H, dd, *J*=4.9, 7.3 Hz), 8.34-8.35 (2H, m) |
| **81** | 56 | 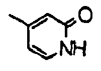 | - | M: 343-347 ; N1: 7.64 (1H, dd, *J*=4.9, 7.3 Hz), 8.66-8.69 (2H, m), 11. 72 (1H, br) |

**Table 12**

| | | | | | | | |
|---|---|---|---|---|---|---|---|
| 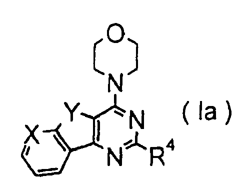 | | | | | | | |

| **Co** | X | Y | R⁴ | **Co** | X | Y | R⁴ |
|---|---|---|---|---|---|---|---|
| **A1** | N | S | 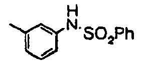 | **A2** | N | S | 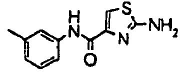 |
| **A3** | N | S | 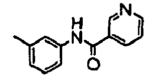 | **A4** | N | S | 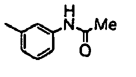 |
| **A5** | N | S | 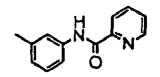 | **A6** | N | S | 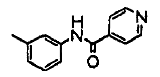 |
| **A7** | N | S | 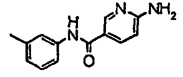 | **A8** | N | O | 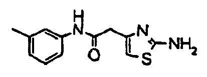 |
| **A9** | N | O | 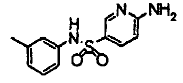 | **A10** | N | S | 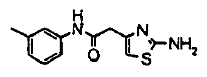 |
| **A11** | N | S | 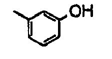 | **A12** | N | S | 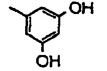 |
| **A13** | N | O | 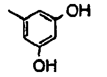 | **A14** | N | S | 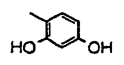 |
| **A15** | N | O | 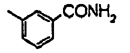 | **A16** | N | S | 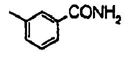 |
| **A17** | N | O | 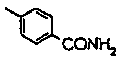 | **A18** | N | S | 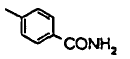 |
| **A19** | N | O | 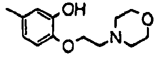 | **A20** | N | S | 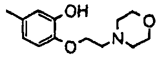 |
| **A21** | N | O | 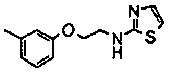 | **A22** | N | S | 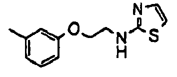 |
| **A23** | N | O | 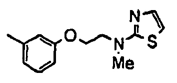 | **A24** | N | S | 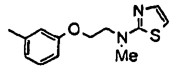 |
| **A25** | N | O | 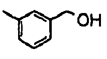 | **A26** | N | S | 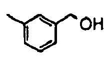 |

**Table 13**

| **Rco** | **Rex** | **Str** | **DAT** | **Rco** | **Rex** | **Str** | **DAT** |
|---|---|---|---|---|---|---|---|
| **50** | 10 | 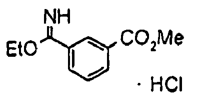 | F: 208 | **51** | 10 | 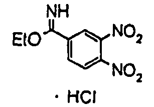 | F: 240 |
| **52** | 11 | 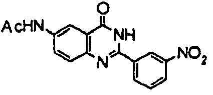 | E: 324 | **53** | 11 | 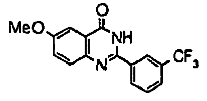 | F: 321 |
| **54** | 11 | 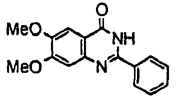 | F: 283 | **55** | 11 | 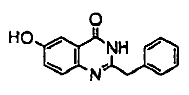 | F: 253 |
| **56** | 11 | 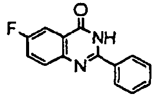 | F: 241 | **57** | 11 | 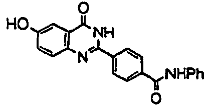 | F: 321 |
| **58** | 11 | 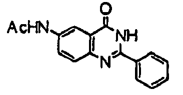 | F: 280 | **59** | 11 | 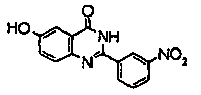 | FN: 282 |
| **60** | 11 | 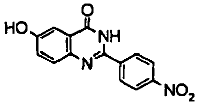 | FN: 282 | **61** | 11 | 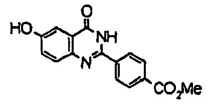 | F: 297 |
| **62** | 11 | 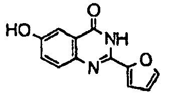 | FN: 227 | **63** | 11 | 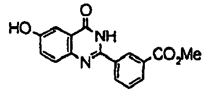 | F: 297 |
| **64** | 11 | 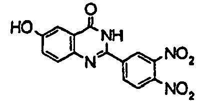 | F: 329 | **65** | 11 | 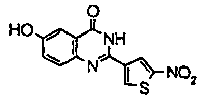 | F: 290 |
| **66** | 11 | 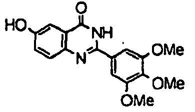 | F: 329 | **67** | 12 | 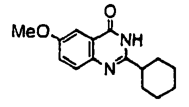 | F: 259 |
| **68** | 12 | 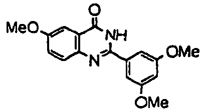 | F: 313 | **69** | 12 | 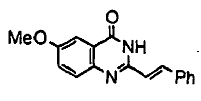 | F: 279 |

**Table 14**

| **Rco** | **Rex** | **Str** | **DAT** | **Rco** | **Rex** | **Str** | **DAT** |
|---|---|---|---|---|---|---|---|
| **70** | 12 | 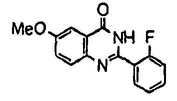 | F: 271 | **71** | 12 | 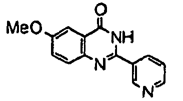 | F: 254 |
| **72** | 13 | 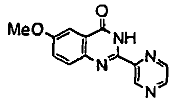 | F: 255 | **73** | 13 | 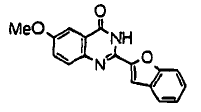 | F: 293 |
| **74** | 14 | 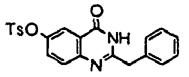 | F: 407 | **75** | 14 | 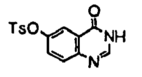 | F: 317 |
| **76** | 14 | 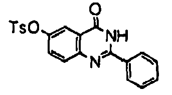 | F: 393 | **77** | 15 | 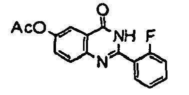 | F: 299 |
| **78** | 15 | 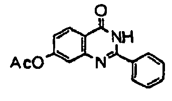 | F: 281 | **79** | 15 | 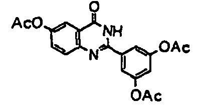 | F: 397 |
| **80** | 15 | 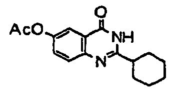 | F: 287 | **81** | 15 | 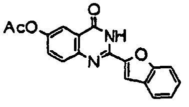 | F: 321 |
| **82** | 15 | 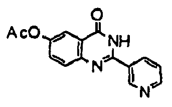 | F: 282 | **83** | 16 | 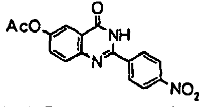 | FN: 324 |
| **84** | 16 | 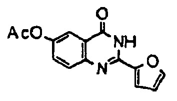 | F: 271 | **85** | 16 | 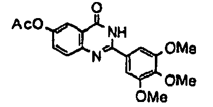 | F: 371 |
| **86** | 16 | 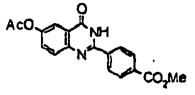 | F: 339 | **87** | 16 | 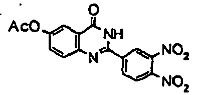 | FN: 369 |
| **88** | 16 | 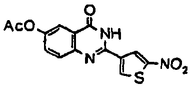 | F:332 | **89** | 16 | 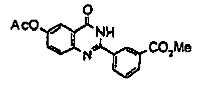 | F: 339 |

**Table 15**

| **Rco** | **Rex** | **Str** | **DAT** | **Rco** | **Rex** | **Str** | **DAT** |
|---|---|---|---|---|---|---|---|
| **90** | 16 | 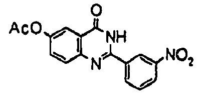 | F: 326 | **91** | 16 | 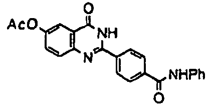 | F: 400 |
| **92** | 17 | 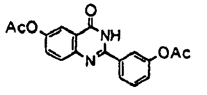 | F: 339 | **93** | 17 | 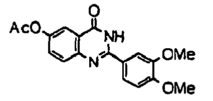 | F: 341 |
| **94** | 17 | 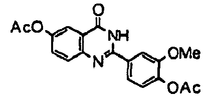 | F: 369 | **95** | 18 | 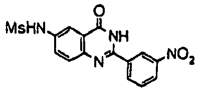 | FN: 359 |
| **96** | 3 | 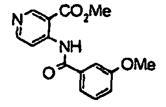 | F: 286 | **97** | 3 | 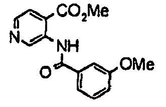 | F: 287 |
| **98** | 3 | 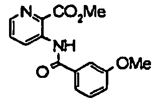 | F: 287 | **99** | 3 | 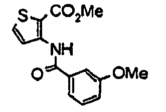 | F: 292 |
| **100** | 3 | 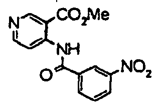 | F: 302 | **101** | 3 | 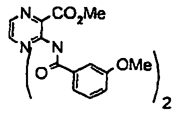 | F: 422 |
| **102** | 8 | 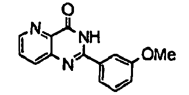 | F: 254 | **103** | 8 | 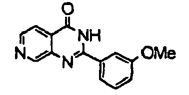 | F: 254 |
| **104** | 8 | 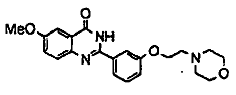 | F: 382 | **105** | 8 | 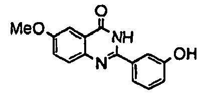 | F: 269 |
| **106** | 9 | 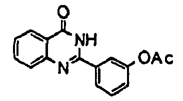 | F: 281 | **107** | 9 | 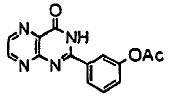 | F: 283 |
| **108** | 9 | 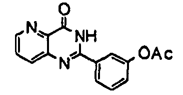 | F: 282 | **109** | 9 | 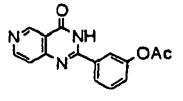 | F: 282 |

**Table 16**

| **Rco** | **Rex** | **Str** | **DAT** | **Rco** | **Rex** | **Str** | **DAT** |
|---|---|---|---|---|---|---|---|
| **110** | 9 | 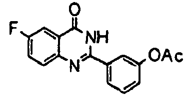 | F: 299 | **111** | 9 | 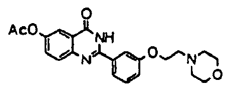 | F: 410 |
| **112** | 9 | 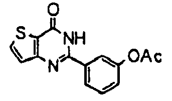 | F: 286 | **113** | 9 | 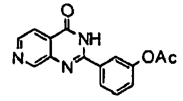 | F: 282 |
| **114** | 9 | 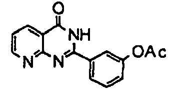 | F: 282 | **115** | 11 | 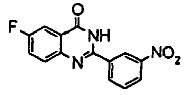 | F: 286 |
| **116** | 20 | 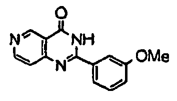 | F: 254 | **117** | 21 | 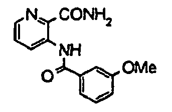 | F: 272 |
| **118** | 21 | 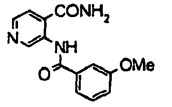 | F: 272 | **119** | 22 | 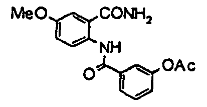 | F: 329 |
| **120** | 22 | 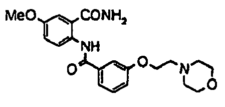 | F: 400 | **121** | 23 | 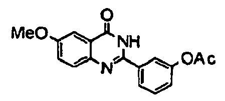 | F: 311 |
| **122** | 24 | 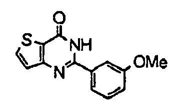 | F: 259 | **123** | 24 | 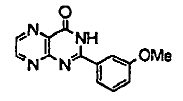 | F: 255 |
| **124** | 24 | 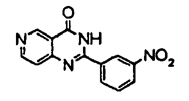 | F: 269 | | | | |

**Table 17**

| | | | | | | | |
|---|---|---|---|---|---|---|---|
| 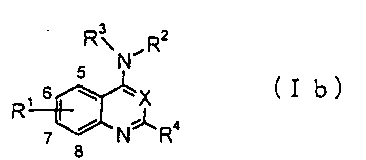 | | | | | | | |

| **Co** | Syn | X | R¹ | NR²R³ | R⁴ | Sal | DAT |
|---|---|---|---|---|---|---|---|
| **82** | 19 | N | H | 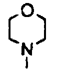 | 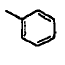 | - | M: 111-112 ; N2: 3.86 (4H, m), 3.95 (4H, m), 8.56 (2H, m) |
| **83** | 19 | N | 6-F | 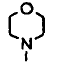 | 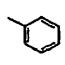 | - | M: 157-159 ; N2: 3.80 (4H, t, *J* = 4.7Hz), 3.94 (4H, t, *J* = 4.7Hz), 8.50-8.54 (2H, m) |
| **84** | 19 | N | 6-MeO-7 -MeO | 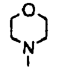 | 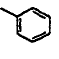 | - | M: 182-186 |
| **85** | 19 | N | 6-NO₂ | 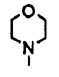 | 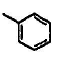 | - | M: 238-240 ; N1: 3.83 (4H, t, *J* = - 4.9Hz), 7.99 (1H, d, *J* = 8.8Hz), 8.82 (1H, d, *J* = 2.4Hz) |
| **86** | 19 | N | 6-AcHN | 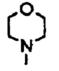 | 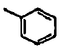 | - | M: 121-124 |
| **87** | 19 | N | 6-MeO | 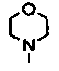 | 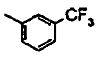 | - | M: 145-146 ; N1: 3.79 (4H, m), 3.87 (4H, m), 3.94 (3H, s) |
| **88** | 19 | N | 6-AcHN | 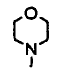 | 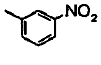 | - | N1: 8.52 (1H, s), 8.86 (1H, m), 10.36 (1H, s) |
| **89** | 19 | N | 6-MeO | 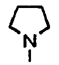 | 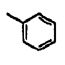 | - | M: 161-163 |
| **89** | 19 | N | 6-MeO | 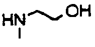 | 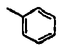 | - | M: 218-220 |
| **90** | 19 | N | 6-MsHN | 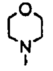 | 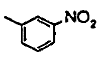 | - | N1: 3.10 (3H, s), 3.80-3.90 (8H, m), 10.18 (1H, br) |
| **91** | 19 | CH | 6-MeO | 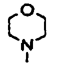 | 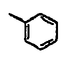 | - | N 1: 3.92 (8H, m), 7.96 (1H, d, J= 8.8 Hz), 8.22 (2H, m) |
| **92** | 20 | N | 6-MeO-7 -OH | 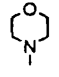 | 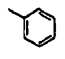 | - | M: 202-204 ; N1: 3.70 (4H, t, *J* = 4.4Hz), 3.98 (3H, s), 7.07 (1H, s) |
| **93** | 20 | N | 6-HO | 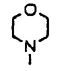 | 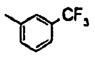 | - | M: 203-205 ; N1: 3.79 (4H, m), 3.87 (4H, m), 10.22 (1H, s) |
| **94** | 20 | N | 6-HO | 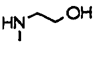 | 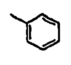 | - | M: 222-225 (dec.) ; N1: 3.72 (4H, m), 4.82 (1H, brs), 8.01 (1H, brs) |

**Table 18**

| **Co** | Syn | X | R¹ | NR²R³ | R⁴ | Sal | DAT |
|---|---|---|---|---|---|---|---|
| **96** | 20 | N | 6-HO | 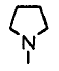 | 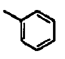 | - | M: 296-345 (dec.) |
| **97** | 21 | N | 6-H₂N | 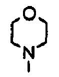 | 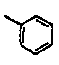 | - | M:184-186 |
| **98** | 22 | N | 6-OHCHN- | 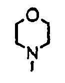 | 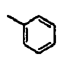 | - | M: 218-222 ; N1: 3.79 (4H, t, *J* = 4.2 Hz), 8.41 (1H, d, *J* = 1.5 Hz) 10.59 (1H, s) |
| **99** | 23 | N | 6-HO | 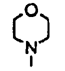 | 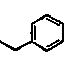 | - | M: 243-249 ; N1: 4.07 (2H, s), 7.67 (1H, d, J = 8.8 Hz), 10.00 (1H, s) |
| **100** | 23 | N | 6-HO | 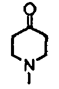 | 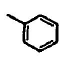 | - | M: 258-262 (dec.) |
| **101** | 23 | N | 6-HO | 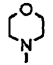 | H | - | M: 259-260 ; N1: 3.57 (4H, t, *J* = 4.7 Hz), 8.55 (1H, s) 10.12 (1H, s) |
| **102** | 23 | N | 6-HO | 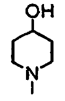 | 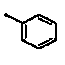 | - | M: 249-250 ; N1: 3.82 (1H, m), 7.77 (1H, d, J = 9.6 Hz), 10.08 (1H, s) |
| **103** | 23 | N | 6-HO | 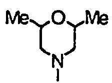 | 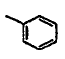 | - | M: 221-225 ; N1: 1.20 (6H, d, J = 6.4 Hz), 7.80 (1H, d, J = 8.8 Hz), 10.12 (1H, s) |
| **104** | 23 | N | 6-HO | 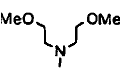 | 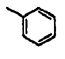 | - | M: 139-141 |
| **105** | 24 | N | 6-AcMeN- | 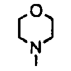 | 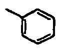 | - | M: 204-206 |
| **106** | 25 | N | 6-TsHN- | 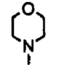 | 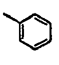 | - | M: 199-200 ; N1: 2.32 (3H, s) 3.62 (4H, t, *J*= 4.4 Hz), 10.65 (1H, s) |
| **107** | 26 | N | 6-Me₂N- | 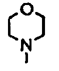 | 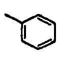 | - | M: 124-125 |
| **108** | 27 | N | 6-HO | 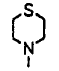 | 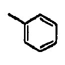 | 0.5 HCl | M: 268-271 |
| **109** | 28 | N | 6-HO | 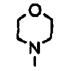 | Me | - | M: 281-284 |

**Table 19**

| **Co** | Ex. | X | R¹ | NR²R³ | R⁴ | Sal | DAT |
|---|---|---|---|---|---|---|---|
| **110** | 29 | N | 7-HO | 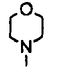 | 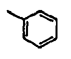 | - | M:245-246 |
| **111** | 29 | N | 6-HO | 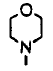 | 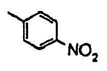 | - | M: 266-269 ; N1: 3.74 (4H, t, *J* = 4.4 Hz), 8.66 (2H, d, *J* = 9.1 Hz), 10.29 (1H, s) |
| **112** | 29 | N | 6-HO | 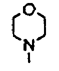 | 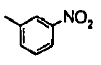 | - | M: 226-227 |
| **113** | 29 | N | 6-HO | 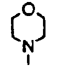 | 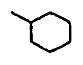 | - | N1: 1.94 (2H, m), 2.69 (1H, m), 7.65 (1H, d, J = 8.8 Hz) |
| **114** | 29 | N | 6-HO | 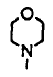 | 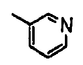 | - | M: 275-277 ; N1: 7.83 (1H, d, J = 8.8 Hz), 9.58 (1H, d, J = 1.5 Hz), 10.09 (1H, brs) |
| **115** | 29 | N | 6-HO | 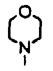 | 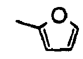 | - | M: 280 (dec.) |
| **116** | 29 | N | 6-HO | 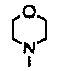 | 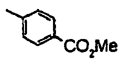 | - | M: 239-241 |
| **117** | 29 | N | 6-HO | 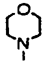 | 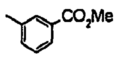 | - | M: 184-186 ; N1: 3.92 (3H, s), 9.03 (1H, br), 10.19 (1H, s) |
| **118** | 29 | N | 6-HO | 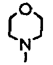 | 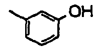 | - | M: 280-284 ; N1: 3.68 (4H, t, *J*= 4.5 Hz), 9.49 (1H, s), 10.12 (1H, s) |
| **119** | 29 | N | 6-HO | 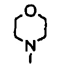 | 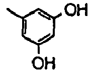 | - | M: 306-311 (dec.) ; N1: 7.75 (1H, d, *J* = 8.8 Hz), 9.29 (2H, s), 10.10 (1H, s) |
| **120** | 29 | N | 6-HO | 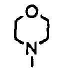 | 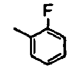 | - | M: 254-255 |
| **121** | 29 | N | 6-HO | 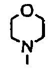 | 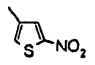 | - | M: 288-290 |
| **122** | 29 | N | 6-HO | 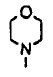 | 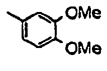 | - | M: 188-190 ; N1: 3.80 (3H, s), 13.83 (3H, s), 10.06 (1H, s) |
| **123** | 29 | N | 6-HO | 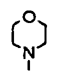 | 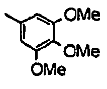 | - | M: 224-227 |

**Table 20**

| **Co** | Syn | X | R¹ | NR²R³ | R⁴ | Sal | DAT |
|---|---|---|---|---|---|---|---|
| **124** | 29 | N | 6-HO | 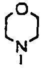 | 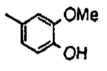 | - | M: 285-288 ; N1: 3.88 (3H, s), 9.37 (1H, s), 10.03 (1 H, s) |
| **125** | 29 | N | 6-HO | 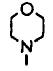 | 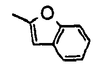 | - | M: 310-313 ; N1: 3.71 (4H, m), 3.87 (4H, m), 10.23 (1H, s) |
| **126** | 29 | N | 6-HO | 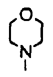 | 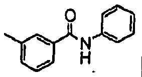 | - | M: 178-180 |
| **127** | 30 | N | 6-HO | 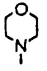 | 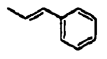 | - | M: 260-263 ; N1: 3.64 (4H, m), 3.86 (4H, m), 1H, 10.12 (1H, s) |
| **128** | 30 | N | 6-HO | 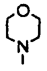 | 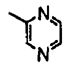 | - | M: 280-282 |
| **129** | 31 | N | 6-HO | 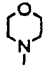 | 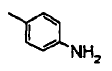 | - | M: 285 (dec.) ; N1: 3.62 (4H, t, *J* = 4.7 Hz), 5.51 (2H, br), 9.95 (1H, s) |
| **130** | 32 | N | 6-HO | 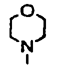 | 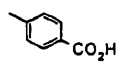 | - | M: 305 (dec.) |
| **131** | 32 | N | 6-HO | 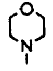 | 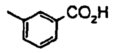 | - | M: 306-309 ; N1: 3.71 (4H, t, *J* = 4.9 Hz), 10.18 (1H, s), 13.08 (1H, s) |
| **132** | 33 | N | 6-HO | 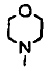 | 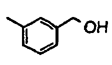 | - | M: 204-206 ; N1: 4.78 (2H, d, *J* = 5.9 Hz), 5.28 (1H, t, *J* = 5.9 Hz), 10.13 (1H, s) |
| **133** | 34 | N | 6-HO | 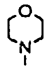 | 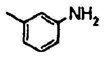 | - | M: 274-277 ; N1: 5.17 (2H, brs), 6.66 (1H, m), 10.08 (1H, s) |
| **134** | 34 | N | 6-MsHN | 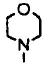 | 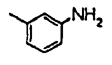 | - | N1: 3.07 (3H, s), 3.72-3.77 (4H, m), 10.07 (1H, br s) |
| **135** | 35 | N | 6-HO | 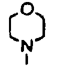 | 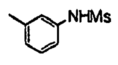 | - | M: 266-267 |
| **136** | 36 | N | 6-HO | 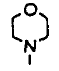 | 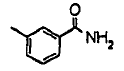 | - | M: 261-264 ; N1: 8.10 (1H, br), 8.91 (1H, t, *J* = 1.4), 10.17 (1H, s) |
| **137** | 36 | N | 6-HO | 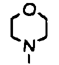 | 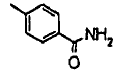 | - | M: 306-309 |
| **138** | 37 | N | 6-HO | 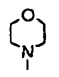 | 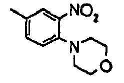 | - | M: 245-248 |

**Table 21**

| **Co** | Syn X | | R¹ | NR²R³ | R⁴ | Sal | DAT |
|---|---|---|---|---|---|---|---|
| **139** | 38 | N | 6-HO | 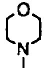 | 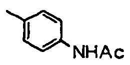 | - | M: 296-299 ; N1: 2.08 (3H, s), 10.08 (1H, s), 10.11 (1H, s) |
| **140** | 39 | N | 6-HO | 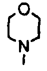 | 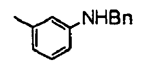 | - | M:152-157 |
| **141** | 40 | N | 6-HO | 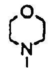 | 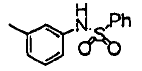 | - | M: 225-228 ; N1: 8.21 (1H, m), 10.16 (1H, brs), 10.44 (1H, brs) |
| **142** | 40 | N | 6-HO | 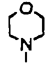 | 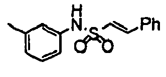 | - | M: 206-207 ; N1: 8.33 (1H, s), 10.12 (1H, s), 10.18 (1H, s) |
| **143** | 40 | N | 6-HO | 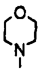 | 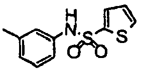 | - | M: 172-174 |
| **144** | 40 | N | 6-AcHN | 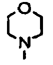 | 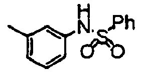 | - | M: 145-150 ; N1: 8.48 (1H, d, J = 2.0 Hz), 10.33 (1H, brs), 10.44 (1H, brs) |
| **145** | 40 | N | 6-MsHN | 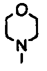 | 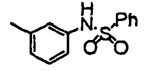 | - | M: 234-236 ; N1: 3.08 (3H, s), 3.74-3.79 (4H, m), 10.30 (2H, br) |
| **146** | 40 | N | 6-AcHN | 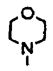 | 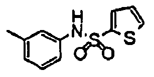 | - | M: 145-148 ; N1: 2.12 (3H, s), 10.34 (1H, s), 10.56 (1H, s) |
| **147** | 40 | N | 6-AcHN | 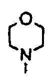 | 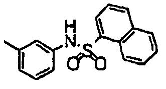 | - | M: 290 (d) ; N1: 2.12 (3H, s), 10.32 (1H, s), 10.83 (1H, s) |
| **148** | 41 | N | 6-HO | 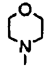 | 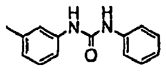 | - | M: 167-169 |
| **149** | 42 | N | 6-HO | 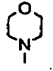 | 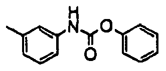 | - | M: 144-147 |
| **150** | 43 | N | 6-HO | 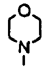 | 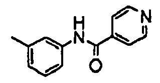 | - | M: 175-178 ; N1: 3.71-3.73 (4H, m), 10.17 (1H, s), 10.68 (1H, s) |
| **151** | 43 | N | .6-HO | 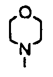 | 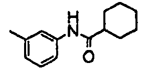 | - | M: 239-243 ; N1: 2.33-2.42 (1H, m), 3.66-3.69 (4H, m), 9.96 (1H, s) |

**Table 22**

| **Co** | Ex. | | X R¹ | NR²-R³ | R⁴ | Sal | DAT |
|---|---|---|---|---|---|---|---|
| **152** | 43 | N | 6-HO | 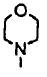 | 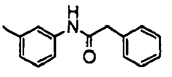 | - | M: 214-216 ; N1: 3.68-3.70 (6H, m), 10.14 (1H, s), 10.34 (1H, s) |
| **153** | 43 | N | 6-HO | 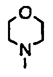 | 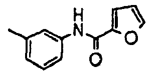 | - | M: 246-247 |
| **154** | 43 | N | 6-HO | 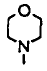 | 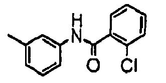 | - | M: 251-252 |
| **155** | 43 | N | 6-HO | 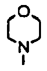 | 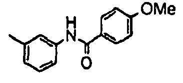 | - | N1: 3.86 (3H, s), 10.14 (1H, s), 10.26 (1H, s) |
| **156** | 43 | N | 6-HO | 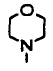 | 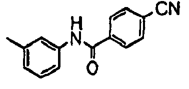 | - | M:182-183 |
| **157** | 43 | N | 6-HO | 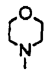 | 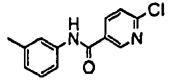 | - | N1: 3.72-3.74 (4H, m), 9.70-9:99 (1H, br), 10.45 (1H, brs) |
| **158** | 43 | N | 6-HO | 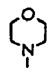 | 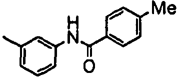 | - | M: 232-233 |
| **159** | 44 | N | 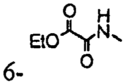 | 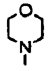 | 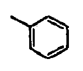 | - | M:182-183 |
| **160** | 45 | N | 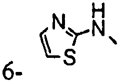 | 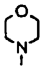 | 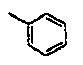 | - | M: 224-227 |
| **161** | 45 | N | 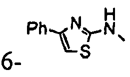 | 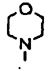 | 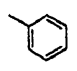 | - | M: 199-202 ; N1: 8.76 (1H, d, J = 2.4 Hz), 8.49 (2H, m), 10.7 (1H, brs) |
| **162** | 46 C | H | 6-HO | 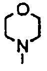 | 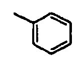 | - | M: 250-253 |

**Table 23**

| | | | | | |
|---|---|---|---|---|---|
| 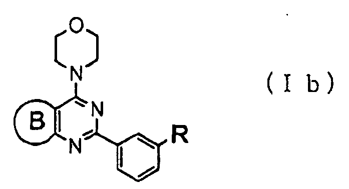 | | | | | |

| **Co** | Syn | B | R | Sal | DAT |
|---|---|---|---|---|---|
| **163** | 17 | 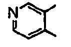 | OMe | 2HCl | N1: 3.84 (4H, t, *J*=4.9 Hz), 3.89 (3H, s), 9.55 (1H, s) |
| **164** | 17 | 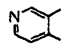 | OH | HCl | M: 261-266; N1: 3.84 (4H, t, *J*=4.9 Hz), 7.91 (1H, s), 9.53 (1H, s) |
| **165** | 18 | 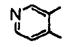 | 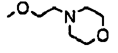 | 3HCl | M: 167-170 ; N1: 3.61 (2H, br s), 4.61 (2H, t, *J*=4.9 Hz), 9.54 (1H, s) |
| **166** | 47 | 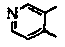 | 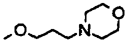 | 3HCl | N1: 3.26-3.31 (2H, m), 7.54 (1H, t, *J*=7.8 Hz), 9.53 (1H, s) |
| **167** | 17 | 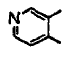 | NO₂ | HCl | M: 272-273; N1: 4.20 (4H, t, *J*=4.9 Hz), 7.97 (1H, d, *J*=6.4 Hz), 9.52 (1H, s) |
| **168** | 34 | 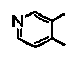 | NH₂ | 2HCl | M: 195-200 ; N1: 4.25 (4H, t, *J*=4.9 Hz), 7.64 (1H, t, *J*=7.8 Hz), 9.55 (1H, s) |
| **169** | 57 | 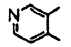 | NHAc | HCl | N1: 2.10 (3H, s), 9.52 (1H, s), 10.32 (1H, s) |
| **170** | 3 | 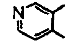 | NHSO₂Ph | HCl | N1: 8.75 (1H, d, *J*=6.4 Hz), 9.49 (1H, s), 10.62 (1H, s) |
| **171** | 2 | 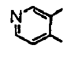 | 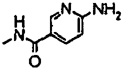 | 2HCl | M: 200-203; N1: 8.89-8.90 (1H, m), 9.53 (1H, s), 10.84 (1H, s) |
| **172** | 17 | 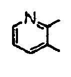 | OH | HCl | M: 233-238 ; N1: 4.73 (4H, br), 7.43 (1H, t, *J*=7.8 Hz), 10.02 (1H, br) |
| **173** | 18 | 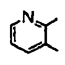 | 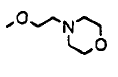 | 2HCl | M: 201-206 ; N1: 3.19-3.29 (2H, m), 7.55 (1H, t, *J*=7.8 Hz), 8.50 (1H, br) |
| **174** | 17 | 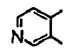 | OH | HCl | M: 269-274; N1: 7.39 (1H, t, *J*=7.8 Hz), 8.06 (1H, d, *J*=5.9 Hz), 9.44 (1H, s) |
| **175** | 18 | 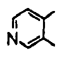 | 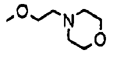 | 2HCl | N1: 3.20-3.29 (2H, m), 4.60 (2H, t, *J*=4.9 Hz), 9.50 (1H, s) |

**Table 24**

| **Co** | Syn | B | R | Sal | DAT |
|---|---|---|---|---|---|
| **176** | 17 | 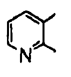 | OMe | HCl | M: 159-162 ; N1: 3.89 (3H, s), 7.55 (1H, t, *J*=7.8 Hz), 8.02 (1H, d, *J*=7.8 Hz) |
| **177** | 17 | 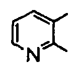 | OH | HCl | M: 274-279 ; N1: 4.22 (4H, t, *J*=4.9 Hz), 7.41 (1H, t, *J*=7.8 Hz), 10.05 (1H, br) |
| **178** | 18 | 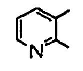 | 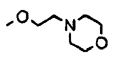 | 2HCl | N1: 4.59 (2H, t, *J*=4.9 Hz), 7.57 (1H, t, *J*=7.8 Hz), 7.68 (1H, dd, *J*=4.9, 8.3 Hz) |
| **179** | 17 | 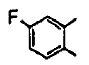 | OH | HCl | M: 235-237 ; N1: 4.19 (4H, br s), 7.43 (1H, t, *J*=7.8 Hz), 8.27-8.34 (1H, m) |
| **180** | 18 | 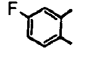 | 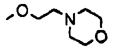 | 2HCl | N1: 4.19 (4H, br s), 8.28 (1H, br s), 8.57 (1H, br) |
| **181** | 17 | 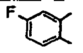 | NO₂ | HCl | N1: 3.78-3.79 (4H, m), 7.83-7.89 (3H, m), 8.88 (1H, d, *J*=7.8 Hz) |
| **182** | 34 | 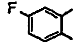 | NH₂ | 2HCl | N1: 4.11 (4H, br s), 7.47 (1H, d, *J*=7.8 Hz), 7.62 (1H, t, *J*=7.8 Hz) |
| **183** | 57 | 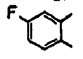 | NHAc | HCl | N1: 2.10 (3H, s), 7.52 (1H, t, *J*=7.8 Hz), 10.25 (1H, s) |
| **184** | 3 | 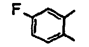 | NHSO₂Ph | HCl | N1: 4.10 (4H, br s), 8.17-8.28 (3H, m), 10.83 (1H, s) |
| **185 2** | | 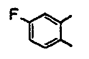 | 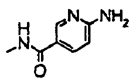 | 2HCl | M: 196-201; N1: 4.15 (4H, br s), 8.85-8.87 (2H, m), 10.97 (1H, s) |
| **186** | 17 | 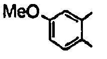 | OH | HCl | M: 252-258 ; N1: 3.95 (3H, s), 4.23 (4H, br s), 10.04 (1H, br) |
| **187** | 18 | 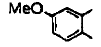 | 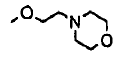 | 2HCl | N1: 4.67 (2H, t, *J*=4.9 Hz), 8.12 (1H, d, *J*=7.8 Hz), 8.49 (1H, br) |
| **188** | 17 | 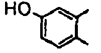 | 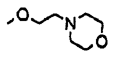 | 2HCl | M: 266-267 ; N1: 4.60-4.63 (2H, m), 8.16 (1H, s), 10.68 (1H, br) |
| **189** | 17 | 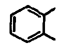 | OH | HCl | M: 214-220 ; N1: 4.26 (4H, br), 7.45 (1H, t, *J*=7.8 Hz), 7.82 (1H, s) |
| **190** | 17 | 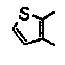 | OH | HCl | M: 207-210 ; N1: 7.40 (1H, t, *J*=7.8 Hz), 7.86 (1H, d, *J*=7.8 Hz), 8.51 (1H, d, *J*=5.3 Hz) |
| **191** | 17 | 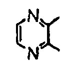 | OH | HCl | M: 262-268 (d); N1: 4.58 (4H, br), 8.87 (1H, d, *J*=2.0 Hz), 9.09 (1H, d, *J*=2.0 Hz) |
| **192** | 58 | 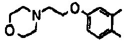 | OH | 2HCl | M: 270-273 ; N1: 4.66-4.68 (2H, m), 7.55 (1H, br s), 10.05 (1H, br) |

**Table 25**

| | | | | | |
|---|---|---|---|---|---|
| 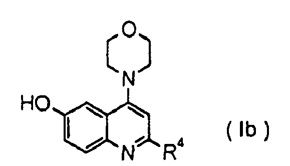 | | | | | |

| **Co** | I R⁴ | **Co** | R⁴ | **Co** | R⁴ |
|---|---|---|---|---|---|
| **B1** | 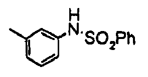 | **B2** | 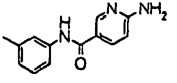 | **B3** | 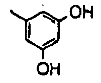 |
| **B4** | 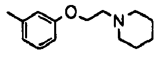 | **B5** | 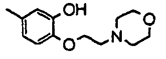 | **B6** | 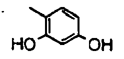 |

**Table 26**

| | | | | | | | | |
|---|---|---|---|---|---|---|---|---|
| 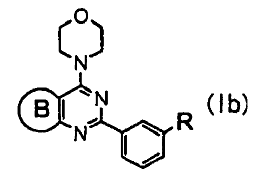 | | | | | | | | |

| **Co** | B | R | **Co** | B | R | **Co** | B | R |
|---|---|---|---|---|---|---|---|---|
| **B7** | 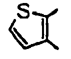 | CO₂NHMe | **B8** | 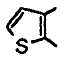 | OH | **B9** | 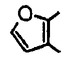 | OH |
| **B10** | 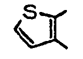 | CH₂OH | **B11** | 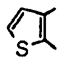 | CH₂OH | **B12** | 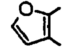 | CH₂OH |
| **B13** | 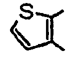 | CONH₂ | **B14** | 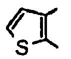 | CONH₂ | **B15** | 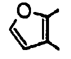 | CONH₂ |
| **B16** | 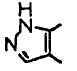 | OH | **B17** | 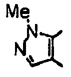 | OH | **B18** | 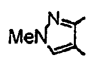 | OH |
| **B19** | 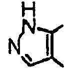 | CH₂OH | **B20** | 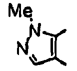 | CH₂OH | **B21** | 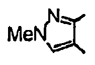 | CH₂OH |
| **B22** | 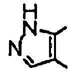 | CONH₂ | **B23** | 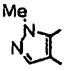 | CONH₂ | **B24** | 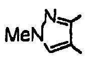 | CONH₂ |
| **B25** | 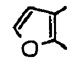 | OH | **B26** | 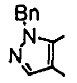 | OH | **B27** | 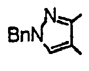 | OH |
| **B28** | 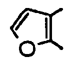 | CH₂OH | **B29** | 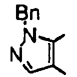 | CH₂OH | **B30** | 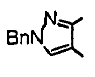 | CH₂OH |
| **B31** | 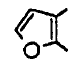 | CONH₂ | **B32** | 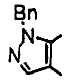 | CONH₂ | **B33** | 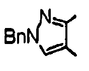 | CONH₂ |

Synthetic methods of the starting compounds shown in the foregoing tables are explained in the following Reference Examples.

Reference Example 1: A suspension of 2-chloro-3-cyanopyridine, ethyl glycolate and sodium carbonate in 3-methyl-1-butanol was refluxed for 3 days. The solvent was evaporated and water was added to the residue to crystallize to give Reference Example Compound (hereinbelow, abbreviated as Rco) 1.

Reference Example 2: A suspension of 2-chloro-3-cyanopyridine, glycine ethyl ester hydrochloride and sodium carbonate in 3-methyl-1-butanol was refluxed for 6 days. The solvent was evaporated. After the obtained residue was diluted with ethyl acetate and water, insoluble solids were filtered off. The separated organic layer was concentrated under reduced pressure. The residue was dissolved in ethanol, sodium ethoxide was added, and the mixture was stirred at room temperature for 15 minutes. The reaction solution was concentrated, and ethyl acetate.and saturated aqueous sodium hydrogencarbonate were added. The separated organic layer was concentrated under reduced pressure and the residue was purified with silica gel column chromatography to give Rco 3.

Reference Example 3: Dimethylaminopyridine and benzoyl chloride were added to a solution of 3-aminothieno[2,3-b]pyridine-2-carboxylic acid ethyl ester in pyridine. The reaction mixture was stirred at room temperature for 18 hours, and concentrated. 1M Hydrochloric acid was added, and the mixture was extracted with chloroform. The organic layer was concentrated under reduced pressure. The obtained residue was purified with silica gel column chromatography to give Rco 4.

Reference Example 4: Phosphorous oxychloride was added to a solution of Rco 49 and 4-nitrobenzoic acid and the reaction mixture was stirred at -15°C for 15 minutes. Ice and water was added to this reaction mixture. The precipitated crystals were collected to give Rco 11.

Reference Example 5: 1M Sodium hydroxide was added to a solution of Rco 4 in methanol. The reaction mixture was stirred at room temperature for 2 hours, and 1M hydrochloric acid was added. The precipitated crystals were collected to give Rco 13.

Reference Example 6: Thionyl chloride was added to Rco 13. The mixture was refluxed for 2 hours, cooled to room temperature and then concentrated. DMF and aqueous ammonia were added to the obtained residue and the reaction mixture was stirred at room temperature for 2 hours. Water was added to the resulting mixture and extracted with chloroform. The organic layer was concentrated under reduced pressure to give Rco 21.

Reference Example 7: Formamide was added to Rco 1 and the mixture was stirred at 200°C for 2 hours. After the mixture was cooled to room temperature, the precipitated crystals were collected to give Rco 29.

Reference Example 8: 2M potassium hydroxide was added to a solution of Rco 21 in methanol and the mixture was stirred at 100°C for 1 hour. After being cooled to room temperature, hydrochloric acid was added. The precipitated crystals were collected to give Rco 30.

Reference Example 9: Acetic acid and 48% hydrobromic acid were added to Rco 36 and the mixture was refluxed for 17 hours. After the reaction solution was concentrated under reduced pressure, diethyl ether was added and the reaction mixture was concentrated under reduced pressure. Sodium acetate and acetic anhydride were added to the obtained residue, and the mixture was stirred at 110°C for 2 hours. Ice and then water were added to this reaction mixture under ice cooling. The precipitated crystals were collected to give Rco 38.

Reference Example 10: Ethanol was added to a solution of 3-cyanobenzoic acid methyl ester in chloroform and gaseous hydrogen chloride was passed into the mixture at 0°C for 15 minutes. Further, the solution was sealed and the solution was stirred at 0°C for 17 hours. The reaction mixture was concentrated, ether was added, and the precipitated crystals were collected to give Rco 50.

Reference Example 11: 2-Propanol was added to a mixture of 5-acetoamidoanthranilic acid, 3-nitrobenzimidic acid ethyl ester hydrochloride and sodium methoxide, and the mixture was refluxed for 3 days. The reaction solution was allowed to cool to room temperature. The obtained solid was collected to give Rco 52.

Reference Example 12: A solution of cyclohexanecarbonyl chloride in benzene was added in dropwise to a solution of 2-amino-5-methoxybenzamide and dimethylaminopyridine in pyridine at room temperature, and the mixture was stirred for 2 hours. The reaction mixture was concentrated, and the residue was dissolved with ethyl acetate. After the organic layer was washed with 1M hydrochloric acid and saturated aqueous sodium hydrogencarbonate, it was concentrated and the obtained residue was dissolved with methanol. 2M Sodium hydroxide was added. After the reaction solution was refluxed for 2 hours, it was neutralized with 12M hydrochloric acid. The solvent was evaporated and the crystals were filtered to give Rco 67.

Reference Example 13: THF and DMF were added to a mixture of 2-amino-5-methoxybenzamide, EDCI hydrochloride, HOBt and pyrazinecarboxylic acid, and the mixture was stirred at room temperature for 3 days. The solvents were evaporated, and the crystals were collected and dissolved in methanol and 2M sodium hydroxide. The reaction solution was refluxed for 3 hours and neutralized with 12M hydrochloric acid. The obtained crystals were collected to give Rco 72.

Reference Example 14: Dimethylaminopyridine, TEA, ethanol and tosyl chloride were added to a suspension of Rco 55 in chloroform, and the reaction mixture was stirred at room temperature for 12 hours. DMSO was added to it to give a solution. Then, the reaction solution was stirred for 12 hours. Again, dimethylaminopyridine, TEA and tosyl chloride were added and the reaction solution was stirred for 18 hours. The reaction solution was concentrated, and the residue was diluted with ethyl acetate and purified according to a conventional method to give Rco 74.

Reference Example 15: 48% Hydrobromic acid was added to a solution of Rco 70 in acetic acid, and the mixture was refluxed for 2 days. After the reaction solution was allowed to cool, it was concentrated, and sodium acetate and acetic anhydride were added to the obtained residue. The reaction solution was refluxed for 3 hours. After the reaction solution was allowed to cool, it was concentrated and ether was added to the solution, followed by collection of crystals to give Rco 77.

Reference Example 16: Sodium acetate and acetic anhydride were added to Rco 60, and the mixture was refluxed for 40 minutes. After the reaction mixture was allowed to cool, the precipitated crystals were collected to give Rco 83.

Reference Example 17: Sodium methoxide was added to a solution of 3-hydroxybenzimidate ethyl ester hydrochloride and 5-hydroxyanthranilic acid in methanol, and the mixture was refluxed for 30 minutes. After the reaction solution was cooled to room temperature, the precipitate was collected. Sodium acetate and acetate anhydride were added to the obtained precipitate and the mixture was refluxed for 30 minutes. After the reaction solution was allowed to cool, the precipitated crystals were collected to give Rco 92.

Reference Example 18: Concentrated hydrochloric acid was added to Rco 52, and the mixture was stirred at 80°C. The reaction mixture was allowed to cool, filtered, and concentrated under reduced pressure to give 6-amino-2-(3-nitrophenyl)-3H-quinazoline-4-one hydrochloride. After the obtained compound was neutralized, pyridine, dimethylaminopyridine and methanesulfonyl chloride were added. The reaction solution was stirred at room temperature for 20 hours, the solvent was evaporated and the crystals were collected to give Rco 95.

Reference Example 19: 1,8-diazabicyclo[5.4.0]-7-undecene (DBU) was added to a solution of 2-chloro-3-cyanopyridine and ethyl glycolate in ethanol and the reaction mixture was refluxed for 21 hours. The resulting mixture was evaporated under reduced pressure, diluted with ethyl acetate and then, washed with water and brine. The organic layer was concentrated under reduced pressure and crystallized to give Rco 49.

Reference Example 20: Aqueous ammonia was added to a solution of Rco 96 in methanol, and the reaction solution was stirred at room temperature for 3 hours. Methanol in the reaction mixture was evaporated under reduced pressure and the crystals were collected to give Rco 116.

Reference Example 21: Aqueous ammonia was added to a solution of Rco 98 in methanol, and the reaction solution was stirred at room temperature overnight. Methanol in the reaction mixture was evaporated under reduced pressure and the crystals were collected to give Rco 117.

Reference Example 22: EDCI hydrochloride and HOBt were added to a solution of 3-acetoxybenzoic acid in DMF, and the reaction mixture was stirred at room temperature for 10 minutes. Then, 2-amino-5-methoxybenzamide was added, and the reaction mixture was stirred at room temperature for 1 hour. The solvent was evaporated under reduced pressure, and water and THF were added. After the extraction with ethyl acetate, the organic layer was washed with brine, evaporated under reduced pressure, and crystallized to give Rco 119.

Reference Example 23: Acetic anhydride was added to Rco 105 and sodium acetate, and the reaction mixture was stirred at 110°C for 1 hour and 15 minutes. Under cooling, the precipitated crystals were collected to give Rco 121.

Reference Example 24: Aqueous ammonia was added to a solution of Rco 99 in dioxane, and the reaction mixture was stirred at room temperature for 13 days. The solvent was evaporated under reduced pressure and the crystals were collected to give a mixture of amido 3-(3-methoxybenzoylamino)thiophene-2-carboxylate and Rco 122. 2M aqueous sodium hydroxide was added to a solution of this mixture in 2-propanol and the reaction solution was refluxed for 21 hours. After being cooled, it was neutralized and the precipitated crystals were collected to give Rco 122.

EXAMPLE 1: Phosphorous oxychloride was added to Rco 33 and the mixture was refluxed for 20 minutes. The reaction mixture was concentrated under reduced pressure, and was azeotropically concentrated with toluene. Morpholine was added to the obtained residue and the mixture was refluxed for 10 minutes. The reaction solution was concentrated under reduced pressure and the obtained crystals were washed with chloroform and water to give Compound (hereinafter, abbreviated as Co) 1.

EXAMPLE 2: 1-Benzyl piperidine-1,2-dicarboxylate, HOBt and EDCI hydrochloride were added to a solution of a free form of Co 31 in DMF, and the mixture was stirred at room temperature for 7 hour. The solvent was evaporated under reduced pressure. After being diluted with ethyl acetate, the organic layer was washed with saturated aqueous sodium hydrogencarbonate and brine. After the solution was dried over anhydrous sodium sulfate, the solvent was evaporated under reduced pressure. The obtained residue was purified with silica gel column chromatography and crystallized to give Co 3.

EXAMPLE 3: Benzenesulfonyl chloride (6.02ml) was added to a solution of a free form of Co 31 (13.6g) in pyridine (480ml) at 0°C and the mixture was stirred at room temperature for 1.5 hours. The solvent was evaporated under reduced pressure. The obtained residue was dissolved in ethyl acetate and the solution was washed with saturated aqueous sodium hydrogencarbonate and brine. After the solution was dried over anhydrous sodium sulfate, the solvent was evaporated under reduced pressure. The obtained residue was purified with silica gel column chromatography (eluent; chloroform : methanol = 96 : 4) and recrystallized (ethanol) to give Co 8 (15.6g).

EXAMPLE 4: Picolinoyl chloride hydrochloride (9.40g) and TEA (14.7ml) were added to a solution of a free form of Co 31 (15.3g) in THF (1L) at 0°C and the mixture was stirred at room temperature for 1.5 hours. Then, additional picolinoyl chloride hydrochloride (4.00g) was added and the mixture was stirred at room temperature for 30 minutes. The reaction mixture was concentrated under reduced pressure. After dissolving in ethyl acetate, the solution was washed with saturated aqueous sodium hydrogencarbonate and brine. After being dried over anhydrous sodium sulfate, the solvent was evaporated under reduced pressure and the obtained residue was purified with silica gel column chromatography (chloroform : methanol = 96 : 4) and recrystallized (ethanol) to give Co 10 (15.4g).

EXAMPLE 5: 3-Hydroxypicolinic acid (140mg), EDCI hydrochloride (190mg) and HOBt (135mg) were added to a solution of a free form of Co 31 (300mg) in DMF (20ml) and the mixture was stirred at room temperature for 2 hours. The solvent was evaporated under reduced pressure. The obtained residue was dissolved with ethyl acetate and THF. The solution was washed with water and saturated aqueous sodium hydrogencarbonate. After the organic layer was dried over anhydrous magnesium sulfate, the solvent was evaporated under reduced pressure. The obtained residue was purified with silica gel column chromatography (chloroform : methanol = 100 : 1). 4M Hydrogen chloride/ethyl acetate was added to a solution of the obtained residue in chloroform and methanol. The solvent was evaporated under reduced pressure, and the obtained solid was crystallized from methanol to give Co 12 (207mg).

EXAMPLE 6: A suspension of a free form of Co 31 (300mg), succinic anhydride (519mg) and acetic acid (1ml) was stirred at 100°C for 30 minutes. The solvent was evaporated under reduced pressure. The obtained residue was purified with silica gel column chromatography (chloroform : methanol = 98 : 2) and recrystallized (methanol) to give Co 24 (106mg).

EXAMPLE 7: To a solution of Co 18 (300mg) in THF (12ml) and ethanol (12ml), 10% Pd-C (35mg) was added, and the mixture was stirred at room temperature under hydrogen atmosphere (1atm) for 4 hours. The reaction solution was filtered by Celite. After the filtrate was concentrated under reduced pressure, the obtained residue was purified with silica gel column chromatography (chloroform : methanol = 98 : 2 - 80 : 20) to give a free form of Co 25 (178mg). 1M Hydrochloric acid (1.00ml) was added to a solution of the obtained free form (153mg) in THF (35ml) and methanol (20ml). The reaction solution was stirred at room temperature, then concentrated under reduced pressure, and recrystallized (methanol) to give dihydrochloride of Co 25 (119mg).

EXAMPLE 8: A suspension of a free form of Co 31 (300mg), succinic anhydride (519mg) and acetic acid (1ml) was stirred at 100°C for 30 minutes. The solvent was evaporated under reduced pressure. The obtained residue was purified with silica gel column chromatography (chloroform : methanol = 98 : 2) and recrystallized (methanol) to give Co 29 (57mg).

EXAMPLE 9: N-Carboethoxyphthalimide (262mg) and TEA (0.166ml) were added to a solution of a free form of Co 31 (346mg) in THF (60ml) and the mixture was stirred at 80°C for 1 day. After the reaction mixture was allowed to cool, water was added, and collected to give a free form of Co 30 (374mg). 1M Hydrochloric acid (1.55ml) was added to a solution of the obtained free form (371mg) in THF (200ml) and the solution was stirred at room temperature. The precipitated crystals were collected to give hydrochloride of Co 30 (287mg).

EXAMPLE 10: Co 1 (22.4g) and ammonium chloride (1.59g) were suspended in a mixture of ethanol (717ml) and water (269ml). Then, iron (33.2g) was added and the solution was refluxed for 9 hours. While the reaction solution was still hot, hot THF was added, and the mixture was filtered with Celite. After most of the solvent was evaporated under reduced pressure, the precipitate was collected, and washed with diethyl ether to give a free form of Co 31 (18.9g). 1M Hydrochloric acid (0.870ml) was added to a solution of the obtained free form (101mg) in THF (25ml) and the solution was stirred at room temperature. The precipitated crystals were collected, and washed with methanol to give dihydrochloride of Co 31 (75mg).

EXAMPLE 11: A solution of Rco 1 (1.03g) in formamide (12ml) was refluxed for 2 hours. After the reaction mixture was cooled to room temperature, the obtained solids were collected to give Rco 29 (648mg). Phosphorous oxychloride (7ml) was added to a solution of obtained Rco 29 (630mg) in pyridne (3.5ml). The reaction mixture was refluxed for 2.5 hours. After being cooled to room temperature, the solvent was evaporated. Toluene (7ml) was added to the obtained residue. After morpholine (7ml) was slowly added in dropwise under ice cooling, the reaction mixture was refluxed for 3.5 hours. Further, THF (3ml) and morpholine (20ml) were added, and the reaction mixture was refluxed for 5 days, and then concentrated under reduced pressure. After the residue was diluted with ethyl acetate, the crystals were collected, washed with ethyl acetate, saturated aqueous sodium hydrogencarbonate and water, and recrystallized (ethanol) to give Co 34 (372mg).

EXAMPLE 12: Phosphorous oxychloride (5ml) was added to Rco 32 (396mg) and the mixture was refluxed for 50 minutes. The solvent was evaporated under reduced pressure. After morpholine (10ml) was slowly added in dropwise into the obtained residue under ice cooling, the reaction mixture was refluxed for 30 minutes. The reaction mixture was concentrated under reduced pressure. The obtained crystals were washed with ethyl acetate and water, and recrystallized (ethanol) to give Co 35 (411mg).

EXAMPLE 13: Ethylenediamine (1.85ml) was added to Co 11 (303mg) and the mixture was stirred at 90°C for 2 hours. The solvent was evaporated under reduced pressure. The obtained residue was purified with silica gel column chromatography (chloroform : methanol = 80 : 20) and crystallized (methanol) to give a free form of Co 40 (269mg). The obtained free form (266mg) was subjected to salt formation as described in EXAMPLE 7, and the obtained residue was recrystallized (methanol) to give dihydrochloride of Co 40 (153mg).

EXAMPLE 14: DMF (10ml) was added to Co 11 (285mg) and the solution was stirred at 110°C for 2 hours and at 80°C for 27 hours. The solvent was evaporated under reduced pressure. The obtained residue was dissolved in ethyl acetate and THF. The reaction solution was washed with aqueous sodium hydrogencarbonate and brine, dried over anhydrous sodium sulfate, and the solvent was evaporated under reduced pressure. The obtained residue was purified with silica gel column chromatography (chloroform : methanol = 98 : 2) to give a free form of Co 44 (167mg). 1M Hydrochloric acid (0.283ml) was added to a solution of the obtained free form (70mg) in THF (5ml) and methanol (10ml), and the solution was stirred at room temperature. The precipitated crystals were collected to give dihydrochloride of Co 44 (72mg).

EXAMPLE 15: Benzoyl chloride (0.118ml) was added to a solution of a free form of Co 31 (297mg) in pyridine (20ml) under ice cooling and the mixture was stirred at room temperature for 20 minutes. The solvent was evaporated under reduced pressure. The obtained residue was dissolved in ethyl acetate and THF. The reaction solution was washed with aqueous sodium hydrogencarbonate and brine. After being dried over anhydrous sodium sulfate, the solvent was evaporated under reduced pressure. The obtained residue was recrystallized (methanol) to give a free form of Co 45 (301mg). The obtained free form (287mg) was subjected to salt formation as described in EXAMPLE 7 to give hydrochloride crystals of Co 45 (249mg).

EXAMPLE 16: 1M Sodium hydroxide (11ml) was added in two portions to a solution of a free form of Co 48 (171mg) in methanol (60ml) and THF (30ml), and the mixture was stirred at room temperature for 2.5 hours. The reaction mixture was acidified with 1M hydrochloric acid, and the organic solvent was evaporated under reduced pressure. The precipitate was collected, and washed with water and diethyl ether. The obtained crystals were recrystallized (methanol / diethyl ether) to give Co 49 (116mg).

EXAMPLE 17: Phosphorous oxychloride (5ml) was added to Rco 38 (452mg), and the mixture was refluxed for 30 minutes. After the reaction solution was cooled to room temperature, it was concentrated under reduced pressure. After adding THF (5ml) and then slowly adding morpholine (4ml) in dropwise to the obtained residue under ice cooling, the ice bath was removed and the solution was refluxed for 1 hour. After the reaction mixture was cooled to room temperature the solvent was evaporated under reduced pressure. The obtained solid was washed with water and diethyl ether and purified with silica gel column chromatography (chloroform: methanol = 98 : 2) to give a free form of Co 50 (411mg). The obtained free form (183mg) was subjected to salt formation as described in EXAMPLE 7 and recrystallized (methanol) to give hydrochloride of Co 50 (129mg).

EXAMPLE 18: 4-(2-Chloroethyl)morpholine hydrochloride (1.53g) and potassium carbonate (1.90g) were added to a solution of a free form of Co 50 (956mg) in DMF (35ml), and the mixture was stirred at 70°C for 2.5 days. After the reaction solution was cooled to room temperature, the solvent was evaporated under reduced pressure. The obtained solid was washed with water and diethyl ether and purified with silica gel column chromatography (chloroform : methanol = 98 : 2) and crystallized (methanol) to give a free form of Co 54 (1.16g). 4M Hydrochloric acid / ethyl acetate (1.14ml) was added to a solution of the obtained free form (1.05g) in THF (140ml) and methanol (70ml), and the solution was stirred at room temperature. The solvents were evaporated under reduced pressure, and the material was recrystallized (methanol) to give dihydrochloride crystals of Co 54 (1.18g).

EXAMPLE 19: Phosphorous oxychloride (5ml) was added to 2-phenyl-3H-quinazoline-4-one (450mg), and the mixture was refluxed for 3 hours. The reaction mixture was concentrated. Saturated aqueous sodium hydrogencarbonate was added and extracted with ethyl acetate. After the organic layer was dried over anhydrous magnesium sulfate, the solvent was evaporated under reduced pressure. The obtained colorless crystals were dissolved in benzene (10ml) and morpholine (325mg) was added. The reaction mixture was refluxed overnight. Insoluble materials were filtered off and the filtrate was diluted with ethyl acetate. The organic layer was washed with saturated aqueous sodium hydrogencarbonate, dried over anhydrous magnesium sulfate, and then concentrated under reduced pressure. The obtained residue was purified with column chromatography (hexane : ethyl acetate = 5 : 1) and recrystallized (hexane / benzene) to give Co 82 (136mg).

EXAMPLE 20: Sodium cyanide (132mg) was added to a solution of Co 84 (190mg) in DMSO (5ml), and the mixture was stirred at 180°C for 2 hours. After the reaction mixture was allowed to cool, water was added to it and the mixture was extracted with ethyl acetate. After the organic layer was washed with brine and dried over anhydrous sodium sulfate, the solvent was evaporated under reduced pressure. The obtained residue was recrystallized (hexane / ethyl acetate) to give Co 92 (40mg).

EXAMPLE 21: Iron (415mg) was added to a solution of Co 85 (500mg) in acetic acid (12ml), and the mixture was stirred at 105°C for 1 hour. After the reaction solution was allowed to cool, chloroform and 1M sodium hydroxide were added. The solution was filtered with Celite and the filtrate was extracted with chloroform. The organic layer was washed with brine, dried over anhydrous sodium sulfate, filtered and concentrated under reduced pressure. 1M Hydrochloric acid (10ml) was added to the obtained residue and the mixture was stirred at 85°C for 90 minutes. After the mixture was allowed to cool, 1M sodium hydroxide was added and extracted with chloroform, and the organic layer was washed with brine. After the solution was dried over anhydrous sodium sulfate, the solvent was evaporated under reduced pressure. The obtained residue was purified with silica gel column chromatography (eluent; chloroform : methanol = 50 : 1), and recrystallized (chloroform / hexane) to give Co 97 (374mg).

EXAMPLE 22: Acetic anhydride (3ml) was added to a solution of Co 97 (149mg) in formic acid (3ml), and the mixture was stirred at room temperature for 2 hours. Water was added and the mixture was extracted with ethyl acetate. The organic layer was washed with saturated aqueous sodium hydrogencarbonate and brine, dried over anhydrous sodium sulfate, and concentrated under reduced pressure. The residue was purified with silica gel column chromatography (eluent; chloroform : methanol = 50 : 1), and recrystallized (chloroform / hexane) to give Co 98 (46mg).

EXAMPLE 23: Phosphorous oxychloride (3ml) was added to Rco 74 (270mg), and the mixture was refluxed for 0.5 hours. The reaction mixture was concentrated under reduced pressure, and morpholine (10ml) was added. After the reaction mixture was refluxed for 1 hour, the reaction mixture was concentrated under reduced pressure and diluted with ethyl acetate. The organic layer was washed with saturated aqueous sodium hydrogencarbonate, dried over anhydrous magnesium sulfate, and concentrated under reduced pressure. After purifying by silica gel column chromatography (eluent; hexane : ethyl acetate = 5 : 1), ethanol (2ml) and 20% potassium hydroxide (100mg) were added, and the mixture was stirred at room temperature for 40 minutes. The reaction mixture was extracted with ethyl acetate, and the organic layer was washed with saturated aqueous sodium hydrogencarbonate, dried over anhydrous magnesium sulfate, and concentrated under reduced pressure. The obtained crystals were washed with a mixture of ethyl acetate and hexane, and recrystallized (ethyl acetate /hexane) to give Co 99 (30mg).

EXAMPLE 24: Sodium hydroxide (43mg), potassium carbonate (37mg) and tetra-n-butylammonium hydrogensulfate (2mg) were added to a solution of Co 86 (95mg) in toluene (15ml), and the mixture was stirred at 35°C for 0.5 hour, followed by addition of dimethylsulfate (34mg) and stirring at 35°C for 2 hours. The reaction solution was filtered and the filtrated was concentrated under reduced pressure. The residue was purified with silica gel column chromatography (eluent; chloroform : methanol = 20 : 1), and recrystallized (chloroform / hexane) to give Co 105 (62mg).

EXAMPLE 25: p-Toluenesulfonyl chloride (124mg) and pyridine (lml) were added to a solution of Co 97 (200mg) in chloroform (6ml), and the mixture was stirred at room temperature for 45 minutes. 1M Hydrochloric acid was added and extracted with ethyl acetate. The organic layer was washed with saturated aqueous sodium hydrogencarbonate and brine, dried over anhydrous sodium sulfate, and concentrated under reduced pressure. The residue was recrystallized (chloroform / hexane) to give Co 106 (165mg).

EXAMPLE 26: 35% Formalin (5ml) and formic acid (5ml) were added to Co 97 (250mg), and the mixture was stirred at 100°C for 90 minutes. After the mixture was allowed to cool, 1M sodium hydroxide was added and the reaction mixture was extracted with ethyl acetate. The organic layer was washed with brine, dried over anhydrous sodium sulfate, and concentrated under reduced pressure. The residue was purified with silica gel column chromatography (eluent; hexane : ethyl acetate = 4 : 1), and recrystallized (chloroform / hexane) to give Co 107 (133mg).

EXAMPLE 27: Phosphorous oxychloride (50ml) was added to Rco 76 (6.2g), and the mixture was refluxed for 1 hour. The reaction solution was concentrated under reduced pressure and the residue was dissolved in chloroform. The chloroform layer was washed twice with saturated aqueous sodium hydrogencarbonate, dried over anhydrous magnesium sulfate, and concentrated under reduced pressure to give 6.0g of crystals. To 802mg of the crystals, thiomorpholine (500mg) and benzene (10ml) were added. The reaction mixture was heated with stirring at 70°C for 1 hour, and then diluted with ethyl acetate. The organic layer was washed with saturated aqueous sodium hydrogencarbonate and brine, dried over anhydrous magnesium sulfate, concentrated under reduced pressure, and purified with silica gel column chromatography (hexane : ethyl acetate = 5 : 1) to give p-toluenesulfonic acid 4-thiomorpholino 2-phenylquinazoline-6-yl (828mg). 802mg of this compound was dissolved in methanol (10ml) and THF (10ml). 20% Potassium hydroxide (1.0g) was added to the solution and the mixture was stirred at 70°C for 1 hour. Water and 1M hydrochloric acid was added to neutralize the solution. Then, the solution was extracted with ethyl acetate, and the organic layer was washed with brine. After the solution was dried over anhydrous magnesium sulfate, the solution was concentrated under reduced pressure and recrystallized (ethyl acetate - hexane) to give Co 108 (151mg).

EXAMPLE 28: Ammonium acetate (1.2g) was added to acetic acid 2-methyl-4-oxo-4H-benzo[d][1,3]oxazine-6-yl ester (3g), and the mixture was stirred at 150°C for 30 minutes. After cooling to 80°C, methanol was added to the mixture and the mixture was stirred at 80°C for 1 hour. After the mixture was allowed to cool, the precipitated crystals were collected and washed with methanol to give crystals (930mg). To a solution of the obtained crystals (918mg) in DMSO (10ml) / chloroform (5ml), toluenesulfonyl chloride (1ml), TEA (1ml) and a catalytic amount of dimethylaminopyridine were added. The mixture was stirred at room temperature for 8 hours, and then extracted with ethyl acetate. The organic layer was washed with saturated aqueous sodium hydrogencarbonate and brine, dried over anhydrous sodium sulfate, and the solvent was evaporated under reduced pressure. Phosphorous oxychloride (15ml) was added to the obtained residue and the mixture was refluxed for 15 hours. After the reaction solution was allowed to cool, phosphorous oxychloride was evaporated under reduced pressure. The residue was extracted with chloroform and washed with saturated aqueous sodium hydrogencarbonate. The organic layer was dried over anhydrous sodium sulfate, and concentrated under reduced pressure. The residue was purified with silica gel column chromatography (eluent; chloroform : methanol = 50 : 1) to give a liquid material (577mg). To a solution of the obtained material (577mg) in toluene (20ml), morpholine (2g) was added, and the mixture was refluxed for 16 hours. The reaction solution was allowed to cool, and concentrated under reduced pressure. To a solution of the obtained residue in ethanol (15ml), 20% potassium hydroxide (1ml) was added, and the mixture was stirred at room temperature for 1 hour. The solvent was evaporated under reduced pressure. The residue was purified with silica gel column chromatography (eluent; chloroform : methanol = 20 : 1), and recrystallized (chloroform / methanol / hexane) to give Co 109 (207mg).

EXAMPLE 29: Phosphorous oxychloride (10mg) was added to Rco 78 (590mg), and the mixture was refluxed for 0.5 hours. The reaction mixture was concentrated, diluted with chloroform, and washed with saturated aqueous sodium hydrogencarbonate. The organic layer was dried over anhydrous magnesium sulfate, and concentrated under reduced pressure. To the obtained colorless crystals, morpholine (10ml) was added, and the mixture was refluxed for 12 hours. The reaction mixture was diluted with chloroform, washed with water and saturated aqueous sodium hydrogencarbonate, and dried over anhydrous magnesium sulfate. The obtained organic layer was concentrated under reduce pressure. The residue was crystallized from chloroform-methanol, and further recrystallized (ethyl acetate / hexane) to give Co 110 (126mg).

EXAMPLE 30: Acetic acid (20ml) and 48% hydrogen bromide (20ml) were added to Rco 67 (957mg), and the mixture was stirred at an oil bath temperature of 135°C for 13 hours. After the mixture was allowed to cool to room temperature, precipitate was collected as a mixture of a starting material and a desired compound. To the obtained solid, acetic anhydride (30ml) and sodium acetate (112mg) were added and the reaction mixture was refluxed for 30 minutes. The reaction solution was allowed to cool, precipitate was collected. To the obtained solids, phosphorous oxychloride (10ml) was added, and the mixture was refluxed for 30 minutes and concentrated under reduced pressure. The obtained residue was dissolved in chloroform and the solution was washed with saturated aqueous sodium hydrogencarbonate. The organic layer was dried over anhydrous magnesium sulfate, and concentrated under reduced pressure. To the obtained residue, morpholine (20ml) was added and the mixture was refluxed for 15 hours. The reaction mixture was concentrated under reduced pressure and purified with silica gel column chromatography (eluent; chloroform : methanol = 50 : 1). The obtained crystals were washed with a mixture of chloroform and ether to give Co 127 (193mg).

EXAMPLE 31: Iron (396mg) was added to a solution of Co 111 (500mg) in acetic acid (12ml), and the mixture was stirred at 105°C for 45 minutes. The reaction solution was allowed to cool. Chloroform and 1M sodium hydroxide were added to it. The mixture was filtered and extracted with chloroform. The organic layer was washed with brine, dried over anhydrous sodium sulfate, and concentrated under reduced pressure. The obtained residue was purified with silica gel column chromatography (eluent; chloroform : methanol = 10 : 1) and recrystallized (chloroform / methanol / hexane) to give Co 129 (120mg).

EXAMPLE 32: 1M Sodium hydroxide (8ml) was added to a solution of Co 116 (564mg) in ethanol (8ml) and THF (8ml), and the reaction mixture was stirred at room temperature for 15 hours. 1M Hydrochloric acid (8ml) was added and the solution was extracted with ether. The organic layer was washed with brine, dried over anhydrous sodium sulfate, and concentrated under reduced pressure. The residue was recrystallized (methanol / ether / hexane) to give Co 130 (163mg).

EXAMPLE 33: Lithium aluminum hydride (67mg) was added to a solution of Co 117 (325mg) in THF (40ml), and the reaction mixture was stirred at 0°C for 2 hours. Water (0.1ml), 1M sodium hydroxide (0.1ml), and then water (0.3ml) were added and the mixture was stirred at room temperature for 30 minutes. The mixture was dried over anhydrous sodium sulfate, filtered through silica gel, and concentrated under reduced pressure. The residue was recrystallized (THF /hexane) to give Co 131 (158mg).

EXAMPLE 34: Co 112 (860mg) was dissolved in a mixture of THF (30ml), methanol (30ml) and ethanol (30ml). 10% Pd-C (130mg) was added and the reaction mixture was stirred under hydrogen atmosphere (1atm) at room temperature for 2 hours. Insoluble materials were removed by filtration and the filtrate was concentrated to give 780mg of solid. Of the solid, 202mg was recrystallized (ethanol / methanol) to give Co 133 (148mg).

EXAMPLE 35: Co 133 (202mg) was dissolved in pyridine (10ml). Methanesulfonyl chloride (96mg) was added to the reaction solution. The reaction mixture was stirred for 15 hours, and concentrated under reduced pressure. The obtained residue was dissolved in ethyl acetate. The organic layer was washed with water, dried over anhydrous magnesium sulfate, and concentrated under reduced pressure. The obtained crystals were purified with column chromatography (chloroform - methanol = 100 : 1) and recrystallized (ethanol - ethyl acetate - hexane) to give Co 135.

EXAMPLE 36: HOBt (75mg) and EDCI hydrochloride (106mg) were added to a solution of Co 131 (177mg) in DMF (12ml), and the reaction solution was stirred at 0°C for 30 minutes and then at room temperature for 30 minutes. Aqueous ammonia (2ml) was added and the mixture was stirred at room temperature for 3 hours. The reaction mixture was extracted with chloroform, and the organic layer was washed with water, dried over anhydrous sodium sulfate, and concentrated under reduced pressure. The residue was purified with silica gel column chromatography (eluent; chloroform : methanol = 10 : 1) and recrystallized (chloroform / methanol / hexane) to give Co 136 (39mg).

EXAMPLE 37: Phosphorous oxychloride (15ml) was added to Rco 87 (1.1g), and the mixture was refluxed for 1 hour. The reaction mixture was allowed to cool, and concentrated under reduced pressure. The mixture was diluted with chloroform and the organic layer was washed with saturated aqueous sodium hydrogencarbonate, dried over anhydrous sodium sulfate, and concentrated under reduced pressure. To a solution of the obtained residue in toluene (40ml), morpholine (5g) was added and the reaction solution was refluxed for 17 hours, allowed to cool, and concentrated under reduced pressure. The residue was purified with silica gel column chromatography (eluent; chloroform : methanol = 30 : 1), and recrystallized (chloroform / ether / hexane) to give Co 138 (869mg).

EXAMPLE 38: Acetic anhydride (0.75ml) and pyridine (1ml) were added to a solution of Co 129 (457mg) in DMF (10ml), and the reaction mixture was stirred at room temperature for 1 hour. 1M Sodium hydroxide (15ml) and water were added and the reaction mixture was extracted with chloroform. The organic layer was dried over anhydrous sodium sulfate, and concentrated under reduced pressure. The residue was purified with silica gel column chromatography (eluent; chloroform : methanol = 10 : 1), recrystallized (chloroform / methanol / hexane), and washed with ether to give Co 139 (358mg).

EXAMPLE 39: Benzene (10ml), benzaldehyde (249mg) and THF (10ml) were added to Co 133 (476mg). The reaction mixture was azeotropically refluxed for 2 hours and concentrated under reduced pressure, and the obtained residue was dissolved in methanol (20ml). Under ice cooling, sodium borohydride (50mg) was added. The reaction mixture was stirred at room temperature for 1 hour, and concentrated under reduced pressure. The residue was dissolved in ethyl acetate, and the organic layer was washed with water and brine, dried over anhydrous magnesium sulfate, and concentrated under reduced pressure. The residue was purified with silica gel column chromatography (chloroform : methanol = 100 : 1), and recrystallized (ethyl acetate /hexane) to give Co 140 (259mg).

EXAMPLE 40: Co 133 (3.0g) was dissolved in pyridine (50ml). Benzenesulfonyl chloride (1.9g) was added to the mixture at room temperature. After stirring the mixture for 2 hours, the solvent was evaporated under reduced pressure. Ethyl acetate and water were added to the obtained residue and then the organic layer was washed with brine three times. The obtained organic layer was dried over anhydrous magnesium sulfate, and concentrated under reduced pressure. The obtained solid was purified with silica gel column chromatography (chloroform : methanol = 100 : 1) and recrystallized (ethanol) to give Co 141 (3.0g).

EXAMPLE 41: THF (15ml) and phenyl isocyanate (207mg) were added to Co 133 (540mg). After the mixture was refluxed for 3 hours, phenyl isocyanate (500mg) was again added. The mixture was then refluxed for 4 hours. 1M Sodium hydroxide (5ml) was added. After the mixture was stirred for 15 minutes, 1M hydrochloric acid (5ml) was added to neutralize it. The reaction mixture was extracted with chloroform, dried over anhydrous magnesium sulfate, and concentrated under reduced pressure. The obtained residue was purified with silica gel column chromatography (chloroform : methanol = 50 : 1) and recrystallized (2-propanol / diethyl ether /hexane) to give Co 148 (180mg).

EXAMPLE 42: THF (15ml) and TEA (520mg) were added to Co 133 (440mg). Phenyl chloroformate (498mg) was added in dropwise to the reaction solution at room temperature, and stirred at room temperature for 2 hours. After adding methanol, 1M sodium hydroxide (5ml) was added under ice cooling and the reaction mixture was stirred for 20 minutes. 1M Hydrochloric acid (5ml) was added to neutralize it. The reaction mixture was extracted with chloroform, dried over anhydrous magnesium sulfate, and concentrated under reduced pressure. The obtained residue was purified with silica gel column chromatography (chloroform : methanol = 50 : 1) and recrystallized (ethyl acetate) to give Co 149 (189mg).

EXAMPLE 43: Isonicotinoyl chloride hydrochloride (280mg) and pyridine (0.127ml) were added to a solution of Co 133 (253mg) in THF (10ml), and the reaction mixture was stirred at room temperature for 5.5 hours. Further, TEA (0.1ml) was added and the mixture was stirred at room temperature for 2.5 hours. Then, 1M sodium hydroxide (0.786ml) and methanol (4ml) were added, and the reaction mixture was stirred at room temperature for 30 minutes to cleave the ester. After neutralization, most of the solvent was evaporated, the resulting precipitate was collected, washed with ethyl acetate and water, and recrystallized (ethanol) to give Co 150 (71mg).

EXAMPLE 44: Chlorooxoacetic acid ethyl ester (190mg) and TEA (lml) were added to a solution of Co 97 (285mg) in chloroform (12ml), and the mixture was stirred at room temperature for 16 hours. The reaction mixture was extracted with chloroform, washed with water, dried over anhydrous sodium sulfate, and concentrated under reduced pressure. The residue was purified with silica gel column chromatography (eluent; chloroform : methanol = 50 : 1), and recrystallized (chloroform / methanol / hexane) to give Co 159 (103mg).

EXAMPLE 45: Benzoyl isothiocyanate (1.2ml) was added to a solution of Co 97 (1.2g) in chloroform (30ml) under ice cooling, and the mixture was stirred at room temperature for 3 hours. The precipitated crystals were collected. To the obtained crystals, 40% methylamine / methanol was added and the reaction mixture was stirred at room temperature for 1 hour, concentrated under reduced pressure, and purified with silica gel column chromatography to give 1-(4-morpholino-2-phenylquinazoline-6-yl)thiourea (1.1g). To 266mg of this compound, ethanol (5ml), methanol (3ml) and 40% chloroacetaldehyde (300mg) were added. The reaction mixture was stirred for 4 days, diluted with ethyl acetate, washed with saturated aqueous sodium hydrogencarbonate, and dried over anhydrous magnesium sulfate. The residue was purified with silica gel column chromatography (hexane : ethyl acetate = 2 :1), and recrystallized (ethanol / hexane) to give Co 160 (49mg).

EXAMPLE 46: Acetic acid (5ml) and 48% hydrobromic acid (5ml) were added to Co 91 (500mg), and the reaction solution was refluxed for 13 hours, and then concentrated. The reaction mixture was neutralized with 1M sodium hydroxide and saturated aqueous sodium hydrogencarbonate, and extracted with ethyl acetate. The organic layer was dried over anhydrous magnesium sulfate, and concentrated under reduced pressure. The obtained solid was purified with silica gel column chromatography (hexane : ethyl acetate = 1 :1), and recrystallized (ethanol) to give Co 162 (112mg).

EXAMPLE 47: 3-Morpholinopropanol (93mg) and a free form of Co 50 (203mg) were added to a solution of diethyl azodicarboxylate (0.101ml) and triphenylphosphine (168mg) in THF (20ml) and the mixture solution was stirred at 60°C for 13 hours. Additional diethyl azodicarboxylate (0.1ml), triphenylphosphine (170mg) and 3-morpholinopropanol (93mg) were added and the mixture was stirred at 60°C. This addition of the reagents was repeated again. After the reaction mixture was allowed to cool, water and ethyl acetate were added, and the reaction mixture was basified with saturated aqueous sodium hydrogencarbonate. After extraction with ethyl acetate, the solution was washed with brine. After the solution was dried over anhydrous sodium sulfate, the solvent was evaporated under reduced pressure. The obtained residue was purified with silica gel column chromatography (chloroform : methanol = 98 :2), and recrystallized (methanol) to give a free form of Co 58 (174mg). The obtained free form (71mg) was subjected to salt formation as described in EXAMPLE 18, and recrystallized (methanol) to give hydrochloride of Co 58 (68mg).

EXAMPLE 48: Potassium carbonate (1.10g) was added to a solution of Co 61 (1.48g) in methanol (15ml) and water (15ml). After the mixture was stirred at 80°C, additional methanol (8ml) and water (8ml) were added and the mixture was stirred at 80°C for 12 hours. After the reaction mixture was allowed to cool, crystals were collected, purified with silica gel column chromatography (chloroform : methanol = 98 :2), and crystallized (methanol) to give a free form of Co 59 (1.13g). The obtained free form (160mg) was subjected to salt formation as described in EXAMPLE 18, and recrystallized (methanol) to give dihydrochloride of Co 59 (146mg).

EXAMPLE 49: Anhydrous trifluoroacetic acid (1.07ml) and dimethylaminopyridine (78mg) were added to a solution of a free form of Co 31 (2.21g) in pyridine (70ml) under ice cooling. After the mixture solution was stirred for 1 hour, more anhydrous trifluoroacetic acid (0.5ml) and dimethylaminopyridine (30mg) were added, and the mixture was stirred under ice cooling for 1 hour. The solvent was evaporated under reduced pressure, water and ethyl acetate were added, and the precipitated crystals were filtered and washed with ethyl acetate to give Co 60 (2.66g).

EXAMPLE 50: Water (0.645ml), dibromoethane (1.11ml), tetrabutylammonium hydrogensulfate (22mg) and 2M aqueous sodium hydroxide (2.58ml) were added to a free form of Co 50 (1.29g), and the mixture was stirred 60°C for 6 hours. Chloroform was added to the mixture, unsoluble materials were filtered, and the filtrate was extracted with chloroform and then washed with brine. After the solution was dried over anhydrous sodium sulfate, the solvent was evaporated under reduced pressure, and the obtained residue was purified with silica gel column chromatography (chloroform : methanol = 98 :2) to give Co 62 (376mg).

EXAMPLE 51: 1-Methylpiperazine (139mg) and potassium carbonate (256mg) were added to a solution of Co 62 (211mg) in DMF (5ml) and the mixture solution was stirred at 60°C for 4 hours. The solvent was evaporated under reduced pressure, water and THF were added to the obtained residue, the mixture was extracted with ethyl acetate, and then the solvent was evaporated under reduced pressure. The obtained residue was purified with silica gel column chromatography (chloroform : methanol = 97 :3 ~ 95 : 5) to give a free form of Co 64 (198mg). The obtained free form (198mg) was subjected to salt formation as described in EXAMPLE 18, and recrystallized (methanol) to give trihydrochloride of Co 64 (160mg).

EXAMPLE 52: tert-Butyl piperazine-1-carboxylate (285mg) and potassium carbonate (282mg) were added to a solution of Co 62 (232mg) in DMF (5ml) and the mixture solution was stirred at 60°C for 17 hours. The solvent was evaporated under reduced pressure, water was added to the obtained residue, and the resulting mixture was extracted with ethyl acetate and then washed with brine. After the solution was dried over anhydrous sodium sulfate, the solvent was evaporated under reduced pressure and the obtained residue was purified with silica gel column chromatography (chloroform : methanol = 99 : 1) to give solid (227mg). 4M Hydrogen chloride/ethyl acetate (lml) were added to a solution of the obtained solid (212mg) in dioxane (3ml) and methanol (3ml), and the mixture was stirred at room temperature for 4 hours. The resulting mixture was concentrated and the residue was recrystallized (methanol) to give Co 69 (144mg).

EXAMPLE 53: Paraformaldehyde (15mg) and acetic acid (81ml) were added to a solution of a free form of Co 59 (216mg) in THF (3ml). After the mixture was stirred at room temperature for 10 minutes, sodium triacetoxyborohydride (199mg) was added and the mixture was stirred at room temperature for 21 hours. Then, liquid formaldehyde (0.44ml), acetic acid (5.5ml) and sodium triacetoxyborohydride (704mg) were added in 3 divided portions, and the reaction solution was stirred at room temperature for 4 days. The reaction mixture was neutralized with 2M aqueous sodium hydroxide, and THF was added. After the reaction solution was extracted with ethyl acetate, it was washed with brine. After it was dried over anhydrous sodium sulfate, the solvent was evaporated under reduced pressure, and the obtained residue was purified with silica gel column chromatography (chloroform : methanol = 98 : 2) to give a free form of Co 70 (162mg). The obtained free form (48mg) was subjected to salt formation as described in EXAMPLE 18, and recrystallized (methanol) to give dihydrochloride of Co 70 (53mg).

EXAMPLE 54: After a free form of Co 50 (322mg), 1,3-dioxolane-2-one (814mg) and potassium carbonate (192mg) were stirred at 100°C for 2 hours, more 1,3-dioxolane-2-one (680mg) was added and the mixture was stirred at 100°C for 17 hours. Then, DMF (3ml) was added and the mixture was stirred at 100°C for 2 hours, and further 1,3-dioxolane-2-one (670mg) was added and the mixture was stirred at 100°C for 20 hours. After the reaction mixture was allowed to cool, the solvent was evaporated under reduced pressure, and water was added. Then, 1M aqueous hydrochloric acid was added until bubbles no longer appeared. The precipitated crystals were collected and recrystallized (methanol) to give Co 77 (164mg).

EXAMPLE 55: Phosphorus oxychloride (10ml) was added to Rco 48 (1.07g), and the mixture was refluxed for 2.5 hours. The solvent was evaporated, and the reaction mixture was azeotropically concentrated with toluene. THF (15ml) was added to the obtained residue. After morpholine (10ml) was slowly added in dropwise under ice cooling, the ice bath was removed and the reaction mixture was refluxed for 30 minutes. Ethyl acetate and THF were added to the reaction mixture and the mixture was washed with water and brine. After it was dried over anhydrous sodium sulfate, the solvent was evaporated under reduced pressure and the obtained residue was purified with silica gel column chromatography (chloroform : methanol = 98 : 2) to give bis(morpholinoamido) 2-(4-morpholinopyrido[3',2':4,5]furo[3,2-d]pyrimidine-2-yl)phenylphosphonate (594mg). Formic acid (4ml) was added to this compound (360mg) and the mixture was stirred at 100°C for 3 days. The solvent was evaporated under reduced pressure, ethyl acetate and water were added, and the mixture was neutralized with saturated aqueous sodium hydrogencarbonate under ice cooling. The precipitated crystals were filtered to give crystals (162mg). The obtained crystals (123mg) were recrystallized (methanol-THF) to give Co 79 (122mg).

EXAMPLE 56: Dioxane (3.9ml) and 6M hydrochloric acid (5.5ml) were added to Co 80 (220mg), and the mixture was refluxed for 3 days. After the reaction mixture was allowed to cool, it was neutralized, extracted with a mixture solution of ethyl acetate and THF, and washed with brine. After it was dried over anhydrous sodium sulfate, the solvent was evaporated under reduced pressure, and the obtained residue was purified with silica gel column chromatography (chloroform : methanol = 96 : 4) to give crystals (83mg). The obtained crystals (81mg) were recrystallized (THF-methanol) to give Co 81 (53mg).

EXAMPLE 57: After a solution of a free form of Co 168 (151mg) in pyridine (9ml) was cooled in an ice bath, acetic anhydride (4.5ml) was added, and the mixture was stirred under ice cooling. After the reaction completed, the reaction mixture was poured into water with ice, extracted with ethyl acetate, and washed with brine. After it was dried over anhydrous sodium sulfate, the solvent was evaporated under reduced pressure to give a free form of Co 183 (158mg). The obtained free form (156mg) was subjected to salt formation as described in EXAMPLE 18, and the obtained crystals were recrystallized (methanol) to give hydrochloride of Co 169 (93mg).

EXAMPLE 58: 2-Morpholinoethanol (806mg) was added in dropwise to a solution of 60% sodium hydroxide (63mg) in DMF (5ml), and the mixture solution was stirred at room temperature for 15 minutes. Then, a free form of Co 179 (285mg) was added and the mixture was stirred at 60°C for 23 hours. Then, a mixture, which was prepared by adding 2-morpholinoethanol (806mg) in dropwise to 60% sodium hydroxide (63mg) in DMF (1ml) and stirring at room temperature for 15 minutes, was added in dropwise to the reaction mixture and the resulting mixture was stirred at 60°C. This addition of sodium 2-morpholinoethoxide was conducted 3 times. The solvent was evaporated under reduced pressure, water and THF were added to the obtained residue, and the mixture was extracted with ethyl acetate and then washed with brine. After it was dried over anhydrous sodium sulfate, the solvent was evaporated under reduced pressure, and the obtained residue was purified with silica gel column chromatography (chloroform : methanol = 95 : 5) to give a free form of Co 192 (529mg). The obtained free form (404mg) was subjected to salt formation as described in EXAMPLE 18, and the obtained crystals were recrystallized (methanol) to give dihydrochloride of Co 192 (320mg).

## Claims

1. A pharmaceutical composition for use as a phosphatidylinositol 3 kinase inhibitor comprising a fused heteroaryl derivative represented by general formula (I) or a salt thereof and a pharmaceutically acceptable carrier: 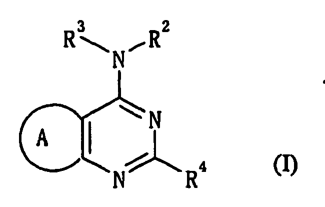 wherein: 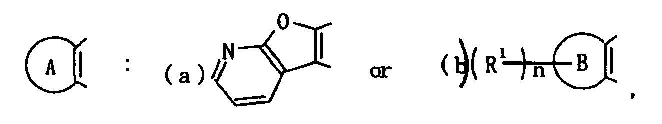 B represents a benzene ring, or pyridine, pyrazine or thiophene rings;
R¹ represents -C₁₋₆ alkyl, -alkenyl of up to 6 carbon atoms, -alkynyl of up to 6 carbon atoms, -C₃₋₈ cycloalkyl, -C₆₋₁₄ aryl which may have 1 to 5 substituents selected from Group A, -a heteroaryl which is a 5- or 6-membered monocyclic heteroaryl containing 1 to 4 hetero atoms selected from N, S and O or a bicyclic heteroaryl made of a monocyclic heteroaryl fused to a benzene ring, which may be partially saturated, and which may have 1 to 5 substituents selected from Group A, -a halogen, -NO₂, -CN, -a halogenated C₁₋₆ alkyl, -ORb, -SRb, -SO₂-Bb, -SO-Rb, -COORb, -CO-Rb, -CONRaRb, -SO₂NRaRb, -NRaRb, -NRa-CORb, -NRa-SO₂Rb, -O-CO-NRaRb or -NRaCO-COORb, -CO-a nitrogen-containing saturated heterocyclic group which is a 5- to 7-membered heterocyclic group containing one or two nitrogen atoms on the ring, and which may further contain one O or S atom and may form a bridge structure or may be fused with one benzene ring, -CONRa-C₁₋₆ alkylene-ORb, -CONRa-C₁₋₆ alkylene-NRbRc, -O-C₁₋₆ alkylene-ORb, -O-C₁₋₆ alkylene-O-C₁₋₆ alkylene-ORb, -O-C₁₋₆ alkylene-NRaRb, -O-C₁₋₆ alkylene-O-C₁₋₆ alkylene-NRaRb, -O-C₁₋₆ alkylene-NRc-C₁₋₆ alkylene-NRaRb, -NRc-C₁₋₆ alkylene- NRaRb, -N(C₁₋₆ alkylene-NRaRb)₂, -CONRa-ORb, -NRa-CO-NRbRc, or -OCORb;
Group A comprises of -C₁₋₆ alkyl, -alkenyl of up to 6 carbon atoms, -alkynyl of up to 6 carbon atoms, -a halogen, -a halogenated C₁₋₆ alkyl, -C₁₋₆ alkylene-OR, -NO₂, -CN, =O, -OR, -O-a halogenated C₁₋₆ alkyl, -O-C₁₋₆ alkylene-NRR', -O-C₁₋₆ alkylene-OR, -O-C₁₋₆ alkylene-C₆₋₁₄ aryl, -SR, -SO₂-C₁₋₆ alkyl, -SO-C₁₋₆ alkyl, -COOR, -COO-C₁₋₆ alkylene- C₆₋₁₄ aryl, -COR, -CO-C₆₋₁₄ aryl, -C₆₋₁₄ aryl, -CONRR', -SO₂NRR' , -NRR', -NR"-C₁₋₆ alkylene-NRR', -NR'-C₁₋₆ alkylene-OR, -NR-C₁₋₆ alkylene-C₆₋₁₄ aryl, -NRCO-C₁₋₆ alkyl, -NRSO₂-C₁₋₆ alkyl, -C₃₋₈ cycloalkyl and -C₃₋₈ cycloalkenyl;
R, R' and R", which may be the same or different, represent H or C₁₋₆ alkyl;
R² and R³ are combined together with the N atom adjacent thereto to form a nitrogen-containing saturated heterocyclic group as -NR²R³ selected from a group consisting of 1-pyrrolidinyl, 1-piperazinyl, piperidino and morpholino, and which may have 1 to 2 substituents selected from the group comprising of -OH, =O and -C₁₋₆ alkyl;
each of Ra and Rc, which may be the same or different, represents -H or -C₁₋₆ alkyl;
Rb represents -H, -C₁₋₆ alkyl, -C₃₋₈ cycloalkyl, -C₆₋₁₄ aryl which may have 1 to 5 substituents selected from Group A or -a heteroaryl which is a 5- or 6-membered monocyclic heteroaryl containing 1 to 4 hetero atoms selected from N, S and O or a bicyclic heteroaryl made of a monocyclic heteroaryl fused to a benzene ring, which may be partially saturated, and which may have 1 to 5 substituents selected from Group A;
n represents 0, 1, 2 or 3, whereas n represents 1, 2 or 3 when B represents a benzene ring;
R⁴ represents - C₆₋₁₄ aryl which may have 1 to 5 substituents selected from Group A⁴, -C₁₋₆ alkylene-C₆₋₁₄ aryl which may have 1 to 5 substituents selected from Group A⁴, -alkenylene of up to 6 carbon atoms -C₆₋₁₄ aryl which may have 1 to 5 substituents selected from Group A⁴, -alkynylene of up to 6 carbon atoms -C₆₋₁₄ aryl which may have 1 to 5 substituents selected from Group A⁴, -C₃₋₈ cycloalkyl which may have 1 to 5 substituents selected from Group A, -C₃₋₈ cycloalkenyl which may have 1 to 5 substituents selected from Group A, -C₁₋₆ alkylene-C₃₋₈ cycloalkyl which may have 1 to 5 substituents selected from Group A, -alkenylene of up to 6 carbon atoms C₃₋₈ cycloalkyl which may have 1 to 5 substituents selected from Group A, -C₁₋₆ alkylene-a nitrogen-containing saturated heterocyclic group which is a 5-to 7-membered heterocyclic group containing one or two nitrogen atoms on the ring, and which may further contain one O or S atom and may form a bridge structure or may be fused with one benzene ring, and which may have 1 to 5 substituents selected from Group A, -alkenylene of up to 6 carbon atoms -a nitrogen-containing saturated heterocyclic group which is a 5- to 7-membered heterocyclic group containing one or two nitrogen atoms on the ring, and which may further contain one O or S atom and may form a bridge structure or may be fused with one benzene ring, and which may have 1 to 5 substituents selected from Group A, -a heteroaryl which is a 5- or 6-membered monocyclic heteroaryl containing 1 to 4 hetero atoms selected from N, S and O or a bicyclic heteroaryl made of a monocyclic heteroaryl fused to a benzene ring, which may be partially saturated, and which may have 1 to 5 substituents selected from Group A⁴, -C₁₋₆ alkylene-a heteroaryl which is a 5- or 6-membered monocyclic heteroaryl containing 1 to 4 hetero toms selected from N, S and O or a bicyclic heteroaryl made of a monocyclic heteroaryl fused to a benzene ring, which may be partially saturated, and which may have 1 to 5 substituents selected from Group A⁴, or -alkenylene of up to 6 carbon atoms -a heteroaryl which is a 5- or 6-membered monocyclic heteroaryl containing 1 to 4 hetero atoms selected from N, S and O or a bicyclic heteroaryl made of a monocyclic heteroaryl fused to a benzene ring, which may be partially saturated, and which may have 1 to 5 substituents selected from Group A⁴;
Group A⁴ comprises of a) : -C₁₋₆ alkyl, -alkenyl of up to 6 carbon atoms, -alkynyl of up to 6 carbon atoms, -a halogen, -a halogenated C₁₋₆ alkyl, -C₁₋₆ alkylene-OR, -NO₂, -CN, =O, -O-halogenated C₁₋₆ alkyl, -SO₂-C₁₋₆ alkyl, -SO-C₁₋₆ alkyl, -COOR, -COO-C₁₋₆ alkylene-C₆₋₁₄ aryl, -COR, -CO-C₆₋₁₄ aryl, -CONRR', -SO₂NRR', -Cyc or -Alp-Cyc, wherein Alp represents C₁₋₆ alkylene, alkenylene of up to 6 carbon atoms or alkynylene of up to 6 carbon atoms, and Cyc represents C₆₋₁₄ aryl which may have 1 to 5 substituents selected from Group A; a heteroaryl which is a 5-or 6-membered monocyclic heteroaryl containing 1 to 4 hetero atoms selected from N, S and O or a bicyclic heteroaryl made of a monocyclic heteroaryl fused to a benzene ring, which may be partially saturated, and which may have 1 to 5 substituents selected from Group A; a nitrogen-containing saturated heterocyclic group which is a 5- to 7-membered heterocyclic group containing one or two nitrogen atoms on the ring, and which may further contain one O or S atom and may form a bridge structure or may be fused with one benzene ring, and which may have 1 to 5 substituents selected from Group A; C₃₋₈ cycloalkyl which may have 1 to 5 substituents selected from Group A; or C₃₋₈ cycloalkenyl which may have 1 to 5 substituents selected from Group A,
b) : -NR-E-F, wherein E represents -CO-, -COO-, -CONR' -, -SO₂NR'-or -SO₂-; F represents -Cyc or -C₁₋₆ alkyl, alkenyl of up to 6 carbon atoms or alkynyl of up to 6 carbon atoms, which may be substituted by one or more substituents selected from the group comprising of -a halogen, -NO₂, -CH, -OR, -O-C₁₋₆ alkylene-NRR', -O-C₁₋₆ alkylene-OR, -SR, -SO₂-C₁₋₆ alkyl, -SO-C₁₋₆ alkyl, -COOR, -COR, -CO-C₆₋₁₄ aryl, -CONRR', -SO₂NRR', -NRCO-C₁₋₆ alkyl, -NRR', -NR'-C₁₋₆ alkylene-OR, -NR"-C₁₋₆ alkylene-NRR', and -Cyc; and
c) : -Z-R', -Z-Cyc, -Z-Alp-Cyc, -Z-Alp-Z' -R' or -Z-Alp-Z' -Cyc, wherein each of Z and Z', which may be the same or different, independently represents O, S or NR;
with the proviso that when 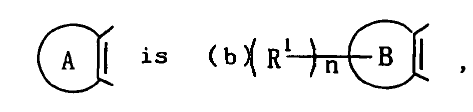 R² and R³ forms -NR²R³ which is -morpholino, and R⁴ represents -C₆₋₁₄ aryl which may have 1 to 5 substituents selected from Group A⁴ or -a heteroaryl which is a 5- or 6-membered monocyclic heteroaryl containing 1 to 4 hetero atoms selected from N, S and O or a bicyclic heteroaryl made of a monocyclic heteroaryl fused to a benzene ring, which may be partially saturated, and which may have 1 to 5 substituents selected from Group A⁴, and that the following compounds are excluded:
(1) 4-(4-morpholinyl)-2-phenylpyrido[2,3-d]pyrimidine,
(2) 4-(4-morpholinyl)-2-phenylpyrido[2,3-d]pyrimidin-7(1H)-one,
(3) 4-(4-morpholinyl)-2-pheny-6-quinazolinol and 6-methoxy-4-(4-morpholinyl)-2-phenyquinazoline,
(4) compounds in which B represents a benzene ring, n is 2 or 3, existing R¹'s all represent -OMe, and R⁴ is an unsubstituted phenyl or a phenyl which is substituted by 1 to 3 substituents selected from -halogen, -NO₂, -C₁₋₆ alkyl, -O-C₁₋₆ alkyl, -a hanogenated C₁₋₆ alkyl and -CONRaRc,
(5) compounds in which B represents a benzene ring, n is 1, R¹ represents -halogen or -a C₁₋₆ alkyl, and R⁴ represents - imidazolyl which may have one or more substituents,
(6) compounds in which B represents a pyridine ring, and R⁴, represents an unsubstituted phenyl or an unsubstituted pyridyl,
(7) compounds in which B represents a pyrazine ring, and R⁴ represents an unsubstituted phenyl, and
(8) 4-(4-morpholinyl)-2-phenylthieno[2,3-d]pyrimidine.

2. The pharmaceutical composition according to one of claims 1, wherein the pharmaceutical composition is an antitumor agent.

3. Use of the fused heteroaryl derivative represented by formula (I) of claim 1 or a salt thereof for the manufacture of medicament for use in the treatment of cancer.

4. A fused heteroaryl derivative represented by general formula (Ia) or a salt thereof: 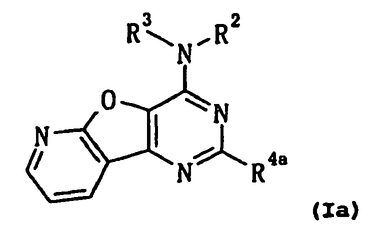 wherein:
; R² and R³ are combined together with the N atom adjacent thereto to form a nitrogen-containing saturated heterocyclic group as -NR²R³ selected from a group consisting of 1-pyrrolidinyl, 1-piperazinyl, piperidino and morpholino, and which may have 1 to 2 substituents selected from the group comprising of -OH, =O and -C₁₋₆ alkyl;
R^{4a} represents - C₆₋₁₄ aryl which may have 1 to 5 substituents selected from Group A⁴, -C₁₋₆ alkylene- C₆₋₁₄ aryl which may have 1 to 5 substituents selected from Group A⁴, -alkenylene of up to 6 carbon atoms -C₆₋₁₄ aryl which may have 1 to 5 substituents selected from Group A⁴, -alkynylene of up to 6 carbon atoms - C₆₋₁₄ aryl which may have 1 to 5 substituents selected from Group A⁴, -C₃₋₈ cycloalkyl which may have 1 to 5 substituents selected from Group A, -C₃₋₈ cycloalkenyl which may have 1 to 5 substituents selected from Group A, -C₁₋₆ alkylene-C₃₋₈ cycloalkyl which may have 1 to 5 substituents selected from Group A, -alkenylene of up to 6 carbon atoms -C₃₋₈ cycloalkyl which may have 1 to 5 substituents selected from Group A, -C₁₋₆ alkylene-a nitrogen-containing saturated heterocyclic group which is a 5-to 7-membered heterocyclic group containing one or two nitrogen atoms on the ring, and which may further contain one **O** or S atom and may form a bridge structure or may be fused with one benzene ring, and which may have 1 to 5 substituents selected from Group A, -alkenylene of up to 6 carbon atoms -a nitrogen-containing saturated heterocyclic group which is a 5- to 7-membered heterocyclic group containing one or two nitrogen atoms on the ring, and which may further contain one **O** or S atom and may form a bridge structure or may be fused with one benzene ring, and which may have 1 to 5 substituents selected from Group A, -a heteroaryl which is a 5- or 6-membered monocyclic heteroaryl containing 1 to 4 hetero atoms selected from N, S and **O** or a bicyclic heteroaryl made of a monocyclic heteroaryl fused to a benzene ring, which may be partially saturated, and which may have 1 to 5 substituents selected from Group A⁴, -C₁₋₆ alkylene-a heteroaryl which is a 5- or 6-membered monocyclic heteroaryl containing 1 to 4 hetero atoms selected from N, S and **O** or a bicyclic heteroaryl fused said monocyclic heteroaryl to a benzene ring, which may be partially saturated, and which may have 1 to 5 substituents selected from Group A⁴, or -alkenylene of up to 6 carbon atoms -a heteroaryl which is a 5- or 6-membered monocyclic heteroaryl containing 1 to 4 hetero atoms selected from N, S and **O** or a bicyclic heteroaryl made of a monocyclic heteroaryl fused to a benzene ring, which may be partially saturated, and which may have 1 to 5 substituents selected from Group A⁴;
Group A comprises of -C₁₋₆ alkyl, -alkenyl of up to 6 carbon atoms, -alkynyl of up to 6 carbon atoms, -a halogen, -a halogenated C₁₋₆ alkyl, -C₁₋₆ alkylene-OR, -NO₂, -CN, =O, -OR, -O-a halogenated C₁₋₆ alkyl, -O-C₁₋₆ alkylene-NRR' , -O-C₁₋₆ alkylene-OR, -O-C₁₋₆ alkylene- C₆₋₁₄ aryl, -SR, -SO₂-C₁₋₆ alkyl, -SO-C₁₋₆ alkyl, -COOR, -COO-C₁₋₆ alkylene- C₆₋₁₄ aryl, -COR, -CO- C₆₋₁₄ aryl, - C₆₋₁₄ aryl, -CONRR' , -SO₂NRR' , -NRR' , -NR' ' -C₁₋₆ alkylene-NRR' , -NR' -C₁₋₆ alkylene-OR, -NR-C₁₋₆ alkylene- C₆₋₁₄ aryl, -NRCO-C₁₋₆ alkyl, -NRSO₂-C₁₋₆ alkyl, -C₃₋₈ cycloalkyl and -C₃₋₈ cycloalkenyl;
R, R' and R'' , which may be the same or different, represent H or C₁₋₆ alkyl ; and,
Group A⁴ comprises of a) : -C₁₋₆ alkyl, -alkenyl of up to 6 carbon atoms, -alkynyl of up to 6 carbon atoms, -a halogen, -a halogenated C₁₋₆ alkyl, -C₁₋₆ alkylene-OR, -NO₂, -CN, =O, -O-halogenated C₁₋₆ alkyl, -SO₂-C₁₋₆ alkyl, -SO-C₁₋₆ alkyl, -COOR, -COO-C₁₋₆ alkylene- C₆₋₁₄ aryl, -COR, -CO-C₆₋₁₄ aryl, -CONRR' , -SO₂NRR' , -Cyc or -Alp-Cyc, wherein Alp represents C₁₋₆ alkylene, alkenylene of up to 6 carbon atoms or alkynylene of up to 6 carbon atoms, and Cyc represents C₆₋₁₄ aryl which may have 1 to 5 substituents selected from Group A, a heteroaryl which is a 5-or 6-membered monocyclic heteroaryl containing 1 to 4 hetero atoms selected from N, S and O or a bicyclic heteroaryl made of a monocyclic heteroaryl fused to a benzene ring, which may be partially saturated, and which may have 1 to 5 substituents selected from Group A, a nitrogen-containing saturated heterocyclic group which is a 5- to 7-membered heterocyclic group containing one or two nitrogen atoms on the ring, and which may further contain one **O** or S atom and may form a bridge structure or may be fused with one benzene ring, and which may have 1 to 5 substituents selected from Group A, C₃₋₈ cycloalkyl which may have 1 to 5 substituents selected from Group A, or C₃₋₈ cycloalkenyl which may have 1 to 5 substituents selected from Group A,
b): -NR-E-F, wherein E represents -CO-, -COO-, -CONR' -, -SO₂NR' - or -SO₂-; F represents -Cyc or -C₁₋₆ alkyl, alkenyl of up to 6 carbon atoms or alkynyl of up to 6 carbon atoms, which may be substituted by one or more substituents selected from the group comprising of -a halogen, -NO₂, -CN, -OR, -O-C₁₋₆ alkylene-NRR' , -O-C₁₋₆ alkylene-OR, -SR, -SO₂-C₁₋₆ alkyl, -SO-C₁₋₆alkyl, -COOR, -COR, -CQ- C₆₋₁₄ aryl , -CONRR' , -SO₂NRR' , -NRCO-C₁₋₆ alkyl, -NRR', -NR'-C₁₋₆ alkylone-OR,- -NR"-C₁₋₆ alkylene-NRR' and -Cyc; and
c): -Z-R', -Z-Cyc, -Z-Alp-Cyc, -Z-Alp-Z'-R' or -Z-Alp-Z'-Cyc, wherein each of Z and Z', which may be the same or different, independently represents O, S or NR.

5. The fused heteroaryl derivative or a salt thereof according to claim 4, wherein R² and R³ forms -HR²R³ which is -morpholino.

6. The fused heteroaryl derivative or a salt thereof according to claim 4, wherein R^{4a} represents - C₆₋₁₄ aryl which may have 1 to 5 substituents selected from Group A⁴ or -a heteroaryl which is a 5- or 6-membered monocyclic heteroaryl containing 1 to 4 hetero atoms selected from N, S and O or a bicyclic heteroaryl made of a monocyclic heteroaryl fused to a benzene ring, which may be partially saturated, and which may have 1 to 5 substituents selected from Group A⁴.

7. The fused heteroaryl derivative or a salt thereof according to claim 4, wherein the fused heteroaryl derivative or a salt thereof is selected from the group comprising of
6-amino-3'-(4-morpholinopyrido[3',2':4,5 furo[3,2-d]pyrimidi n-2-yl)nicotinanilide,
4-(4-morpholinopyrido[3',2':4,5]furo[3,2-d]pyrimidin-2-yl)an iline,
3-(4-morpholinopyrido[3',2':4,5]furo[3,2-d]pyrimidin-2-yl)ph enol,
4-morpholino-2-[3-(2-piperazin-1-ylethoxy)phenyl]pyrido[3',2 ':4,5]furo[3,2-d]pyrimidine,
3'-(4-morpholinopyrido[3',2':4,5]furo[3,2-d]pyrimidin-2-yl)a crylanilide, and salts thereof.

8. A fused heteroaryl derivative represented by general formula (Ib) or a salt thereof: 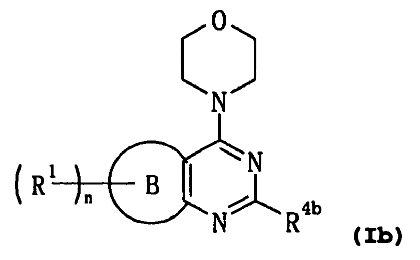 wherein:
B represents a benzene ring, or pyridine, pyrazine or thiophene rings;
R¹ represents -C₁₋₆ alkyl , -alkenyl of up to 6 carbon atoms, -alkynyl of up to 6 carbon atoms, -C₃₋₈ cycloalkyl, - C₆₁₄ aryl which may have 1 to 5 substituents selected from Group A, -a heteroaryl which is a 5- or 6-membered monocyclic heteroaryl containing 1 to 4 hetero atoms selected from N, S and **O** or a bicyclic heteroaryl made of a monocyclic heteroaryl made of a money clic heteroryl fused to a benzene ring, which may be partially saturated, and which may have 1 to 5 substituents selected from Group A, -a halogen, -NO₂, -CN, -a halogenated C₁₋₆ alkyl, -ORb, -SRb, -SO₂-Rb, -SO-Rb, -COORb, -CO-Rb, -CONRaRb, -SO₂NRaRb, -NRaRb, -NRa-CORb, -NRa-SO₂Rb, -O-CO-NRaRb, -NRaCO-COORb, -NRaCOORb, -NRaCO-C₁₋₆ alkylene- C₆₋₁₄ aryl, -NRa-SO₂-C₁₋₆ alkylene- C₆₋₁₄ aryl, -NRa-C₁₋₆ alkylene- C₆₋₁₄ aryl, -C₁₋₆ alkylene-ORb, -C₁₋₆ alkylene-NRaRb, -CO-a nitrogen-containing saturated heterocyclic group which is a 5-to 7-membered heterocyclic group containing one or two nitrogen atoms on the ring, and which may further contain one **O** or S atom and may form a bridge structure or may be fused with one benzene ring, -CONRa-C₁₋₆ alkylene-ORb, -CONRa-C₁₋₆ alkylene-NRcRb, -CONRa-C₁₋₆ alkylene-a nitrogen-containing saturated heterocyclic group which is a 5- to 7-membered heterocyclic group containing one or two nitrogen atoms on the ring, and which may further contain one O or S atom and may form a bridge structure or may be fused with one benzene ring, -O-C₁₋₆ alkyleae-ORb, -O-C₁₋₆ alkylene-NRaRb, -O-C₁₋₆ alkylene-a nitrogen-containing saturated heterocyclic group which is a 5- to 7-membered heterocyclic group containing one or two nitrogen atoms on the ring, and which may further contain one O or S atom and may form a bridge structure or may be fused with one benzene ring, -O-C₁₋₆ alkylene-O-C₁₋₆ alkylene-OBb, -O-C₁₋₆ alkylene-O-C₁₋₆ alkylene-NRaRb, -O-C₁₋₆ alkylene-NRc-C₁₋₆ alkylene-NRaRb, -NRc-C₁₋₆alkylene-NRaRb, -N(C₁₋₆ alkylene-NRaRb)_{2,} -CONRa-ORb, -NRa-CO-NRbRc, or -OCORb;
Group A comprises of -C₁₋₆ alkyl, -alkenyl of up to 6 carbon atoms, -alkynyl of up to 6 carbon atoms, -a halogen, -a halogenated C₁₋₆ alkyl, -C₁₋₆ alkylene-OR, -NO₂, -CN, =O, -OR, -O-a halogenated C₁₋₆ alkyl, -O-C₁₋₆ alkylene-NRR' , -O-C₁₋₆ alkylene-OR, -O-C₁₋₆ alkylene-C₆₋₁₄ aryl, -SR, -SO₂-C₁₋₆ alkyl, -SO-C₁₋₆ alkyl, -COOR, -COO-C₁₋₆ alkylene- C₆₋₁₄ aryl, -COR, -CO- C₆₋₁₄ aryl, - C₆₋₁₄ n aryl, -CONRR' , -SO₂NRR' , -NRR' , -NR' ' -C₁₋₆ alkylene-NRR' , -NR' -C₁₋₆ alkylone-OR, -NR-C₁₋₆ alkylene- C₆₋₁₄ aryl, -NRCO-C₁₋₆ alkyl, -NRSO₂-C₁₋₆ alkyl, -C₃₋₈ cycloalkyl and -C₃₋₈ cycloalkenyl;
R, R' and R'' , which may be the same or different, represent H or C₁₋₆ alkyl; ;
Ra and Rc, which may be the same or different, represent -H or -C₁₋₆ alkyl;
Rb represents -H, -C₁₋₆ alkyl, -C₃₋₈ cycloalkyl , - C₆₋₁₄ aryl which may have 1 to 5 substituents selected from Group A or -a heteroaryl which is a 5- or 6-membered monocyclic heteroaryl containing 1 to 4 hetero atoms made of a money clic heteroryl fused selected from N, S and **O** or a bicyclic heteroaryl atoms made of a monocyclic heteroaryl fused to a benzene ring, which may be partially saturated, and which may have 1 to 5 substituents selected from Group A;
n represents 0,1, 2 or 3, whereas n represents 1, 2 or 3 when B represents a benzene ring;
R^{4b} represents -C₆₋₁₄ aryl which may have 1 to 5 substituents selected from Group A⁴ or -a heteroaryl which is a 5- or 6-membered monocyclic heteroaryl containing 1 to 4 hetero atoms selected from N, S and **O** or a bicyclic heteroaryl made of a monocyclic heteroaryl fused to a benzene ring, which may be partially saturated, and which may have 1 to 5 substituents selected from Group A⁴ and,
Group A⁴ comprises of a) : -C₁₋₆ alkyl, -alkenyl of up to 6 carbon atoms, -alkynyl of up to 6 carbon atoms, -a halogen, -a halogenated C₁₋₆ alkyl, -C₁₋₆ alkylene-OR, -NO₂, -CN, =O, -O-halogenated C₁₋₆ alkyl, -SO₂-C₁₋₆ alkyl, -SO-C₁₋₆ alkyl, -COOR, -COO-C₁₋₆ alkylene- C₆₋₁₄ aryl, -COR, -CO- C₆₋₁₄ aryl, -CONRR' , -SO₂NRR' , -Cyc or -Alp-Cyc, wherein Alp represents C₁₋₆ alkylene, alkenylene of up to 6 carbon atoms or alkynylene of up to 6 carbon atoms, and Cyc represents C₆₋₁₄ aryl which may have 1 to 5 substituents selected from Group A; a heteroaryl which is a 5-or 6-membered monocyclic heteroaryl containing 1 to 4 hetero atoms selected from N, S and **O** or a bicyclic heteroaryl made of a monocyclic heteroaryl fused to a benzene ring, which may be partially saturated, and which may have 1 to 5 substituents selected from Group A; a nitrogen-containing saturated heterocyclic group which is a 5- to 7-membered heterocyclic group containing one or two nitrogen atoms on the ring, and which may further contain one **O** or S atom and may form a bridge structure or may be fused with one benzene ring, and which may have 1 to 5 substituents selected from Group A; C₃₋₈ cycloalkyl which may have 1 to 5 substituents selected from Group A; or C₃₋₈ cycloalkenyl which may have 1 to 5 substituents selected from Group A,
b) : -NR-E-F, wherein E represents -CO-, -COO-, -CONR' -, -SO₂NR' - or -SO₂-; F represents -Cyc or -C₁₋₆ alkyl, alkenyl of up to 6 carbon atoms or alkynyl of up to 6 carbon atoms, which may be substituted by one or more substituents selected from the group comprising of -a halogen, -NO₂, -CN, -OR, -O-C₁₋₆ alkylene-NRR' , -O-C₁₋₆ alkylene-OR, -SR, -SO₂-C₁₋₆ alkyl, -SO-C₁₋₆ alkyl, -COOR, -COR, -CO-C₆₋₁₄ aryl , -CONRR' , -SO₂NRR' , -NRCO-C₁₋₆ alkyl, -NRR' , -NR'-C₁₋₆ alkylene-OR, -NR"-C₁₋₆ alkylene-NRR' and -Cyc, and
c) : -Z-R' , -Z-Cyc, -Z-Alp-Cyc, -Z-Alp-Z' -R' or -Z-Alp-Z' -Cyc, wherein each of Z and Z', which may be the same or different, independently represents O, S or NR;
with the proviso that the following compounds are excluded:
(1) 4-(4-morpholinyl)-2-phenylpyrido[2,3-d]pyrimidine,
(2)
4-(4-morpholinyl)-2-phenylpyrido[2,3-d]pyrimidin-7(1H)-one,
(3) 4-(4-morpholinyl)-2-pheny-6-quinazolinol and 6-methoxy-4-(4-morpholinyl)-2-phenyquinazoline,
(4) compounds in which B represents a benzene ring, n is 2 or 3, existing R¹' s all represent -OMe, and R^{4b} is an unsubstituted phenyl or a phenyl which is substituted by 1 to 3 substituents selected from -halogen, -NO₂, -C₁₋₆ alkyl, -O-C₁₋₆ alkyl, -a hanogenated C₁₋₆ alkyl and -CONRaRc,
(5) compounds in which B represents a benzene ring, n is 1, R¹ represents -halogen or -a lower alkyl, and R^{4b} represents -(imidazolyl which may have one or more substituents),
(6) compounds in which B represents a pyridine ring, and R^{4b} represents an unsubstituted phenyl or an unsubstituted pyridyl,
(7) compounds in which B represents a pyrazine ring, and R^{4b} represents an unsubstituted phenyl, and
(8) 4-(4-morpholinyl)-2-phenylthieno[2,3-d]pyrimidine.

9. The fused heteroaryl derivative or a salt thereof according to claim 8, wherein R^{4b} represents C₆₋₁₄ aryl which has one or more substituents selected from the group comprising of -C₁₋₆ alkylene-OR, -CONRR' , -NR-CO-Cyc¹, -NR-SO₂-Cyc¹, -OR, -NRR', -O-C₁₋₆ alkylene-NRR' and -O-C₁₋₆ alkylene-a nitrogen-containing saturated heterocyclic group which is a 5- to 7-membered heterocyclic group containing one or two nitrogen atoms on the ring, and which may further contain one **O** or S atom and may form a bridge structure or may be fused with one benzene ring, and which may have 1 to 5 substituents selected from Group A; wherein Cyc¹ is - C₆₋₁₄ aryl which may have 1 to 5 substituents selected from Group A; -a heteroaryl which is a 5- or 6-membered monocyclic heteroaryl containing 1 to 4 hetero atoms selected from N, S and O or a bicyclic heteroaryl made of a monocyclic heteroaryl fused to a benzene ring, which may be partially saturated, and which may have 1 to 5 substituents selected from Group A; or -a nitrogen-containing saturated heterocyclic group which is a 5-to 7-membered heterocyclic group containing one or two nitrogen atoms on the ring, and which may further contain one **O** or S atom and may form a bridge structure or may be fused with one benzene ring, and which may have 1 to 5 substituents selected from Group A.

10. The fused heteroaryl derivative or a salt thereof according to claim 8, wherein B represents a benzene ring, n is 1 or 2, and R¹ represents -NO₂, -CN, -a halogenated C₁₋₆ alkyl, -ORb, -SRb, -NRaRb, -NRa-CORb or -NRa-SO₂Rb.

11. The fused heteroaryl derivative or a salt thereof according to claim 8, wherein B represents a pyridine, pyrazine or thiophene ring, n is 0, and R^{4b} represents a phenyl which has at least one substituent which is selected from -OH, -CH₂OH and -CONH₂.

12. The fused heteroaryl derivative or a salt thereof according to claim 8, wherein B represents a pyridine ring.

13. The fused heteroaryl derivative or a salt thereof according to claim 8, wherein B represents pyrazine ring.

14. The fused heteroaryl derivative or a salt thereof according to claim 8, wherein B represents thiophene ring.

15. The fused heteroaryl derivative or a salt thereof according to claim 8, wherein the fused heteroaryl derivative or a salt thereof is selected from the group comprising of
N-[2-(3-benzenesulfonylaminophenyl)-4-morphoniloquinazolin-6 -yl]acetamide,
3-(4-morpholinopyrido[4,3-d]pyrimidin-2-yl)phenol,
3-(4-morpholinopyrido[3,2-d]pyrimidin-2-yl)phenol,
3-(4-morpholinopyrido[3,4-d]pyrimidin-2-yl)phenol,
3-(6-methoxy-4-morpholinoquinazolin-2-yl)phenol,
3-(4-morpholinothieao[3,2-d]pyrimidin-2-yl)phenol,
3-(4-morpholinopteridin-2-yl)phenol, and salts thereof.

16. A pharmaceutical composition comprising a fused heteroaryl derivative or a salt thereof according to claim 4, and a pharmaceutically acceptable carrier.

17. A pharmaceutical composition comprising a fused heteroaryl derivative or a salt thereof according to claim 8, and a pharmaceutically acceptable carrier.

## Patentansprüche

1. Pharmazeutische Zusammensetzung zur Verwendung als Phosphatidylinositol-3-Kinase-Inhibitor, die ein kondensiertes Heteroarylderivat, repräsentiert durch die allgemeine Formel (I), oder ein Salz davon und einen pharmazeutisch akzeptablen Träger umfasst: 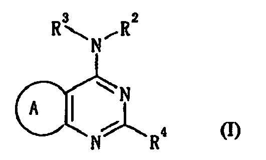 wobei: 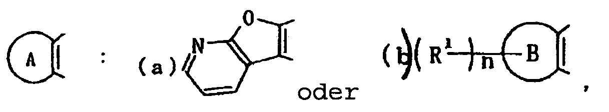 B einen Benzolring oder Pyridin-, Pyrazin- oder Thiophenringe repräsentiert;
R¹ Folgendes repräsentiert: -C₁₋₆ Alkyl, -Alkenyl mit bis zu 6 Kohlenstoffatomen, -Alkynyl mit bis zu 6 Kohlenstoffatomen, -C₃₋₈ Cycloalkyl, -C₆₋₁₄ Aryl, das 1 bis 5 Substituenten haben kann, die ausgewählt sind aus der Gruppe A, -a Heteroaryl, das ein 5- oder 6-gliedriges monozyklisches Heteroaryl mit 1 bis 4 Heteroatomen, ausgewählt aus N, S und O, oder ein bizyklisches Heteroaryl ist, das aus einem monozyklischen Heteroaryl besteht, das an einen Benzolring kondensiert ist, das teilweise gesättigt sein kann und das 1 bis 5 Substituenten haben kann, die ausgewählt sind aus der Gruppe A, -a Halogen, -NO₂, -CN, -a halogeniertes C₁₋₆ Alkyl, -ORb, -SRb, -SO₂-Rb, -SO-Rb, -COORb, -CO-Rb, -CONRaRb, -SO₂NRaRb, -NRaRb, -NRa-CORb, -NRa-SO₂Rb, -O-CO-NRaRb oder -NRaCO-COORb, -CO-a stickstoffhaltige, gesättigte heterozyklische Gruppe, die eine 5- bis 7-gliedrige heterozyklische Gruppe ist, die ein oder zwei Stickstoffatome auf dem Ring enthält und die ferner ein O- oder S-Atom enthalten und eine Brückenstruktur bilden kann oder mit einem Benzolring kondensiert sein kann, -CONRa-C₁₋₆ Alkylen-ORb, -CONRa-C₁₋₆ Alkylen-NRbRc, -O-C₁₋₆ Alkylen-ORb, -O-C₁₋₆ Alkylen-O-C₁₋₆ alkylen-ORb, -O-C₁₋₆ Alkylen-NRaRb, -O-C₁₋₆ Alkylen-O-C₁₋₆ alkylen-NRaRb, -O-C₁₋₆ Alkylen-NRc-C₁₋₆ alkylen-NRaRb, -NRc-C₁₋₆ Alkylen-NRaRb, -N(C₁₋₆ Alkylen-NRaRb)₂, -CONRa-ORb, -NRa-CO-NRbRc oder -OCORb;
Gruppe A Folgendes umfasst: -C₁₋₆ Alkyl, -Alkenyl mit bis zu 6 Kohlenstoffatomen, -Alkynyl mit bis zu 6 Kohlenstoffatomen, -a Halogen, -a halogeniertes C₁₋₆ Alkyl, - C₁₋₆ Alkylen-OR, -NO₂, -CN, =O, -OR, -O-a halogeniertes C₁₋₆ Alkyl, -O-C₁₋₆ Alkylen-NRR', -O-C₁₋₆ Alkylen-OR, -O-C₁₋₆ Alkylen-C₆₋₁₄ Aryl, -SR, -SO₂-C₁₋₆-Alkyl, -SO-C₁₋₆ Alkyl, -COOR, -COO-C₁₋₆ Alkylen-C₆₋₁₄ Aryl, -COR, -CO-C₆₋₁₄ Aryl, -C₆₋₁₄Aryl, -CONRR' , -SO₂NRR' , -NRR' , -NR" -C₁₋₆ Alkylen-NRR', -NR'-C₁₋₆ Alkylen-OR, -NR-C₁₋₆ Alkylen-C₆₋₁₄ Aryl, -NRCO-C₁₋₆ Alkyl, -NRSO₂-C₁₋₆ Alkyl, -C₃₋₈ Cycloalkyl und -C₃₋₈ Cycloalkenyl;
R, R' und R ", die gleich oder unterschiedlich sein können, H oder C₁₋₆ Alkyl repräsentieren;
R² und R³ mit dem N-Atom daneben kombiniert sind, um eine stickstoffhaltige, gesättigte heterozyklische Gruppe wie -NR²R³ zu bilden, ausgewählt aus der Gruppe bestehend aus 1-Pyrrolidinyl, 1-Piperazinyl, Piperidino und Morpholino, die 1 bis 2 Substituenten haben kann, die ausgewählt sind aus der Gruppe bestehend aus -OH, =O und -C₁₋₆ Alkyl;
Ra und Rc, die gleich oder unterschiedlich sein können, jeweils -H oder -C₁₋₆ Alkyl repräsentieren;
Rb Folgendes repräsentiert: -H, -C₁₋₆ Alkyl, -C₃₋₈ Cycloalkyl, -C₆₋₁₄ Aryl, das 1 bis 5 Substituenten haben kann, die ausgewählt sind aus der Gruppe A, oder -a Heteroaryl, das ein 5- oder 6-gliedriges monozyklisches Heteroaryl mit 1 bis 4 Heteroatomen, ausgewählt aus N, S und O, oder ein bizyklisches Heteroaryl ist, das aus einem monozyklischen Heteroaryl besteht, das an einen Benzolring kondensiert ist, das teilweise gesättigt sein kann und das 1 bis 5 Substituenten haben kann, die ausgewählt sind aus der Gruppe A;
n 0, 1, 2 oder 3 repräsentiert, wobei n 1, 2, oder 3 repräsentiert, wenn B einen Benzolring repräsentiert;
R⁴ Folgendes repräsentiert: -C₆₋₁₄ Aryl, das 1 bis 5 Substituenten haben kann, die ausgewählt sind aus der Gruppe A⁴, -C₁₋₆ Alkylen-C₆₋₁₄ Aryl, das 1 bis 5 Substituenten haben kann, die ausgewählt sind aus der Gruppe A⁴, -Alkenylen mit bis zu 6 Kohlenstoffatomen -C₆₋₁₄ Aryl, das 1 bis 5 Substituenten haben kann, die ausgewählt sind aus der Gruppe A⁴, -Alkynylen mit bis zu 6 Kohlenstoffatomen -C₆₋₁₄ Aryl, das 1 bis 5 Substituenten haben kann, die ausgewählt sind aus der Gruppe A⁴, -C₃₋₈ Cycloalkyl, das 1 bis 5 Substituenten haben kann, die ausgewählt sind aus der Gruppe A, -C₃₋₈ Cycloalkenyl, das 1 bis 5 Substituenten haben kann, die ausgewählt sind aus der Gruppe A, -C₁₋₆ Alkylen-C₃₋₈ Cycloalkyl, das 1 bis 5 Substituenten haben kann, die ausgewählt sind aus der Gruppe A, -Alkenylen mit bis zu 6 Kohlenstoffatomen -C₃₋₈ Cycloalkyl, das 1 bis 5 Substituenten haben kann, die ausgewählt sind aus der Gruppe A, -C₁₋₆ Alkylen-a stickstoffhaltige, gesättigte heterozyklische Gruppe, die eine 5- bis 7-gliedrige heterozyklische Gruppe ist, die ein oder zwei Stickstoffatome auf dem Ring enthält und die ferner ein 0-oder S-Atom enthalten und eine Brückenstruktur bilden kann oder mit einem Benzolring kondensiert sein kann und die 1 bis 5 Substituenten haben kann, die ausgewählt sind aus der Gruppe A, -Alkenylen mit bis zu 6 Kohlenstoffatomen -a stickstoffhaltige, gesättigte heterozyklische Gruppe, die eine 5- bis 7-gliedrige heterozyklische Gruppe ist, die ein oder zwei Stickstoffatome auf dem Ring enthält und die ferner ein O- oder S-Atom enthalten und eine Brückenstruktur bilden kann oder mit einem Benzolring kondensiert sein kann und die 1 bis 5 Substituenten haben kann, die ausgewählt sind aus der Gruppe A, -a Heteroaryl, das ein 5- oder 6-gliedriges monozyklisches Heteroaryl mit 1 bis 4 Heteroatomen, ausgewählt aus N, S und O, oder ein bizyklisches Heteroaryl ist, das aus einem monozyklischen Heteroaryl besteht, das an einen Benzolring kondensiert ist, das teilweise gesättigt sein kann und das 1 bis 5 Substituenten haben kann, die ausgewählt sind aus der Gruppe A⁴, -C₁₋₆-Alkylen-a Heteroaryl, das ein 5- oder 6-gliedriges monozyklisches Heteroaryl mit 1 bis 4 Heteroatomen, ausgewählt aus N, S und O, oder ein bizyklisches Heteroaryl ist, das aus einem monozyklischen Heteroaryl besteht, das an einen Benzolring kondensiert ist, das teilweise gesättigt sein kann und das 1 bis 5 Substituenten haben kann, die ausgewählt sind aus der Gruppe A⁴, oder -Alkenylen mit bis zu 6 Kohlenstoffatomen -a Heteroaryl, das ein 5- oder 6-gliedriges monozyklisches Heteroaryl mit 1 bis 4 Heteroatomen, ausgewählt aus N, S und O, oder ein bizyklisches Heteroaryl ist, das aus einem monozyklischen Heteroaryl besteht, das an einen Benzolring kondensiert ist, das teilweise gesättigt sein kann und das 1 bis 5 Substituenten haben kann, die ausgewählt sind aus der Gruppe A⁴ ;
Gruppe A⁴ Folgendes umfasst: a) -C₁₋₆ Alkyl, -Alkenyl mit bis zu 6 Kohlenstofatomen, -Alkynyl mit bis zu 6 Kohlenstoffatomen, -a Halogen, -a halogeniertes C₁₋₆ Alkyl, -C₁₋₆ Alkylen-OR, -NO₂, -CN, =O, -O-halogeniertes C₁₋₆ Alkyl, -SO₂-C₁₋₆ Alkyl, -SO-C₁₋₆ Alkyl, -COOR, -COO-C₁₋₆ Alkylen-C₆₋₁₄ Aryl, -COR, -CO-C₆₋₁₄-Aryl, -CONRR', -SO₂NRR', -Cyc oder -Alp-Cyc, wobei Alp C₁₋₆ Alkylen, Alkenylen mit bis zu 6 Kohlenstoffatomen oder Alkynylen mit bis zu 6-Kohlenstoffatomen repräsentiert und Cyc C₆₋₁₄ Aryl repräsentiert, das 1 bis 5 Substituenten haben kann, die ausgewählt sind aus der Gruppe A; ein Heteroaryl, das ein 5- oder 6-gliedriges monozyklisches Heteroaryl mit 1 bis 4 Heteroatomen, ausgewählt aus N, S und O, oder ein bizyklisches Heteroaryl ist, das aus einem monozyklischen Heteroaryl besteht, das an einen Benzolring kondensiert ist, das teilweise gesättigt sein kann und das 1 bis 5 Substituenten haben kann, die ausgewählt sind aus der Gruppe A; eine stickstoffhaltige, gesättigte heterozyklische Gruppe, die eine 5- bis 7-gliedrige heterozyklische Gruppe ist, die ein oder zwei Stickstoffatome auf dem Ring enthält und die ferner ein 0-oder S-Atom enthalten und eine Brückenstruktur bilden kann oder mit einem Benzolring kondensiert sein kann und die 1 bis 5 Substituenten haben kann, die ausgewählt sind aus der Gruppe A; C₃₋₈ Cycloalkyl, das 1 bis 5 Substituenten haben kann, die ausgewählt sind aus der Gruppe A; oder C₃₋₈ Cycloalkenyl, das 1 bis 5 Substituenten haben kann, die ausgewählt sind aus der Gruppe A,
b) -NR-E-F, wobei E -CO-, -COO-, -CONR'-, -SO₂NR'- oder -SO₂- repräsentiert; F -Cyc oder -C₁₋₆ Alkyl, Alkenyl mit bis zu 6 Kohlenstoffatomen oder Alkynyl mit bis zu 6 Kohlenstoffatomen repräsentiert, das substituiert sein kann durch einen oder mehrere Substituenten, die ausgewählt sind aus der Gruppe bestehend aus -a Halogen, -NO₂, -CN, -OR, -0-C₁₋₆ Alkylen-NRR' , -O-C₁₋₆ Alkylen-OR, -SR, -SO₂ -C₁₋₆ Alkyl, -SO- C₁₋₆ Alkyl, -COOR, -COR, -CO-C₆₋₁₄ Aryl, -CONRR', -SO₂NRR', -NRCO-C₁₋₆ Alkyl, -NRR' , -NR'-C₁₋₆ Alkylen-OR, -NR"-C₁₋₆ Alkylen-NRR' und -Cyc; und
c) -Z-R', -Z-Cyc, -Z-Alp-Cyc, -Z-Alp-Z'-R' oder -Z-Alp-Z'-Cyc, wobei Z und Z', die gleich oder unterschiedlich sein können, jeweils unabhängig O, S oder NR repräsentieren;
unter der Voraussetzung, dass, wenn 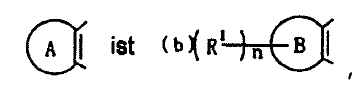 R² und R³ -NR²R³ bilden, das -Morpholino ist, und R⁴ -C₆₋₁₄-Aryl repräsentiert, das 1 bis 5 Substituenten haben kann, die ausgewählt sind aus der Gruppe A⁴, oder -a Heteroaryl, das ein 5- oder 6-gliedriges monozyklisches Heteroaryl mit 1 bis 4 Heteroatomen, ausgewählt aus N, S und O, oder ein bizyklisches Heteroaryl ist, das aus einem monozyklischen Heteroaryl besteht, das an einen Benzolring kondensiert ist, das teilweise gesättigt sein kann und das 1 bis 5 Substituenten haben kann, die ausgewählt sind aus der Gruppe A⁴, und dass die folgenden Verbindungen ausgeschlossen sind:
(1) 4-(4-Morpholinyl)-2-phenylpyrido[2, 3-d]pyrimidin,
(2) 4-(4-Morpholinyl)-2-phenylpyrido[2,3-d]pyrimidin-7(1H)-on,
(3) 4-(4-Morpholinyl)-2-phenyl-6-chinazolinol und 6-Methoxy-4-(4-morpholinyl)-2-phenylchinazolin,
(4) Verbindungen, bei denen B einen Benzolring repräsentiert, n 2 oder 3 ist, alle vorhandenen R¹ -OMe repräsentieren und R⁴ ein unsubstituiertes Phenyl oder ein Phenyl ist, das substituiert ist durch 1 bis 3 Substituenten, die ausgewählt sind aus -Halogen, -NO₂, -C₁₋₆ Alykl, -O-C₁₋₆ Alkyl, -a halogeniertem -C₁₋₆ Alkyl und -CONRaRc,
(5) Verbindungen, bei denen B einen Benzolring repräsentiert, n 1 ist, R¹ -Halogen oder -a C₁₋₆ Alkyl repräsentiert und R⁴ Imidazolyl repräsentiert, das einen oder mehrere Substituenten haben kann,
(6) Verbindungen, bei denen B einen Pyridinring repräsentiert und R⁴ ein unsubstituiertes Phenyl oder ein unsubstituiertes Pyridyl repräsentiert,
(7) Verbindungen, bei denen B einen Pyrazinring repräsentiert und R⁴ ein unsubstituiertes Phenyl repräsentiert, und
(8) 4-(4-Morpholinyl)-2-phenylthieno[2,3-d]pyrimidin.

2. Pharmazeutische Zusammensetzung nach Anspruch 1, wobei die pharmazeutische Zusammensetzung ein Antitumormittel ist.

3. Verwendung des kondensierten Heteroarylderivats, das durch die Formel (I) aus Anspruch 1 repräsentiert ist, oder eines Salzes davon zur Herstellung eines Medikaments zur Verwendung bei der Behandlung von Krebs.

4. Kondensiertes Heteroarylderivat, das durch die allgemeine Formel (Ia) repräsentiert ist, oder ein Salz davon: 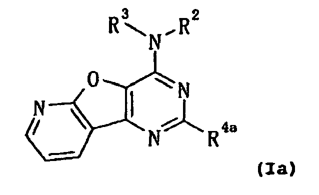 wobei:
R² und R³ mit dem N-Atom daneben kombiniert sind, um eine stickstoffhaltige, gesättigte heterozyklische Gruppe wie -NR²R³ zu bilden, ausgewählt aus der Gruppe bestehend aus 1-Pyrrolidinyl, 1-Piperazinyl, Piperidino und Morpholino, die 1 bis 2 Substituenten haben kann, die ausgewählt sind aus der Gruppe bestehend aus -OH, =O und -C₁₋₆ Alkyl;
R^{4a} Folgendes repräsentiert: -C₆₋₁₄ Aryl, das 1 bis 5 Substituenten haben kann, die ausgewählt sind aus der Gruppe A⁴, -C₁₋₆ Alkylen-C₆₋₁₄ Aryl, das 1 bis 5 Substituenten haben kann, die ausgewählt sind aus der Gruppe A⁴, -Alkenylen mit bis zu 6 Kohlenstoffatomen -C₆₋₁₄ Aryl, das 1 bis 5 Substituenten haben kann, die ausgewählt sind aus der Gruppe A⁴, -Alkynylen mit bis zu 6 Kohlenstoffatomen -C₆₋₁₄ Aryl, das 1 bis 5 Substituenten haben kann, die ausgewählt sind aus der Gruppe A⁴, -C₃₋₈ Cycloalkyl, das 1 bis 5 Substituenten haben kann, die ausgewählt sind aus der Gruppe A, -C₃₋₈ Cycloalkenyl, das 1 bis 5 Substituenten haben kann, die ausgewählt sind aus der Gruppe A, -C₁₋₆ Alkylen-C₃₋₈ Cycloalkyl, das 1 bis 5 Substituenten haben kann, die ausgewählt sind aus der Gruppe A, -Alkenylen mit bis zu 6 Kohlenstoffatomen -C₃₋₈ Cycloalkyl, das 1 bis 5 Substituenten haben kann, die ausgewählt sind aus der Gruppe A, -C₁₋₆ Alkylen-a stickstoffhaltige, gesättigte heterozyklische Gruppe, die eine 5- bis 7-gliedrige heterozyklische Gruppe ist, die ein oder zwei Stickstoffatome auf dem Ring enthält und die ferner ein 0-oder S-Atom enthalten und eine Brückenstruktur bilden kann oder mit einem Benzolring kondensiert sein kann und die 1 bis 5 Substituenten haben kann, die ausgewählt sind aus der Gruppe A, -Alkenylen mit bis zu 6 Kohlenstoffatomen -a stickstoffhaltige, gesättigte heterozyklische Gruppe, die eine 5- bis 7-gliedrige heterozyklische Gruppe ist, die ein oder zwei Stickstoffatome auf dem Ring enthält und die ferner ein O- oder S-Atom enthalten und eine Brückenstruktur bilden kann oder mit einem Benzolring kondensiert sein kann und die 1 bis 5 Substituenten haben kann, die ausgewählt sind aus der Gruppe A, -a Heteroaryl, das ein 5- oder 6-gliedriges monozyklisches Heteroaryl mit 1 bis 4 Heteroatomen, ausgewählt aus N, S und O, oder ein bizyklisches Heteroaryl ist, das aus einem monozyklischen Heteroaryl besteht, das an einen Benzolring kondensiert ist, das teilweise gesättigt sein kann und das 1 bis 5 Substituenten haben kann, die ausgewählt sind aus der Gruppe A⁴, -C₁₋₆ Alkylen-a Heteroaryl, das ein 5- oder 6-gliedriges monozyklisches Heteroaryl mit 1 bis 4 Heteroatomen ist, ausgewählt aus N, S und O, oder ein bizyklisches Heteroaryl kondensiert genanntes monozyklisches Heteroaryl an einen Benzolring [sic], das teilweise gesättigt sein kann und das 1 bis 5 Substituenten haben kann, die ausgewählt sind aus der Gruppe A⁴, oder -Alkenylen mit bis zu 6 Kohlenstoffatomen -a Heteroaryl, das ein 5- oder 6-gliedriges monozyklisches Heteroaryl mit 1 bis 4 Heteroatomen, ausgewählt aus N, S und O, oder ein bizyklisches Heteroaryl ist, das aus einem monozyklischen Heteroaryl besteht, das an einen Benzolring kondensiert ist, das teilweise gesättigt sein kann und das 1 bis 5 Substituenten haben kann, die ausgewählt sind aus der Gruppe A⁴ ;
Gruppe A Folgendes umfasst: -C₁₋₆ Alkyl, -Alkenyl mit bis zu 6 Kohlenstoffatomen, -Alkynyl mit bis zu 6 Kohlenstoffatomen, -a Halogen, -a halogeniertes C₁₋₆ Alkyl, -C₁₋₆ Alkylen-OR, -NO₂, -CN, =O, -OR, -O-a halogeniertes C₁₋₆ Alkyl, -O-C₁₋₆ Alkylen-NRR' , -O-C₁₋₆ Alkylen-OR, -O-C₁₋₆ Alkylen-C₆₋₁₄ Aryl, -SR, -SO₂-C₁₋₆ Alkyl, -SO-C₁₋₆ Alkyl, -COOR, -COO-C₁₋₆ Alkylen-C₆₋₁₄ Aryl, -COR, -CO-C₆₋₁₄ Aryl, -C₆₋₁₄ Aryl, -CONRR', -SO₂NRR', -NRR', -NR"-C₁₋₆ Alkylen-NRR', -NR'-C₁₋₆ Alkylen-OR, -NR-C₁₋₆ Alkylen-C₆₋₁₄ Aryl, -NRCO-C₁₋₆ Alkyl, -NRSO₂-C₁₋₆ Alkyl, -C₃₋₈ Cycloalkyl und -C₃₋₈ Cycloalkenyl;
R, R' und R ", die gleich oder unterschiedlich sein können, H oder C₁₋₆-Alkyl repräsentieren; und
Gruppe A⁴ Folgendes umfasst: a) -C₁₋₆ Alkyl, -Alkenyl mit bis zu 6 Kohlenstofatomen, -Alkynyl mit bis zu 6 Kohlenstoffatomen, -a Halogen, -a halogeniertes C₁₋₆ Alkyl, -C₁₋₆ Alkylen-OR, -NO₂, -CN, =O, -O-halogeniertes C₁₋₆ Alkyl, -SO₂-C₁₋₆ Alkyl, -SO-C₁₋₆ Alkyl, -COOR, -COO-C₁₋₆ Alkylen-C₆₋₁₄ Aryl, -COR, -CO-C₆₋₁₄ Aryl, -CONRR', -SO₂NRR', -Cyc oder -Alp-Cyc, wobei Alp C₁₋₆ Alkylen, Alkenylen mit bis zu 6 Kohlenstoffatomen oder Alkynylen mit bis zu 6-Kohlenstoffatomen repräsentiert und Cyc C₆₋₁₄ Aryl repräsentiert, das 1 bis 5 Substituenten haben kann, die ausgewählt sind aus der Gruppe A, ein Heteroaryl, das ein 5- oder 6-gliedriges monozyklisches Heteroaryl mit 1 bis 4 Heteroatomen, ausgewählt aus N, S und O, oder ein bizyklisches Heteroaryl ist, das aus einem monozyklischen Heteroaryl besteht, das an einen Benzolring kondensiert ist, das teilweise gesättigt sein kann und das 1 bis 5 Substituenten haben kann, die ausgewählt sind aus der Gruppe A, eine stickstoffhaltige, gesättigte heterozyklische Gruppe, die eine 5- bis 7-gliedrige heterozyklische Gruppe ist, die ein oder zwei Stickstoffatome auf dem Ring enthält und die ferner ein 0-oder S-Atom enthalten und eine Brückenstruktur bilden kann oder mit einem Benzolring kondensiert sein kann und die 1 bis 5 Substituenten haben kann, die ausgewählt sind aus der Gruppe A, C₃₋₈ Cycloalkyl, das 1 bis 5 Substituenten haben kann, die ausgewählt sind aus der Gruppe A, oder C₃₋₈ Cycloalkenyl, das 1 bis 5 Substituenten haben kann, die ausgewählt sind aus der Gruppe A,
b) -NR-E-F, wobei E -CO-, -COO-, -CONR'-, -SO₂NR'- oder -SO₂- repräsentiert; F -Cyc oder -C₁₋₆ Alkyl, Alkenyl mit bis zu 6 Kohlenstoffatomen oder Alkynyl mit bis zu 6 Kohlenstoffatomen repräsentiert, das substituiert sein kann durch einen oder mehrere Substituenten, die ausgewählt sind aus der Gruppe bestehend aus -a Halogen, -NO₂, -CN, -OR, -0-C₁₋₆ Alkylen-NRR', -O-C₁₋₆ Alkylen-OR, -SR, -SO₂-C₁₋₆ Alkyl, -SO-C₁₋₆ Alkyl, -COOR, -COR, -CO-C₆₋₁₄ Aryl, -CONRR', -SO₂NRR' , -NRCO-C₁₋₆ Alkyl, -NRR' , -NR' -C₁₋₆ Alkylen-OR, -NR"-C₁₋₆ Alkylen-NRR' und -Cyc; und
c) -Z-R', -Z-Cyc, -Z-Alp-Cyc, -Z-Alp-Z'-R' oder -Z-Alp-Z'-Cyc, wobei Z und Z', die gleich oder unterschiedlich sein können, jeweils unabhängig O, S oder NR repräsentieren.

5. Kondensiertes Heteroarylderivat oder ein Salz davon nach Anspruch 4, wobei R² und R³ -NR²R³ bilden, das -Morpholino ist.

6. Kondensiertes Heteroarylderivat oder ein Salz davon nach Anspruch 4, wobei R^{4a} -C₆₋₁₄ Aryl repräsentiert, das 1 bis 5 Substituenten haben kann, die ausgewählt sind aus der Gruppe A⁴, oder -a Heteroaryl, das ein 5- oder 6-gliedriges monozyklisches Heteroaryl mit 1 bis 4 Heteroatomen, ausgewählt aus N, S und O, oder ein bizyklisches Heteroaryl ist, das aus einem monozyklischen Heteroaryl besteht, das an einen Benzolring kondensiert ist, das teilweise gesättigt sein kann und das 1 bis 5 Substituenten haben kann, die ausgewählt sind aus der Gruppe A⁴.

7. Kondensiertes Heteroarylderivat oder ein Salz davon nach Anspruch 4, wobei das kondensierte Heteroarylderivat oder ein Salz davon ausgewählt ist aus der Gruppe bestehend aus
6-Amino-3'-(4-morpholinopyrido[3',2':4,5]furo(3,2-d]pyrimidin-2-yl)nicotinanilid,
4- (4-Morpholinopyrido [3', 2' :4, 5] furo [3, 2-d] pyrimidin-2-yl)anilin,
3- (4-Morpholinopyrido [3', 2' :4, 5] furo [3, 2-d] pyrimidin-2-yl)phenol,
4-Morpholino-2-[3-(2-piperazin-1-ylethoxy) phenyl] pyrido [3', 2' :4, 5] furo [3, 2-d] pyrimidin,
3'- (4-Morpholinopyrido [3', 2' :4, 5] furo [3, 2-d] pyrimidin-2-yl)acrylanilid und Salzen davon.

8. Kondensiertes Heteroarylderivat, das durch die allgemeine Formel (Ib) repräsentiert ist, oder ein Salz davon: 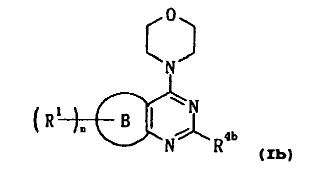 wobei:
B einen Benzolring oder Pyridin-, Pyrazin- oder Thiophenringe repräsentiert;
R¹ Folgendes repräsentiert: -C₁₋₆ Alkyl, -Alkenyl mit bis zu 6 Kohlenstoffatomen, -Alkynyl mit bis zu 6 Kohlenstoffatomen, -C₃₋₈ Cycloalkyl, -C₆₋₁₄ Aryl, das 1 bis 5 Substituenten haben kann, die ausgewählt sind aus der Gruppe A, -a Heteroaryl, das ein 5- oder 6-gliedriges monozyklisches Heteraryl mit 1 bis 4 Heteroatomen, ausgewählt aus N, S und O, oder ein bizyklisches Heteroaryl ist, das aus einem monozyklischen Heteroaryl besteht, das an einen Benzolring kondensiert ist, das teilweise gesättigt sein kann und das 1 bis 5 Substituenten haben kann, die ausgewählt sind aus der Gruppe A, -a Halogen, -NO₂, -CN, -a halogeniertes C₁₋₆ Alkyl, -ORb, -SRb, -SO₂-Rb, -SO-Rb, -COORb, -CO-Rb, -CONRaRb, -SO₂NRaRb, -NRaRb, -NRa-CORb, -NRa-SO₂Rb, -O-CO-NRaRb, -NRaCO-COORb, -NRaCOORb, -NRaCO-C₁₋₆ Alkylen-C₆₋₁₄ Aryl, -NRa-SO₂-C₁₋₆ Alkylen-C₆₋₁₄ Aryl, -NRa-C₁₋₆ Alkylen-C₆₋₁₄ Aryl, -C₁₋₆ Alkylen-ORb, -C₁₋₆ Alkylen-NRaRb, -CO-a stickstoffhaltige, gesättigte heterozyklische Gruppe, die eine 5- bis 7-gliedrige heterozyklische Gruppe ist, die ein oder zwei Stickstoffatome auf dem Ring enthält und die ferner ein 0-oder S-Atom enthalten und eine Brückenstruktur bilden kann oder mit einem Benzolring kondensiert sein kann, -CONRa-C₁₋₆ Alkylen-ORb, -CONRa-C₁₋₆ Alkylen-NRcRb, -CONRa-C₁₋₆ Alkylen-a stickstoffhaltige, gesättigte heterozyklische Gruppe, die eine 5- bis 7-gliedrige heterozyklische Gruppe ist, die ein oder zwei Stickstoffatome auf dem Ring enthält und die ferner ein O- oder S-Atom enthalten und eine Brückenstruktur bilden kann oder mit einem Benzolring kondensiert sein kann, -O-C₁₋₆ Alkylen-ORb, -O-C₁₋₆ Alkylen-N RaRb, O-C₁₋₆-Alkylen-a stickstoffhaltige, gesättigte heterozyklische Gruppe, die eine 5- bis 7-gliedrige heterozyklische Gruppe ist, die ein oder zwei Stickstoffatome auf dem Ring enthält und die ferner ein 0-oder S-Atom enthalten und eine Brückenstruktur bilden kann oder mit einem Benzolring kondensiert sein kann, -O-C₁₋₆ Alkylen-O-C₁₋₆ Alkylen-ORb, -O-C₁₋₆ Alkylen-O-C₁₋₆ Alkylen-NRaRb, -O-C₁₋₆ Alkylen-NRc-C₁₋₆ Alkylen-NRaRb, -NRc-C₁₋₆ Alkylen-NRaRb, -N(C₁₋₆-Alkylen-NRaRb)₂, -CONRa-ORb, -NRa-CO-NRbRc oder -OCORb;
Gruppe A Folgendes umfasst: -C₁₋₆ Alkyl, -Alkenyl mit bis zu 6 Kohlenstoffatomen, -Alkynyl mit bis zu 6 Kohlenstoffatomen, -a Halogen, -a halogeniertes C₁₋₆ Alkyl, -C₁₋₆ Alkylen-OR, -NO₂, -CN, =O, -OR, -O-a halogeniertes C₁₋₆ Alkyl, -O-C₁₋₆ Alkylen-NRR', -O-C₁₋₆ Alkylen-OR, -O-C₁₋₆ Alkylen-C₆₋₁₄ Aryl, -SR, -SO₂-C₁₋₆ Alkyl, -SO-C₁₋₆Alkyl, -COOR, -COO-C₁₋₆ Alkylen-C₆₋₁₄ Aryl, -COR, -CO-C₆₋₁₄ Aryl, -C₆₋₁₄ n Aryl, -CONRR' , -SO₂NRR', -NRR' , -NR" -C₁₋₆ Alkylen-NRR', -NR'-C₁₋₆ Alkylen-OR, -NR-C₁₋₆ Alkylen-C₆₋₁₄ Aryl, -NRCO-C₁₋₆ Alkyl, -NRSO₂-C₁₋₆ Alkyl, -C₃₋₈ Cycloalkyl und -C₃₋₈ Cycloalkenyl;
R, R' und R", die gleich oder unterschiedlich sein können, H oder C₁₋₆-Alkyl repräsentieren;
Ra und Rc, die gleich oder unterschiedlich sein können, -H oder -C₁₋₆ Alkyl repräsentieren;
Rb Folgendes repräsentiert: -H, -C₁₋₆ Alkyl, -C₃₋₈-Cycloalkyl, -C₆₋₁₄ Aryl, das 1 bis 5 Substituenten haben kann, die ausgewählt sind aus der Gruppe A, oder ein -a Heteroaryl, das ein 5- oder 6-gliedriges monozyklisches Heteroaryl mit 1 bis 4 Heteroatomen, ausgewählt aus N, S und O, oder ein bizyklisches Heteroaryl ist, das aus einem monozyklischen Heteroaryl besteht, das an einen Benzolring kondensiert ist, das teilweise gesättigt sein kann und das 1 bis 5 Substituenten haben kann, die ausgewählt sind aus der Gruppe A;
n 0, 1, 2 oder 3 repräsentiert, wobei n 1, 2, oder 3 repräsentiert, wenn B einen Benzolring repräsentiert;
R^{4b} Folgendes repräsentiert: -C₆₋₁₄ Aryl, das 1 bis 5 Substituenten haben kann, die ausgewählt sind aus der Gruppe A⁴, oder -a Heteroaryl, das ein 5- oder 6-gliedriges monozyklisches Heteroaryl mit 1 bis 4 Heteroatomen, ausgewählt aus N, S und O, oder ein bizyklisches Heteroaryl ist, das aus einem monozyklischen Heteroaryl besteht, das an einen Benzolring kondensiert ist, das teilweise gesättigt sein kann und das 1 bis 5 Substituenten haben kann, die ausgewählt sind aus der Gruppe A⁴, und
Gruppe A⁴ Folgendes umfasst: a) -C₁₋₆ Alkyl, -Alkenyl mit bis zu 6 Kohlenstoffatomen, -Alkynyl mit bis zu 6 Kohlenstoffatomen, -a Halogen, -a halogeniertes C₁₋₆ Alkyl, -C₁₋₆ Alkylen-OR, -NO₂, -CN, =O, -O-halogeniertes C₁₋₆ Alkyl, -SO₂C₁₋₆ Alkyl, -SO-C₁₋₆ Alkyl, -COOR, -COO-C₁₋₆ Alkylen-C₆₋₁₄ Aryl, -COR, -CO-C₆₋₁₄ Aryl, -CONRR', -SO₂NRR', -Cyc oder -Alp-Cyc, wobei Alp C₁₋₆ Alkylen, Alkenylen mit bis zu 6 Kohlenstoffatomen oder Alkynylen mit bis zu 6 Kohlenstoffatomen repräsentiert und Cyc C₆₋₁₄ Aryl repräsentiert, das 1 bis 5 Substituenten haben kann, die ausgewählt sind aus der Gruppe A; ein Heteroaryl, das ein 5- oder 6-gliedriges monozyklisches Heteroaryl mit 1 bis 4 Heteroatomen, ausgewählt aus N, S und O, oder ein bizyklisches Heteroaryl ist, das aus einem monozyklischen Heteroaryl besteht, das an einen Benzolring kondensiert ist, das teilweise gesättigt sein kann und das 1 bis 5 Substituenten haben kann, die ausgewählt sind aus der Gruppe A; eine stickstoffhaltige, gesättigte heterozyklische Gruppe, die eine 5- bis 7-gliedrige heterozyklische Gruppe ist, die ein oder zwei Stickstoffatome auf dem Ring enthält und die ferner ein 0-oder S-Atom enthalten und eine Brückenstruktur bilden kann oder mit einem Benzolring kondensiert sein kann und die 1 bis 5 Substituenten haben kann, die ausgewählt sind aus der Gruppe A; C₃₋₈ Cycloalkyl, das 1 bis 5 Substituenten haben kann, die ausgewählt sind aus der Gruppe A; oder C₃₋₈ Cycloalkenyl, das 1 bis 5 Substituenten haben kann, die ausgewählt sind aus der Gruppe A,
b) -NR-E-F, wobei E -CO-, -COO-, -CONR'-, -SO₂NR'- oder -SO₂- repräsentiert; F -Cyc oder -C₁₋₆ Alkyl, Alkenyl mit bis zu 6 Kohlenstoffatomen oder Alkynyl mit bis zu 6 Kohlenstoffatomen repräsentiert, das substituiert sein kann durch einen oder mehrere Substituenten, die ausgewählt sind aus der Gruppe bestehend aus -a Halogen, -NO₂, -CN, -OR, -O-C₁₋₆ Alkylen-NRR', -O-C₁₋₆ Alkylen-OR, -SR, -SO₂-C₁₋₆ Alkyl, -SO-C₁₋₆ Alkyl, -COOR, -COR, -CO-C₆₋₁₄ Aryl, -CONRR', -SO₂NRR' , -NRCO-C₁₋₆ Alkyl, -NRR' , -NR' -C₁₋₆ Alkylen-OR, -NR"-C₁₋₆-Alkylen NRR' und -Cyc; und
c) -Z-R', -Z-Cyc, -Z-Alp-Cyc, -Z-Alp-Z'-R' oder -Z-Alp-Z'-Cyc, wobei Z und Z', die gleich oder unterschiedlich sein können, jeweils unabhängig O, S oder NR repräsentieren;
unter der Voraussetzung, dass die folgenden Verbindungen ausgeschlossen sind:
(1) 4-(4-Morpholinyl)-2-phenylpyrido[2,3-d]pyrimidin,
(2) 4-(4-Morpholinyl)-2-phenylpyrido[2,3-d]pyrimidin-7 (1H) -on,
(3) 4-(4-Morpholinyl)-2-phenyl-6-chinazolinol und 6-Methoxy-4-(4-morpholinyl)-2-phenylchinazolin,
(4) Verbindungen, bei denen B einen Benzolring repräsentiert, n 2 oder 3 ist, alle vorhandenen R¹ -OMe repräsentieren und R^{4b} ein nichtsubstituiertes Phenyl oder ein Phenyl ist, das substituiert ist durch 1 bis 3 Substituenten, die ausgewählt sind aus -Halogen, -NO₂, -C₁₋₆ Alykl, -O-C₁₋₆ Alkyl, a- halogeniertem -C₁₋₆ Alkyl und -CONRaRc,
(5) Verbindungen, bei denen B einen Benzolring repräsentiert, n 1 ist, R¹ -Halogen oder -a niederes Alkyl repräsentiert und R^{4b} -Imidazolyl repräsentiert, das einen oder mehrere Substituenten haben kann,
(6) Verbindungen, bei denen B einen Pyridinring repräsentiert und R^{4b} ein unsubstituiertes Phenyl oder ein unsubstituiertes Pyridyl repräsentiert,
(7) Verbindungen, bei denen B einen Pyrazinring repräsentiert und R^{4b} ein unsubstituiertes Phenyl repräsentiert, und
(8) 4-(4-Morpholinyl)-2-phenylthieno[2,3-d]pyrimidin.

9. Kondensiertes Heteroarylderivat oder ein Salz davon nach Anspruch 8, wobei R^{4b} Folgendes repräsentiert: C₆₋₁₄ Aryl, das einen oder mehrere Substituenten hat, die ausgewählt sind aus der Gruppe bestehend aus -C₁₋₆ Alkylen-OR, -CONRR', -NR-CO-Cyc¹, -NR-SO₂-Cyc¹, -OR, -NRR', -O-C₁₋₆ Alkylen-NRR' und -O-C₁₋₆-Alkylen-a stickstoffhaltige, gesättigte heterozyklische Gruppe, die eine 5- bis 7-gliedrige heterozyklische Gruppe ist, die ein oder zwei Stickstoffatome auf dem Ring enthält und die ferner ein 0-oder S-Atom enthalten und eine Brückenstruktur bilden kann oder mit einem Benzolring kondensiert sein kann und die 1 bis 5 Substituenten haben kann, die ausgewählt sind aus der Gruppe A; wobei Cyc¹ -C₆₋₁₄ Aryl ist, das 1 bis 5 Substituenten haben kann, die ausgewählt sind aus der Gruppe A; -a Heteroaryl, das ein 5- oder 6-gliedriges monozyklisches Heteroaryl mit 1 bis 4 Heteroatomen, ausgewählt aus N, S und O, oder ein bizyklisches Heteroaryl ist, das aus einem monozyklischen Heteroaryl besteht, das an einen Benzolring kondensiert ist, das teilweise gesättigt sein kann und das 1 bis 5 Substituenten haben kann, die ausgewählt sind aus der Gruppe A; oder -a stickstoffhaltige gesättigte heterozyklische Gruppe, die eine 5- bis 7-gliedrige heterozyklische Gruppe ist, die ein oder zwei Stickstoffatome auf dem Ring enthält und die ferner ein O- oder S-Atom enthalten und eine Brückenstruktur bilden kann oder mit einen Benzolring kondensiert sein kann und die 1 bis 5 Substituenten haben kann, die ausgewählt sind aus der Gruppe A.

10. Kondensiertes Heteroarylderivat oder ein Salz davon nach Anspruch 8, wobei B einen Benzolring repräsentiert, n 1 oder 2 ist und R¹ -NO₂, -CN, -a halogeniertes -C₁₋₆ Alkyl, -ORb, -SRb, -NRaRb, -NRa-CORb oder -NRa-SO₂Rb repräsentiert.

11. Kondensiertes Heteroarylderivat oder ein Salz davon nach Anspruch 8, wobei B einen Pyridin-, Pyrazin- oder Thiophenring repräsentiert, n 0 ist und R^{4b} ein Phenyl repräsentiert, das wenigstens einen Substituenten hat, der ausgewählt ist aus -OH, -CH₂OH und -CONH₂.

12. Kondensiertes Heteroarylderivat oder ein Salz davon nach Anspruch 8, wobei B einen Pyridinring repräsentiert.

13. Kondensiertes Heteroarylderivat oder ein Salz davon nach Anspruch 8, wobei B einen Pyrazinring repräsentiert.

14. Kondensiertes Heteroarylderivat oder ein Salz davon nach Anspruch 8, wobei B einen Thiophenring repräsentiert.

15. Kondensiertes Heteroarylderivat oder ein Salz davon nach Anspruch 8, wobei das kondensierte Heteroarylderivat oder ein Salz davon ausgewählt ist aus der Gruppe bestehend aus
N-[2-(3-Benzolsulfonylaminophenyl)-4-morpholinochinazolin-6-yl]acetamid,
3-(4-Morpholinopyrido[4,3-d]pyrimidin-2-yl)phenol,
3-(4-Morpholinopyrido[3,2-d]pyrimidin-2-yl)phenol,
3-(4-Morpholinopyrido[3,4-d]pyrimidin-2-yl)phenol,
3-(6-Methoxy-4-morpholinochinazolin-2-yl)-phenol,
3- (4-Morpholinothieno [3, 2-d] pyrimidin-2-yl) phenol,
3-(4-Morpholinopteridin-2-yl)phenol und Salzen davon.

16. Pharmazeutische Zusammensetzung, die ein kondensiertes Heteroarylderivat oder ein Salz davon nach Anspruch 4 und einen pharmazeutisch akzeptablen Träger umfasst.

17. Pharmazeutische Zusammensetzung, die ein kondensiertes Heteroarylderivat oder ein Salz davon nach Anspruch 8 und einen pharmazeutisch akzeptablen Träger umfasst.

## Revendications

1. Composition pharmaceutique à utiliser en qualité d'inhibiteur de la phosphatidylinositol 3 kinase, comprenant un dérivé hétéroaryle fusionné représenté par la formule générale (I) ou un sel de celui-ci et un excipient pharmaceutiquement acceptable : 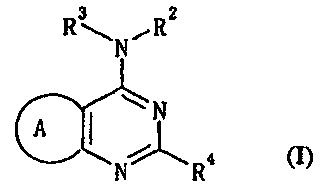 où 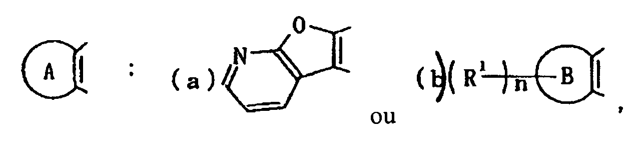 B représente un cycle benzénique ou des cycles pyridine, pyrazine ou thiophène;
R¹ représente -alkyle C₁₋₆, -alcényle de jusqu'à 6 atomes de carbone, -alcynyle de jusqu'à 6 atomes de carbone, -cycloalkyle C₃₋₈, -aryle C₆₋₁₄ qui peut avoir 1 à 5 substituants sélectionnés parmi le Groupe A, un hétéroaryle qui est un hétéroaryle monocyclique à 5 ou 6 chaînons contenant 1 à 4 hétéroatomes sélectionnés parmi N, S et O ou un hétéroaryle bicyclique constitué d'un hétéroaryle monocyclique fusionné à un cycle benzénique, qui peut être partiellement saturé et qui peut avoir 1 à 5 substituants sélectionnés parmi le Groupe A, - un halogène, -NO₂, -CN, - un alkyle C₁₋₆ halogéné, -ORb, -SRb, -SO₂-Rb, -SO-Rb, -COORb, -CO-Rb, -CONRaRb, -SO₂NRaRb, -NRaRb, -NRa-CORb, -NRa-SO₂Rb, -O-CO-NRaRb ou -NRaCO-COORb, -CO- un groupe hétérocyclique saturé contenant de l'azote qui est un groupe hétérocyclique de 5 à 7 chaînons contenant un ou deux atomes d'azote sur le cycle et qui peut contenir en outre un atome O ou S et qui peut former une structure pontée ou peut être fusionné avec un cycle benzénique, -CONRa-alkylène C₁₋₆-ORb, -CONRa-alkylène C₁₋₆-NRbRc, -O-alkylène C₁-₆-ORb, -O-alkylène C₁₋₆-O-alkylène C₁₋₆-ORb, -O-alkylène C₁₋₆-NRaRb, -O-alkylène C₁₋₆-O-alkylène C₁₋₆-NRaRb, -O-alkylène C₁₋₆-NRc-alkylène C₁₋₆-NRaRb, -NRc-alkylène C₁₋₆-NRaRb, -N(alkylène C₁₋₆-NRaRb)₂, -CONRa-ORb, -NRa-CO-NRbRc ou -OCORb;
Le Groupe A consiste en - alkyle C₁₋₆, - alcényle de jusqu'à 6 atomes de carbone, - alcynyle de jusqu'à 6 atomes de carbone, - halogène, - alkyle C₁₋₆ halogéné, -alkylène C₁₋₆-OR, -NO₂, -CN, =O, -OR, -O-alkyle C₁₋₆ halogéné, -O-alkylène C₁₋₆-NRR', -O-alkylène C₁₋₆-OR, -O-alkylène C₁₋₆-aryle C₆₋₁₄, -SR, -SO₂-alkyle C₁₋₆, -SO-alkyle C₁₋₆, -COOR, - COO-alkylène C₁₋₆-aryle C₆₋₁₄, -COR, -CO-aryle C₆₋₁₄, aryle C₆₋₁₄, -CONRR', -SO₂NRR', - NRR', -NR"-alkylène C₁₋₆-NRR', -NR'-alkylène C₁₋₆-OR, -NR-alkylène C₁₋₆-aryle C₆₋₁₄, - NRCO-alkyle C₁₋₆, -NRSO₂-alkyle C₁₋₆, -cycloalkyle C₃₋₈ et -cycloalcényle C₃₋₈;
R, R' et R", qui peuvent être les mêmes ou différents, représentent H ou un alkyle C₁₋₆;
R² et R³ sont combinés ensemble avec l'atome N qui leur est adjacent pour former un groupe hétérocyclique saturé contenant de l'azote tel que -NR²R³, sélectionné parmi le groupe consistant en 1-pyrrolidinyle, 1-pipérazinyle, pipéridino et morpholino et qui peut avoir 1 à 2 substituants sélectionnés parmi le groupe consistant en -OH, =O et -alkyle C₁₋₆;
chaque Ra et Rc, qui peuvent être les mêmes ou différents, représente -H ou -akyle C₁₋₆;
Rb représente -H, -alkyle C₁₋₆, -cycloalkyle C₃₋₈, -aryle C₆₋₁₄ qui peut avoir 1 à 5 substituants sélectionnés parmi le Groupe A ou - un hétéroaryle qui est un hétéroaryle monocyclique à 5 ou 6 chaînons contenant 1 à 4 hétéroatomes sélectionnés parmi N, S et O ou un hétéroaryle bicyclique constitué d'un hétéroaryle monocyclique fusionné à un cycle benzénique, qui peut être partiellement saturé et qui peut avoir 1 à 5 substituants sélectionnés parmi le Groupe A;
n représente 0, 1, 2 ou 3, tandis que n représente 1, 2 ou 3 lorsque B représente un cycle benzénique;
R⁴ représente -aryle C₆₋₁₄ qui peut avoir 1 à 5 substituants sélectionnés parmi le Groupe A⁴, -alkylène C₁₋₆-aryle C₆₋₁₄ qui peut avoir 1 à 5 substituants sélectionnés parmi le Groupe A⁴, -alcénylène de jusqu'à 6 atomes de carbone -aryle C₆₋₁₄ qui peut avoir 1 à 5 substituants sélectionnés parmi le Groupe A⁴, -alcynylène de jusqu'à 6 atomes de carbone -aryle C₆₋₁₄ qui peut avoir 1 à 5 substituants sélectionnés parmi le Groupe A⁴, -cycloalkyle C₃₋₈ qui peut avoir 1 à 5 substituants sélectionnés parmi le Groupe A, -cycloalcényle C₃₋₈ qui peut avoir 1 à 5 substituants sélectionnés parmi le Groupe A, -alkylène C₁₋₆-cycloalkyle C₃₋₈ qui peut avoir 1 à 5 substituants sélectionnés parmi le Groupe A, - alcénylène de jusqu'à 6 atomes de carbone cycloalkyle C₃₋₈ qui peut avoir 1 à 5 substituants sélectionnés parmi le Groupe A, -alkylène C₁₋₆- un groupe hétérocyclique saturé contenant de l'azote qui est un groupe hétérocyclique de 5 à 7 chaînons contenant un ou deux atomes d'azote sur le cycle et qui peut contenir en outre un atome O ou S et peut former une structure pontée ou peut être fusionné avec un cycle benzénique et qui peut avoir 1 à 5 substituants sélectionnés parmi le Groupe A, -alcénylène de jusqu'à 6 atomes de carbone - un groupe hétérocyclique saturé contenant de l'azote qui est un groupe hétérocyclique de 5 à 7 chaînons contenant un ou deux atomes d'azote sur le cycle et qui peut contenir en outre un atome O ou S et peut former une structure pontée ou peut être fusionné avec un cycle benzénique et qui peut avoir 1 à 5 substituants sélectionnés parmi le Groupe A, - un hétéroaryle qui est un hétéroaryle monocyclique à 5 ou 6 chaînons contenant 1 à 4 hétéroatomes sélectionnés parmi N, S et O ou un hétéroaryle bicyclique constitué d'un hétéroaryle monocyclique fusionné à un cycle benzénique, qui peut être partiellement saturé et qui peut avoir 1 à 5 substituants sélectionnés parmi le Groupe A⁴, -alkylène C₁₋₆ - un hétéroaryle qui est un hétéroaryle monocyclique à 5 ou 6 chaînons contenant 1 à 4 hétéroatomes sélectionnés parmi N, S et O ou un hétéroaryle bicyclique constitué d'un hétéroaryle monocyclique fusionné à un cycle benzénique, qui peut être partiellement saturé et qui peut avoir 1 à 5 substituants sélectionnés parmi le Groupe A⁴, ou - un alcénylène de jusqu'à 6 atomes de carbone - un hétéroaryle qui est un hétéroaryle monocyclique à 5 ou 6 chaînons contenant 1 à 4 hétéroatomes sélectionnés parmi N, S et O ou un hétéroaryle bicyclique constitué d'un hétéroaryle monocyclique fusionné à un cycle benzénique, qui peut être partiellement saturé et qui peut avoir 1 à 5 substituants sélectionnés parmi le Groupe A⁴;
Le Groupe A⁴ consiste en a) : -alkyle C₁₋₆, -alcényle de jusqu'à 6 atomes de carbone, -alcynyle de jusqu'à 6 atomes de carbone, - un halogène, - un alkyle C₁₋₆ halogéné, -alkylène C₁₋₆-OR, -NO₂, -CN, =O, -O-alkyle C₁₋₆ halogéné, -SO₂-alkyle C₁₋₆, -SO-alkyle C₁₋₆, -COOR, -COO-alkylène C₁₋₆-aryle C₆₋₁₄, -COR, -CO-aryle C₆₋₁₄, -CONRR', -SO₂NRR', -Cyc ou-Alp-Cyc, où Alp représente alkylène C₁₋₆, alcénylène de jusqu'à 6 atomes de carbone ou alcynylène de jusqu'à 6 atomes de carbone et Cyc représente aryle C₆₋₁₄ qui peut avoir 1 à 5 substituants sélectionnés parmi le Groupe A; un hétéroaryle qui est un hétéroaryle monocyclique à 5 ou 6 chaînons contenant 1 à 4 hétéroatomes sélectionnés parmi N, S et O ou un hétéroaryle bicyclique constitué d'un hétéroaryle monocyclique fusionné à un cycle benzénique, qui peut être partiellement saturé et qui peut avoir 1 à 5 substituants sélectionnés parmi le Groupe A; un groupe hétérocyclique saturé contenant de l'azote qui est un groupe hétérocyclique de 5 à 7 chaînons contenant un ou deux atomes d'azote sur le cycle et qui peut contenir en outre un atome O ou S et peut former une structure pontée ou peut être fusionné avec un cycle benzénique et qui peut avoir 1 à 5 substituants sélectionnés parmi le Groupe A; cycloalkyle C₃₋₈ qui peut avoir 1 à 5 substituants sélectionnés parmi le Groupe A, ou cycloalcényle C₃₋₈ qui peut avoir 1 à 5 substituants sélectionnés parmi le Groupe A,
b) : -NR-E-F, où E représente -CO-, -COO-, -CONR'-, -SO₂NR'- ou -SO₂-; F représente -Cyc ou-alkyle C₁₋₆, alcényle de jusqu'à 6 atomes de carbone ou alcynyle de jusqu'à 6 atomes de carbone, qui peut être substitué par un ou plusieurs substituants sélectionnés parmi le groupe consistant en - un halogène, -NO₂-, -CN, -OR, -O-alkylène C₁₋₆-NRR', -O-alkylène C₁₋₆-OR, -SR, -SO₂-alkyle C₁₋₆, -SO-alkyle C₁₋₆, -COOR, -COR, -CO-aryle C₆₋₁₄, -CONRR', -SO₂NRR', -NRCO-alkyle C₁₋₆, -NRR', -NR'-alkylène C₁₋₆-OR, -NR"-alkylène C₁₋₆-NRR' et -Cyc; et
c) : -Z-R', -Z-Cyc, -Z-Alp-Cyc, -Z-Alp-Z'-R' ou -Z-Alp-Z'-Cyc, où chaque Z et Z', qui peuvent être les mêmes ou différents, représente indépendamment O, S ou NR;
à condition que lorsque 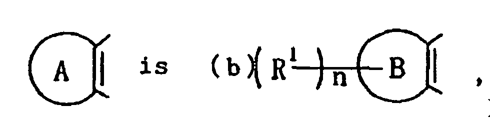 R² et R³ forment -NR²-R³ qui est - morpholino et R⁴ représente -aryle C₆₋₁₄ qui peut avoir 1 à 5 substituants sélectionnés parmi le Groupe A⁴ ou - un hétéroaryle qui est un hétéroaryle monocyclique à 5 ou 6 chaînons contenant 1 à 4 hétéroatomes sélectionnés parmi N, S et O ou un hétéroaryle bicyclique constitué d'un hétéroaryle monocyclique fusionné à un cycle benzénique, qui peut être partiellement saturé et qui peut avoir 1 à 5 substituants sélectionnés parmi le Groupe A⁴, et que les composés suivants soient
exclus :
(1) 4-(4-morpholinyl)-2-phénylpyrido[2,3-d]pyrimidine,
(2) 4-(4-morpholinyl-2-phénylpyrido[2,3-d]pyrimidin-7(1H)-one,
(3) 4-(4-morpholinyl)-2-phény-6-quinazolinol et 6-méthoxy-4-(4-morpholinyl)-2-phényquinazoline,
(4) des composés dans lesquels B représente un cycle benzénique, n est 2 ou 3, les R¹ existants représentent tous -OMe et R⁴ est un phényle non substitué ou un phényle qui est substitué par 1 à 3 substituants sélectionnés parmi -halogène, -NO₂, -alkyle C₁₋₆, - O-alkyle C₁₋₆, - un alkyle C₁₋₆ halogéné et -CONRaRc,
(5) des composés dans lesquels B représente un cycle benzénique, n est 1, R¹ représente -halogène ou - un alkyle C₁₋₆ et R⁴ représente - un imidazolyle qui peut avoir un ou plusieurs substituants,
(6) des composés dans lesquels B représente un cycle pyridine et R⁴ représente un phényle non substitué ou un pyridyle non substitué,
(7) des composés dans lesquels B représente un cycle pyrazine et R⁴ représente un phényle non substitué, et
(8) 4-(4-morpholinyl)-2-phénylthiéno[2,3-d]pyrimidine.

2. La composition pharmaceutique selon l'une des revendications 1, où la composition pharmaceutique est un agent antitumoral.

3. Utilisation du dérivé hétéroaryle fusionné représenté par la formule (I) de la revendication 1 ou d'un sel de celui-ci dans la fabrication d'un médicament à utiliser dans le traitement du cancer.

4. Dérivé hétéroaryle fusionné représenté par la formule générale (Ia) ou un sel de celui-ci : 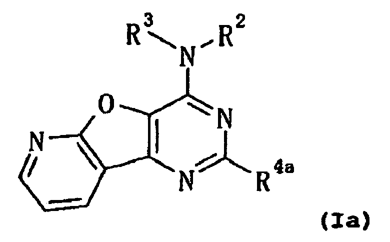 où :
R²et R³ sont combinés ensemble avec l'atome N qui leur est adjacent pour former un groupe hétérocyclique saturé contenant de l'azote tel que -NR²R³, sélectionné parmi un groupe consistant en 1-pyrrolidinyle, 1-pipérazinyle, pipéridino et morpholino, et qui peut avoir 1 à 2 substituants sélectionnés parmi le groupe consistant en -OH, =O et -alkyle C₁-6;
R^{4a} représente -aryle C₆₋₁₄ qui peut avoir 1 à 5 substituants sélectionnés parmi le Groupe A⁴, -alkylène C₁₋₆-aryle C₆₋₁₄ qui peut avoir 1 à 5 substituants sélectionnés parmi le Groupe A⁴, -alcénylène de jusqu'à 6 atomes de carbone -aryle C₆₋₁₄ qui peut avoir 1 à 5 substituants sélectionnés parmi le Groupe A⁴, -alcynylène de jusqu'à 6 atomes de carbone -aryle C₆₋₁₄ qui peut avoir 1 à 5 substituants sélectionnés parmi le Groupe A⁴, -cycloalkyle C₃₋₈ qui peut avoir 1 à 5 substituants sélectionnés parmi le Groupe A, -cycloalcényle C₃₋₈ qui peut avoir 1 à 5 substituants sélectionnés parmi le Groupe A, -alkylène C₁₋₆-cycloalkyle C₃₋₈ qui peut avoir 1 à 5 substituants sélectionnés parmi le Groupe A, - alcénylène de jusqu'à 6 atomes de carbone -cycloalkyle C₃₋₈ qui peut avoir 1 à 5 substituants sélectionnés parmi le Groupe A, -alkylène C₁₋₆- un groupe hétérocyclique saturé contenant de l'azote qui est un groupe hétérocyclique de 5 à 7 chaînons contenant un ou deux atomes d'azote sur le cycle et qui peut contenir en outre un atome O ou S et peut former une structure pontée ou peut être fusionné avec un cycle benzénique et qui peut avoir 1 à 5 substituants sélectionnés parmi le Groupe A, -alcénylène de jusqu'à 6 atomes de carbone - un groupe hétérocyclique saturé contenant de l'azote qui est un groupe hétérocyclique de 5 à 7 chaînons contenant un ou deux atomes d'azote sur le cycle et qui peut contenir en outre un atome O et S et peut former une structure pontée ou peut être fusionné avec un cycle benzénique et qui peut avoir 1 à 5 substituants sélectionnés parmi le Groupe A, - un hétéroaryle qui est un hétéroaryle monocyclique à 5 ou 6 chaînons contenant 1 à 4 hétéroatomes sélectionnés parmi N, S ou O ou un hétéroaryle bicyclique constitué d'un hétéroaryle monocyclique fusionné à un cycle benzénique, qui peut être partiellement saturé et qui peut avoir 1 à 5 substituants sélectionnés parmi le Groupe A⁴, -alkylène C₁₋₆- un hétéroaryle qui est un hétéroaryle monocyclique à 5 ou 6 chaînons contenant 1 à 4 hétératomes sélectionnés parmi N, S et O ou un hétéroaryle bicyclique constitué d'un hétéroaryle monocyclique fusionné à un cycle benzénique, qui peut être partiellement saturé et qui peut avoir 1 à 5 substituants sélectionnés parmi le Groupe A⁴, ou -alcénylène de jusqu'à 6 atomes de carbone - un hétéroaryle qui est un hétéroaryle monocyclique à 5 ou 6 chaînons contenant 1 à 4 hétéroatomes sélectionnés parmi N, S et O ou un hétéroaryle bicyclique constitué d'un hétéroaryle monocyclique fusionné à un cycle benzénique, qui peut être partiellement saturé et qui peut avoir 1 à 5 substituants sélectionnés parmi le Groupe A⁴;
le Groupe A consiste en -alkyle C₁₋₆, -alcényle de jusqu'à 6 atomes de carbone, - alcynyle de jusqu'à 6 atomes de carbone, - un halogène, - un alkyle C₁₋₆ halogéné, - alkylène C₁₋₆-OR, -NO₂, -CN, =O, -OR, -O- un alkyle C₁₋₆ halogéné, -O-alkylène C₁₋₆-NRR', -O-alkylène C₁₋₆-OR, -O-alkylène C₁₋₆-aryle C₆₋₁₄, -SR, -SO₂-alkyle C₁₋₆, -SO-alkyle C₁₋₆, -COOR, -COO-alkylène C₁₋₆-aryle C₆₋₁₄, -COR, -CO-aryle C₆₋₁₄, -aryle C₆₋₁₄, CONRR',
-SO₂NRR', -NRR', -NR"-alkylène C₁₋₆-NRR', -NR'-alkylène C₁₋₆-OR, -NR-alkylène C₁₋₆-aryle C₆₋₁₄, -NRCO-alkyle C₁₋₆, -NRSO₂-alkyle C₁₋₆, -cycloalkyle C₃₋₈ et -cycloalcényle C₃₋₈;
R, R' et R", qui peuvent être les mêmes ou différents, représentent H ou alkyle C₁₋₆; et
le Groupe A⁴ consiste en a) : -alkyle C₁₋₆, -alcényle de jusqu'à 6 atomes de carbone, -alcynyle de jusqu'à 6 atomes de carbone, - un halogène, - un alkyle C₁₋₆ halogéné, -alkylène C₁₋₆-OR, -NO₂, -CN, =O, -O-alkyle C₁₋₆ halogéné, -SO₂-alkyle C₁₋₆, - SO-alkyle C₁₋₆, -COOR, -COO-alkylène C₁₋₆-aryle C₆₋₁₄, -COR, -CO-aryle C₆₋₁₄, - CONRR', -SO₂NRR', -Cyc ou-Alp-Cyc, où Alp représente alkylène C₁₋₆, alcénylène de jusqu'à 6 atomes de carbone ou alcynylène de jusqu'à 6 atomes de carbone et Cyc représente aryle C₆₋₁₄ qui peut avoir 1 à 5 substituants sélectionnés parmi le Groupe A, un hétéroaryle qui est un hétéroaryle monocyclique à 5 ou 6 chaînons contenant 1 à 4 hétéroatomes sélectionnés parmi N, S et O ou un hétéroaryle bicyclique constitué d'un hétéroaryle monocyclique fusionné à un cycle benzénique, qui peut être partiellement saturé et qui peut avoir 1 à 5 substituants sélectionnés parmi le Groupe A, un groupe hétérocyclique saturé contenant de l'azote qui est un groupe hétérocyclique de 5 à 7 chaînons contenant un ou deux atomes d'azote sur le cycle et qui peut contenir en outre un atome O ou S et peut former une structure pontée ou peut être fusionné avec un cycle benzénique et qui peut avoir 1 à 5 substituants sélectionné parmi le Groupe A, cycloalkyle C₃₋₈ et qui peut avoir 1 à 5 substituants sélectionnés parmi le Groupe A, ou cycloalcényle C₃₋₈ qui peut avoir 1 à 5 substituants sélectionnés parmi le Groupe A,
b) : -NR-E-F, où E représente -CO-, -COO-, -CONR'-, -SO₂NR'- ou -SO₂-; F représente -Cyc ou-alkyle C₁₋₆, alcényle de jusqu'à 6 atomes de carbone ou alcynyle de jusqu'à 6 atomes de carbone, qui peut être substitué par un ou plusieurs substituants sélectionnés parmi le groupe consistant en - un halogène, -NO₂, -CN, -OR, -O-alkylène C₁₋₆-NRR', -O-alkylène C₁₋₆-OR, -SR, -SO₂-alkyle C₁₋₆, -SO-alkyle C₁₋₆, -COOR, -COR, -CO-aryle C₆₋₁₄, -CONRR', -SO₂NRR', -NRCO-alkyle C₁₋₆, -NRR', -NR'-alkylène C₁₋₆-OR, -NR"-alkylène C₁₋₆-NRR' et -Cyc; et
c) : -Z-R', -Z-Cyc, -Z-Alp-Cyc, -Z-Alp-Z'-R' ou Z-Alp-Z'-Cyc, où chaque Z et Z', qui peuvent être les mêmes ou différents, représente indépendamment O, S ou NR.

5. Le dérivé hétéroaryle fusionné ou un sel de celui-ci selon la revendication 4, dans lequel R²et R³ forment -NR²R³ qui est -morpholino.

6. Le dérivé hétéroaryle fusionné ou un sel de celui-ci selon la revendication 4, dans lequel R^{4a} représente -aryle C₆₋₁₄ qui peut avoir 1 à 5 substituants sélectionnés parmi le Groupe A⁴ ou - un hétéroaryle qui est un hétéroaryle monocyclique à 5 ou 6 chaînons contenant 1 à 4 hétéroatomes sélectionnés parmi N, S et O ou un hétéroaryle bicyclique constitué d'un hétéroaryle monocyclique fusionné à un cycle benzénique, qui peut être partiellement saturé et qui peut avoir 1 à 5 substituants sélectionnés parmi le Groupe A⁴.

7. Le dérivé hétéroaryle fusionné ou un sel de celui-ci selon la revendication 4, dans lequel le dérivé hétéroaryle fusionné ou un sel de celui-ci est sélectionné parmi le groupe consistant en 6-amino-3'-(4-morpholinopyrido[3',2':4,5]furo[3,2-d]pyrimidin-2-yl)nicotinanilide, 4-(4-morpholinopyrido[3',2':4,5]furo[3,2-d]pyrimidin-2-yl)aniline, 3-(4-morpholinopyrido[3',2':4,5]furo[3,2-d]pyrimidin-2-yl)phénol, 4-morpholino-2-[3-(2-pipérazin-1-yléthoxy)phényl]pyrido[3',2':4,5]furo[3,2-d]pyrimidine, 3'-(4-morpholinopyrido[3',2':4,5]furo[3,2-d]pyrimidin-2-yl)acrylanilide, et en des sels de ceux-ci.

8. Dérivé hétéroaryle fusionné représenté par la formule générale (Ib) ou un sel de celui-ci : 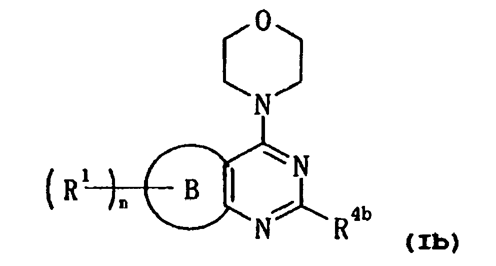 où :
B représente un cycle benzénique ou des cycles pyridine, pyrazine ou thiophène;
R¹ représente -alkyle C₁₋₆, -alcényle de jusqu'à 6 atomes de carbone, -alcynyle de jusqu'à 6 atomes de carbone, -cycloalkyle C₃₋₈ -aryle C₆₋₁₄ qui peut avoir 1 à 5 substituants sélectionnés parmi le Groupe A, - un hétéroaryle qui est un hétéroaryle monocyclique à 5 ou 6 chaînons contenant 1 à 4 hétéroatomes sélectionnés parmi N, S et O ou un hétéroaryle bicyclique constitué d'un hétéroaryle monocyclique fusionné à un cycle benzénique, qui peut être partiellement saturé et qui peut avoir 1 à 5 substituants sélectionnés parmi le Groupe A, - un halogène, -NO₂, -CN, - un alkyle C₁₋₆ halogéné, -ORb, -SRb, -SO₂-Rb, -SO-Rb, -COORb, -CO-Rb, -CONRaRb, -SO₂NRaRb, -NRaRb, -NRa-CORb, -NRa-SO₂Rb -O-CO-NRaRb, -NRaCO-COORb, -NRaCOORb, -NRaCO-alkylène C₁₋₆-aryle C₆₋₁₄, -NRa-SO₂-alkylène C₁₋₆-aryle C₆₋₁₄, -NRa-alkylène C₁₋₆-aryle C₆-₁₄, -alkylène C₁₋₆-ORb, -alkylène C₁₋₆-NRaRb, -CO- un groupe hétérocyclique saturé contenant de l'azote qui est un groupe hétérocyclique de 5 à 7 chaînons contenant un ou deux atomes d'azote sur le cycle et qui peut contenir en outre un atome O ou S et peut former une structure pontée ou peut être fusionné avec un cycle benzénique, -CONRa-alkylène C₁₋₆-ORb, -CONRa-alkylène C₁₋₆-NRcRb, -CONRa-alkylène C₁₋₆- un groupe hétérocyclique saturé contenant de l'azote qui est un groupe hétérocyclique de 5 à 7 chaînons contenant un ou deux atomes d'azote sur le cycle et qui peut contenir en outre un atome O ou S et peut former une structure pontée ou peut être fusionné avec un cycle benzénique, -O-alkylène C₁₋₆-ORb, -O-alkylène C₁₋₆-NRaRb, -O-alkylène C₁₋₆- un groupe hétérocyclique saturé contenant de l'azote qui est un groupe hétérocyclique de 5 à 7 chaînons contenant un ou deux atomes d'azote sur le cycle et qui peut contenir en outre un atome O ou S et peut former une structure pontée ou peut être fusionné avec un cycle benzénique, -O-alkylène C₁₋₆-O-alkylène C₁₋₆-ORb, -O-alkylène C₁₋₆-O-alkylène C₁₋₆-NRaRb, -O-alkylène C₁₋₆-NRc-alkylène C₁₋₆-NRaRb, -NRc-alkylène C₁₋₆-NRaRb, -N(alkylène C₁₋₆-NRaRb)₂, -CONRa-ORb, -NRa-CO-NRbRc ou -OCORb;
le Groupe A consiste en -alkyle C₁₋₆, -alcényle de jusqu'à 6 atomes de carbone, - alcynyle de jusqu'à 6 atomes de carbone, - un halogène, - un alkyle C₁₋₆ halogéné, - alkylène C₁₋₆-OR, -NO₂, -CN, =O, -OR, -O- un alkyle C₁₋₆ halogéné, -O-alkylène C₁₋₆-NRR', -O-alkylène C₁₋₆-OR, -O-alkylène C₁₋₆-aryle C₆₋₄, -SR, -SO₂-alkyle C₁₋₆, -SO-alkyle C₁₋₆, -COOR, -COO-alkylène C₁₋₆-aryle C₆₋₁₄, -COR, -CO-aryle C₆₋₁₄, n aryle C₆₋₁₄, -CONRR', -SO₂NRR', -NRR', -NR"-alkylène C₁₋₆-NRR', -NR'-alkylène C₁₋₆-OR, -NR-alkylène C₁₋₆-aryle C₆₋₁₄, -NRCO-alkyle C₁₋₆, -NRSO₂-alkyle C₁₋₆, -cycloalkyle C₃₋₈ et -cycloalcényle C₃₋₈;
R, R' et R", qui peuvent être les mêmes ou différents, représentent H ou alkyle C₁₋₆;
Ra et Rc, qui peuvent être les mêmes ou différents, représentent -H ou -alkyle C₁₋₆;
Rb représente -H, -alkyle C₁₋₆, -cycloalkyle C₃₋₈, -aryle C₆₋₁₄ qui peut avoir 1 à 5 substituants sélectionnés parmi le Groupe A ou - un hétéroaryle qui est un hétéroaryle monocyclique à 5 ou 6 chaînons contenant 1 à 4 hétéroatomes sélectionnés parmi N, S et O ou un hétéroaryle bicyclique constitué d'un hétéroaryle monocyclique fusionné à un cycle benzénique, qui peut être partiellement saturé et qui peut avoir 1 à 5 substituants sélectionnés parmi le Groupe A;
n représente 0, 1, 2 ou 3, tandis que n représente 1, 2 ou 3 lorsque B représente un cycle benzénique;
R^{4b} représente -aryle C₆₋₁₄ qui peut avoir 1 à 5 substituants sélectionnés parmi le Groupe A⁴ ou - un hétéroaryle qui est un hétéroaryle monocyclique à 5 ou 6 chaînons contenant 1 à 4 hétéroatomes sélectionnés parmi N, S et O ou un hétéroaryle bicyclique constitué d'un hétéroaryle monocyclique fusionné à un cycle benzénique, qui peut être partiellement saturé et qui peut avoir 1 à 5 substituants sélectionnés parmi le Groupe A⁴ et,
le Groupe A⁴ consiste en a) : -alkyle C₁₋₆, -alcényle de jusqu'à 6 atomes de carbone, -alcynyle de jusqu'à 6 atomes de carbone, - un halogène, - un alkyle C₁₋₆ halogéné, -alkylène C₁₋₆-OR, -NO₂, -CN, =O, -O-alkyle C₁₋₆ halogéné, -SO₂-alkyle C₁₋₆, -SO-alkyle C₁₋₆, -COOR, -COO-alkylène C₁₋₆-aryle C₆₋₁₄, -COR, -CO-aryle C₆₋₁₄,-CONRR', -SO₂NRR', -Cyc ou -Alp-Cyc, où Alp représente alkylène C₁₋₆, alcénylène de jusqu'à 6 atomes de carbones ou alcynylène de jusqu'à 6 atomes de carbone et Cyc représente aryle C₆₋₁₄ qui peut avoir 1 à 5 substituants sélectionnés parmi le Groupe A; un hétéroaryle qui est un hétéroaryle monocyclique à 5 ou 6 chaînons contenant 1 à 4 hétéroatomes sélectionnés parmi N, S et O ou un hétéroaryle bicyclique constitué d'un hétéroaryle monocyclique fusionné à un cycle benzénique, qui peut être partiellement saturé et qui peut avoir 1 à 5 substituants sélectionnés parmi le Groupe A; un groupe hétérocyclique saturé contenant de l'azote qui est un groupe hétérocyclique de 5 à 7 chaînons contenant un ou deux atomes d'azote sur le cycle et qui peut contenir en outre un atome O ou S et peut former une structure pontée ou peut être fusionné avec un cycle benzénique et qui peut avoir 1 à 5 substituants sélectionnés parmi le Groupe A; cycloalkyle C₃₋₈ qui peut avoir 1 à 5 substituants sélectionnés parmi le Groupe A; ou un cycloalcényle C₃₋₈ qui peut avoir 1 à 5 substituants sélectionnés parmi le Groupe A,
b) : -NR-E-F, où E représente -CO-, COO-, -CONR'-, -SO₂NR'- ou -SO₂-; F représente -Cyc ou -alkyle C₁₋₆, alcényle de jusqu'à 6 atomes de carbone ou alcynyle de jusqu'à 6 atomes de carbone, qui peut être substitué par un ou plusieurs substituants sélectionnés parmi le groupe consistant en - un halogène, -NO₂, -CN, -OR, -O-alkylène C₁₋₆-NRR', -O-alkylène C₁₋₆-OR, -SR, -SO₂-alkyle C₁₋₆, -SO-alkyle C₁₋₆, -COOR, -COR, -CO-aryle C₆₋₁₄, -CONRR', -SO₂NRR', -NRCO-alkyle C₁₋₆, -NRR', -NR'-alkylène C₁₋₆-OR, -NR"-alkylène C₁₋₆-NRR' et -Cyc, et
c) : -Z-R', -Z-Cyc, -Z-Alp-Cyc, -Z-Alp-Z'-R' ou -Z-Alp-Z'-Cyc, où chaque Z et Z', qui peuvent être les mêmes ou différents, représente indépendamment O, S ou NR;
à condition que les composés suivants soient exclus :
(1) 4-(4-morpholinyl)-2-phénylpyrido[2,3-d]pyrimidine,
(2) 4-(4-morpholinyl-2-phénylpyrido[2,3-d]pyrimidin-7(1H)-one,
(3) 4-(4-morpholinyl)-2-phény-6-quinazolinol et 6-méthoxy-4-(4-morpholinyl)-2-phényquinazoline,
(4) des composés dans lesquels B représente un cycle benzénique, n est 2 ou 3, les R¹ existants représentent tous -OMe et R^{4b} est un phényle non substitué ou un phényle qui est substitué par 1 à 3 substituants sélectionnés parmi -halogène, -NO₂, -alkyle C₁₋₆,-O-alkyle C₁₋₆, - un alkyle C₁₋₆ halogéné et -CONRaRc,
(5) des composés dans lesquels B représente un cycle benzénique, n est 1, R¹ représente -halogène ou - un alkyle inférieur et R^{4b} représente - (imidazolyle qui peut avoir un ou plusieurs substituants),
(6) des composés dans lesquels B représente un cycle pyridine et R^{4b} représente un phényle non substitué ou un pyridyle non substitué,
(7) des composés dans lesquels B représente un cycle pyrazine et R^{4b} représente un phényle non substitué, et
(8) 4-(4-morpholinyl)-2-phénylthiéno[2,3-d]pyrimidine.

9. Le dérivé hétéroaryle fusionné ou un sel de celui-ci selon la revendication 8, dans lequel R^{4b} représente aryle C₆₋₁₄ qui a un ou plusieurs substituants sélectionnés parmi le groupe consistant en -alkylène C₁₋₆-OR, -CONRR', -NR-CO-Cyc¹, -NR-SO₂-Cyc¹, -OR, - NRR', -O-alkylène C₁₋₆-NRR' et -O-alkylène C₁₋₆- un groupe hétérocyclique saturé contenant de l'azote qui est un groupe hétérocyclique de 5 à 7 chaînons contenant un ou deux atomes d'azote sur le cycle et qui peut contenir en outre un atome O ou S et peut former une structure pontée ou peut être fusionné avec un cycle benzénique et qui peut avoir 1 à 5 substituants sélectionnés parmi le Groupe A; où Cyc¹ est -aryle C₆₋₁₄ qui peut avoir 1 à 5 substituants sélectionnés parmi le Groupe A; - un hétéroaryle qui est un hétéroaryle monocyclique à 5 ou 6 chaînons contenant 1 à 4 hétéroatomes sélectionnés parmi N, S et O ou un hétéroaryle bicyclique constitué d'un hétéroaryle monocyclique fusionné à un cycle benzénique, qui peut être partiellement saturé et qui peut avoir 1 à 5 substituants sélectionnés parmi le Groupe A; ou - un groupe hétérocyclique saturé contenant de l'azote qui est un groupe hétérocyclique de 5 à 7 chaînons contenant un ou deux atomes d'azote sur le cycle et qui peut contenir en outre un atome O ou S et peut former une structure pontée ou peut être fusionné avec un cycle benzénique et qui peut avoir 1 à 5 substituants sélectionnés parmi le Groupe A.

10. Le dérivé hétéroaryle fusionné ou un sel de celui-ci selon la revendication 8, dans lequel B représente un cycle benzénique, n est 1 ou 2 et R¹ représente -NO₂, -CN, - un alkyle C₁₋₆ halogéné, -ORb, -SRb, -NRaRb, -NRa-CORb ou NRa-SO₂Rb.

11. Le dérivé hétéroaryle fusionné ou un sel de celui-ci selon la revendication 8, dans lequel B représente un cycle pyridine, pyrazine ou thiophène, n est 0 et R^{4b} représente un phényle qui a au moins un substituant qui est sélectionné parmi -OH, -CH₂OH et - CONH₂.

12. Le dérivé hétéroaryle fusionné ou un sel de celui-ci selon la revendication 8, dans lequel B représente un cycle pyridine.

13. Le dérivé hétéroaryle fusionné ou un sel de celui-ci selon la revendication 8, dans lequel B représente un cycle pyrazine.

14. Le dérivé hétéroaryle fusionné ou un sel de celui-ci selon la revendication 8, dans lequel B représente un cycle thiophène.

15. Le dérivé hétéroaryle fusionné ou un sel de celui-ci selon la revendication 8, dans lequel le dérivé hétéroaryle fusionné ou un sel de celui-ci est sélectionné parmi le groupe consistant en N-[2-(3-benzènesulfonylaminophényl)-4-morphoniloquinazolin-6-yl]acétamide, 3-(4-morpholinopyrido[4,3-d]pyrimidin-2-yl)phénol, 3-(4-morpholinopyrido[3,2-d]pyrimidin-2-yl)phénol, 3-(4-morpholinopyrido[3,4-d]pyrimidin-2-yl)phénol, 3-(6-méthoxy-4-morpholinoquinazolin-2-yl)phénol, 3-(4-morpholinothiéno[3,2-d]pyrimidin-2-yl)phénol, 3-(4-morpholinoptéridin-2-yl)phénol, et des sels de ceux-ci.

16. Composition pharmaceutique comprenant un dérivé hétéroaryle fusionné ou un sel de celui-ci selon la revendication 4 et un excipient pharmaceutiquement acceptable.

17. Composition pharmaceutique comprenant un dérivé hétéroaryle fusionné ou un sel de celui-ci selon la revendication 8 et un excipient pharmaceutiquement acceptable.
